# FASCICULE DE BREVET EUROPEEN

(11) **EP 4 084 799 B1**
(45) Date de publication et mention de la délivrance du brevet: **17.04.2024**
(21) Numéro de dépôt: 21701672.4
(22) Date de dépôt: 12.01.2021
(51) Int. Cl.: A61K 31/706, A61P 19/02, A61P 29/00

(54) **UTILISATION DE NMN POUR LA PREVENTION ET/OU LE TRAITEMENT D'UNE DOULEUR ARTICULAIRE INDUITE PAR L'ACTIVITE PHYSIQUE ET COMPOSITIONS CORRESPONDANTES**
VERWENDUNG VON NMN ZUR VORBEUGUNG UND/ODER BEHANDLUNG VON DURCH KÖRPERLICHE AKTIVITÄT INDUZIERTEN GELENKSCHMERZEN UND ENTSPRECHENDE ZUSAMMENSETZUNGEN
USE OF NMN FOR THE PREVENTION AND/OR TREATMENT OF JOINT PAIN INDUCED BY PHYSICAL ACTIVITY, AND CORRESPONDING COMPOSITIONS

(30) Priorité: 13.01.2020 FR 2000270
(43) Date de publication de la demande: 09.11.2022
(73) Titulaire: Nuvamid SA, 1260 Nyon (Suisse) (CH)
(72) Inventeur: BERMOND, Guillaume, 13007 MARSEILLE (FR); GARCON, Laurent, 13960 SAUSSET LES PINS (FR)
(74) Mandataire: August Debouzy
(86) Numéro de dépôt international: PCT/EP2021/050499
(87) Numéro de publication internationale: WO 2021/144274

(56) Documents cités:
- WO-A1-2017/024255
- WO-A1-2019/222360
- WO-A1-2019/222368
- WO-A2-2020/131578
- US-A1- 2017 080 011
- US-A1- 2017 266 213

## Description

### DOMAINE DE L'INVENTION

La présente invention porte sur l'utilisation de nicotinamide mononucléotide (NMN), un de ses dérivés pharmaceutiquement acceptables ou un de ses sels pharmaceutiquement acceptables, ainsi que de compositions le comprenant, pour la prévention et/ou le traitement d'une douleur articulaire, de préférence une gonalgie, induite par l'activité physique, notamment par la pratique d'une activité sportive.

### ARRIERE-PLAN TECHNIQUE

L'activité physique, et en particulier la pratique d'un sport, permet de maintenir une bonne condition physique et d'améliorer sa santé. L'OMS formule des recommandations mondiales en matière d'activité physique pour la santé, variant en termes de durée et d'intensité en fonction de l'âge du sujet (consultables sous le numéro ISBN: 9789241599979).

Toutefois, une pratique trop intensive, mal exécutée ou avec un mauvais équipement peut provoquer des douleurs articulaires. Par exemple, les douleurs articulaires au coude, à l'épaule ou au genou sont fréquentes au tennis. Les douleurs articulaires aux genoux sont également fréquentes pour les adeptes de la course à pied, notamment lorsque les individus la pratiquent en ville sur des terrains particulièrement durs pour les articulations ou lorsque leurs chaussures ne sont pas adaptées. Les joueurs de golf peuvent également ressentir des douleurs à l'épaule, au dos ou au genou.

On peut également ressentir une douleur articulaire lorsqu'on reste trop longtemps dans une mauvaise position, par exemple lorsqu'on reste longtemps accroupi en jardinant ou en bricolant. De même, des gestes répétitifs dans le cadre d'un travail, peut occasionner des douleurs articulaires. Par exemple, monter et descendre un escabeau pour ranger des produits dans une réserve peut occasionner des douleurs au genou ou à l'épaule.

De telles douleurs ne sont donc pas provoquées par un état pathologique sous-jacent tels que l'arthrose, une pathologie inflammatoire comme l'arthrite ou une inflammation des cartilages, une tumeur, une maladie auto-immune, une ostéopathie, une chondropathie etc. Elles ne sont pas non plus provoquées par un état traumatique tels qu'une fracture, une entorse, une luxation ou des contusions, à moins d'être induit par la pratique sportive.

De telles douleurs sont généralement passagères, localisées et peu intenses. Le traitement de telles douleurs relève souvent de l'automédication et comprend généralement l'administration d'antalgiques, d'anti-inflammatoires non stéroïdiens (AINS) ou de dérivés de cortisone, par voie topique ou voie orale.

Cependant, l'administration de ces médicaments endommagent, entre autres, l'estomac, le foie et les reins. De plus, leur efficacité se réduit avec le temps nécessitant l'augmentation des doses. Par ailleurs, l'utilisation chronique de dérivés de la cortisone induit notamment une fragilité osseuse, des effets neuropsychiatriques, une fonte musculaire et une réduction de l'immunité, laissant le patient vulnérable aux infections.

Il existe donc un besoin de développer de nouvelles compositions pour le traitement et/ou la prévention des douleurs articulaires induites par l'activité physique, en particulier la pratique d'un sport, qui réduisent les inconvénients de l'art antérieur.

### RESUME DE L'INVENTION

Ces objectifs sont atteints grâce à l'invention tel que décrite ci-dessous.

La présente invention a pour objet le nicotinamide mononucléotide (NMN), un de ses dérivés pharmaceutiquement acceptables ou un de ses sels pharmaceutiquement acceptables, pour son utilisation par voie topique dans la prévention et/ou le traitement d'une douleur articulaire induite par l'activité physique.

Le dérivé pharmaceutiquement acceptable de NMN est choisi parmi un composé de formule (I) : 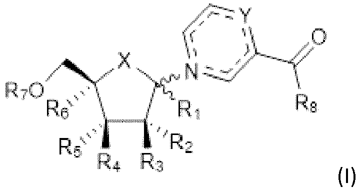 ou un de ses stéréoisomères, un de ses sels, un de ses hydrates, un de ses solvates ou un de ses cristaux pharmaceutiquement acceptable de celui-ci, dans lequel :
- X est choisi parmi O, CH₂, S, Se, CHF, CF₂ et C=CH₂;
- R₁ est choisi parmi H, azido, cyano, alkyle en C₁-C₈, thio-alkyle en C₁-C₈, hétéroalkyle en C₁-C₈ et OR ; dans lequel R est choisi parmi H et alkyle en C₁-C₈ ;
- R₂, R₃, R₄ et R₅ sont choisis indépendamment l'un de l'autre parmi H, halogène, azido, cyano, hydroxyle, alkyle en C₁-C₁₂, thio-alkyle en C₁-C₁₂, hétéroalkyle en C₁-C₁₂, haloalkyle en C₁-C₁₂ et OR ; dans lequel R est choisi parmi H, alkyle en C₁-C₁₂, C(O)(C₁-C₁₂)alkyle, C(O)NH(C₁-C₁₂)alkyle, C(O)O(C₁-C₁₂)alkyle, C(O)aryle, C(O)(C₁-C₁₂)alkyle aryle, C(O)NH(C₁-C₁₂)alkyle aryle, C(O)O(C₁-C₁₂)alkyle aryle et C(O)CHR_{AA}NH₂ ; dans lequel R_{AA} est une chaîne latérale choisie parmi un acide aminé protéinogène ;
- R₆ est choisi parmi H, azido, cyano, C₁-C₈ alkyle, C₁-C₈ thio-alkyle, C₁-C₈ hétéroalkyle et OR ; dans lequel R est choisi parmi H et C₁-C₈ alkyle;
- R₇ est choisi parmi H, P(O)R₉R₁₀, P(S)R₉R₁₀ et 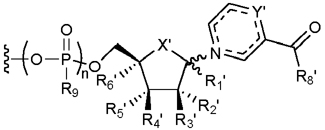 où n est un entier choisi parmi 1, 2 ou 3 ; dans lequel
- R₉ et R₁₀ sont choisis indépendamment l'un de l'autre parmi OH, OR₁₁, NHR₁₃, NR₁₃R₁₄, un alkyle en C₁-C₈, un alcényle en C₂-C₈, un alcynyl en C₂-C₈, un cycloalkyl en C₃-C₁₀, un aryle en C₅-C₁₂, (C₁-C₈)alkyle aryle, (C₁-C₈)aryle alkyle, (C₁-C₈) heteroalkyle, (C₁-C₈) heterocycloalkyle, un heteroaryle et NHCHR_{A}R_{A'}-C(O)R₁₂ ; dans lequel :
   - R₁₁ est choisi parmi un groupe C₁-C₁₀ alkyle, C₃-C₁₀ cycloalkyle, C₅-C₁₈ aryle, C₁-C₁₀ alkylaryle, C₅-C₁₂ aryle substitué, C₁-C₁₀ heteroalkyle, C₃₋C₁₀ heterocycloalkyle, C₁-C₁₀ haloalkyle, un heteroaryle, - (CH₂)ₙC(O)(C₁-C₁₅)alkyle, -(CH₂)ₙOC(O)(C₁-C₁₅)alkyle, -(CH₂)ₙOC(O)O(C₁-C₁₅)alkyle, -(CH₂)ₙSC(O)(C₁-C₁₅)alkyle, -(CH₂)ₙC(O)O(C₁-C₁₅)alkyle and -(CH₂)ₙC(O)O(C₁-C₁₅)alkyle aryle; dans lesquels n est un entier choisi de 1 à 8; P(O)(OH)OP(O)(OH)₂; halogène, nitro, cyano, C₁-C₆ alkoxy, C₁-C₆ haloalkoxy, -N(R₁₁ₐ)₂, C₁-C₆ acylamino, -COR_{11b}, -O COR_{11b}; NHSO₂(C₁-C₆ alkyl), - SO₂N(R₁₁ₐ)₂ SO₂ dans lequel chacun de R₁₁ₐ est indépendamment choisi parmi H et un C₁-C₆ alkyle et R_{11b} est indépendamment choisi parmi OH, C₁-C₆ alkoxy, NH₂, NH(C₁-C₆alkyle) ou N(C₁-C₆alkyle)₂ ;
   - R₁₂ est choisi parmi H, C₁₋C₁₀ alkyle, C₂-C₈ alcényle, C₂-C₈ alcynyle, C₁₋C₁₀ haloalkyle, C₃₋C₁₀ cycloalkyle, C₃₋C₁₀ heterocycloalkyle, C₅-C₁₈ aryle, C₁-C₄ alkylaryle and C₅-C₁₂ heteroaryle; dans lesquels lesdits groupes aryle ou heteroaryle sont optionnellement substitué parmi un ou deux groupes choisi parmi un halogène, trifluoromethyl, C₁-C₆ alkyle, C₁-C₆ alkoxy et cyano; et
   - R_{A} et R_{A'} sont indépendamment choisi parmi H, un C₁₋C₁₀ alkyle, C₂-C₁₀ alcényle, C₂-C₁₀ alcynyle, C₃₋C₁₀ cycloalkyle, C₁₋C₁₀ thio-alkyle, C₁₋C₁₀ hydroxylalkyle, C₁₋C₁₀ alkylaryle and C₅-C₁₂ aryle, C₃₋C₁₀ heterocycloalkyle, un heteroaryle, -(CH₂)₃NHC(=NH)NH₂, (1H-indol-3-yl)methyl, (1H-imidazol-4-yl)methyl et une chaine latérale choisie parmi un acide aminé protéinogène ou un acide aminé non protéinogène; dans lesquels lesdits groupes aryle sont optionnellement substitué avec un groupe choisi parmi un hydroxyle, C₁₋C₁₀alkyl, C₁₋C₆ alkoxy, un halogène, un nitro et un cyano; ou
   - R₉ et R₁₀ forment ensemble avec les atomes de phosphore auxquels ils sont attaché un cycle à 6 membres dans lequel -R₉-R₁₀- représente -CH₂-CH₂-CHR-; dans lequel R est choisi parmi H, un groupe (C₅-C₆) aryle et (C₅-C₆) heteroaryle, dans lequel lesdits groupes aryle ou heteroaryle sont optionnellement substitués par un halogène, trifluoromethyl, un C₁-C₆ alkyle, un C₁-C₆ alkoxy et cyano; ou
   R₉ et R₁₀ forment ensemble avec les atomes de phosphore auxquels ils sont attaché un cycle à 6 membres dans lequel -R₉-R₁₀- représente -O-CH₂-CH₂-CHR-O-; dans lequel R est choisi parmi H, un groupe (C₅-C₆) aryle et (C₅-C₆) heteroaryle, dans lequel lesdits groupes aryle ou heteroaryle sont optionnellement substitués par un halogène, trifluoromethyl, un (C₁-C₆) alkyle, un (C₁-C₆) alkoxy et cyano;
   - R₈ est choisi parmi H, OR, NHR₁₃, NR₁₃R₁₄, NH-NHR₁₃, SH, CN, N₃ et halogène ; dans lequel R₁₃ et R₁₄ sont choisis indépendamment l'un de l'autre parmi H, (Cᵢ-C_{g}) alkyle, (C₁-C₈) alkyle aryle, et -CR_{B}R_{C}-C(O)-OR_{D} dans lequel R_{B} et R_{C} sont indépendamment un atome d'hydrogène, un (C₁-C₆) alkyle, un (C₁-C₆) alkoxy, benzyle, indolyle ou imidazolyle, où le (C₁-C₆) alkyle et le (C₁-C₆)) alkoxy peuvent être éventuellement et indépendamment l'un de l'autre substitué par un ou plusieurs des groupes halogène, amino, amido, guanidyle, hydroxyle, thiol ou carboxyle, et le groupe benzyle est éventuellement substitué par un ou plusieurs des groupes halogène ou hydroxyle, ou R_{B} et Reforment ensemble avec l'atome de carbone auquel ils sont attachés un groupe cycloalkyle en C₃-C₆ éventuellement substitué par un ou plusieurs halogènes, amino, amido, guanidyle, hydroxyle, thiol et carboxyle et R_{D} est un hydrogène, un (C₁-C₆) alkyle, un (C₂-C₆) alcényle, un (C₂-C₆) alcynyle ou un (C₃-C₆) cycloalkyle ;
   - Y est choisi parmi CH, CH₂, C(CH₃)₂ et CCH₃ ;
   - -̅ -̅ -̅ représente une simple ou une double liaison selon Y ; et
   - 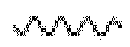 représente l'anomère alpha ou bêta selon la position de R₁ ou un composé de formule (la) :

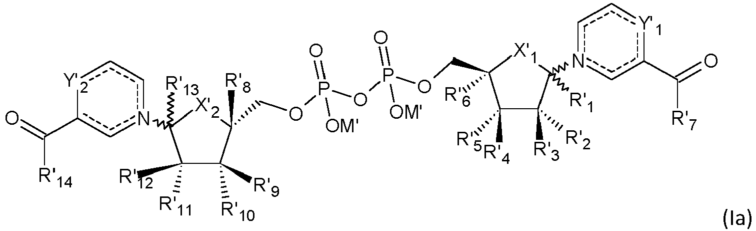

   ou un de ses stéréoisomères, un de ses sels, un de ses hydrates, un de ses solvates ou un de ses cristaux, dans lequel
   X'₁ et X'₂ sont indépendamment choisis parmi O, CH₂, S, Se, CHF, CF₂, et C=CH₂ ;
   R'₁ et R'13 sont indépendamment choisis parmi H, azido, cyano, un alkyl en C1-C8, un thio-alkyl en C1-C8, un hétéroalkyl en C1-C8, et OR, dans lequel R est choisi parmi H et un alkyl en C1-C8,
   R'₂, R'₃, R'₄, R'₅, R'₉, R'₁₀, R'₁₁, R'₁₂ sont indépendamment choisis parmi H, un halogène, un azido, un cyano, un hydroxyl, un alkyl en C₁-C₁₂, un thioalkyl en C₁-C₁₂, un hétéro-alkyl en C₁-C₁₂, un haloalkyl en C₁-C₁₂ et OR, dans lequel R peut être choisi parmi H, un alkyl en C₁-C₁₂, un C(O)( C₁-C₁₂) alkyle, un C(O)NH(C₁-C₁₂) alkyle, un C(O)O(C₁-C₁₂) alkyle, un C(O) aryle, un C(O)( C₁-C₁₂) aryle, un C(O)NH(C₁-C₁₂) alkyle aryle, un C(O)O(C₁-C₁₂) alkyle aryle ou un groupe C(O)CHR_{AA}NH2 dans lequel R_{AA} est une chaine latérale choisi parmi un acide aminé protéinogène ;
   R'₆ et R'₈ sont indépendamment choisis parmi H, un azido, un cyano, un alkyl en C₁-C₈ et OR, dans lequel R est choisi parmi H et un alkyl en C₁-C₈ ;
   R'₇ et R'₁₄ sont indépendamment choisis parmi H, OR, NHR, NRR', NH-NHR, SH, CN, N₃ et un halogène, dans lequel R et R' sont indépendamment choisit parmi H et un (C₁-C₈) alkyle aryle ;
   Y'₁ et Y'₂ sont indépendamment choisis parmi CH, CH₂, C(CH₃)₂ ou CCH₃ ;
   M' est choisi parmi H ou un contre-ion adapté ;
   représente une liaison simple ou double en fonction de Y'₁ et Y'₂ ; et 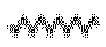 représente un anomère alpha ou béta en fonction de la position de R'₁ et R'₁₃;
   et leurs combinaisons.

Au sens de l'invention, M' peut être un contre-ion interne ou externe.

Dans un premier mode de réalisation préféré, le dérivé pharmaceutiquement acceptable est le composé de formule (I).

Dans une variante du premier mode de réalisation, X représente un oxygène.

Dans une variante du premier mode de réalisation, R₁ et R₆ représentent chacun indépendamment l'un de l'autre un hydrogène.

Dans une variante du premier mode de réalisation, R₂, R₃, R₄ et R₅ représentent chacun indépendamment l'un de l'autre un hydrogène ou un OH.

Dans une variante du premier mode de réalisation, Y représente un CH.

Dans une variante du premier mode de réalisation, Y représente un CH₂.

Dans une variante du premier mode de réalisation, R₇ représente un hydrogène.

Dans une variante du premier mode de réalisation, R₇ représente P(O)(OH)₂.

Dans une variante du premier mode de réalisation,
X représente un oxygène ; et/ou
R₁ et R₆ représente chacun indépendamment un hydrogène ; et/ou
R₂, R₃, R₄ et R₅ représente chacun indépendamment un hydrogène ou R₂, R₃, R₄ et R₅ représente indépendamment OH ; et/ou
Y représente un CH ou un CH₂; et/ou
R₇ représente P(O)R₉R₁₀, dans lequel R₉ et R₁₀ sont indépendamment choisis parmi OH, OR₁₁, NHR₁₃, NR₁₃R₁₄, C₁-C₈alkyle, C₂-C₈ alcényle, C₂-C₈ alcynyle, C₃₋C₁₀cycloalkyle, C₅-C₁₂ aryle, C₁-C₈ aryle alkyle, C₁-C₈ alkyle aryle, C₁-C₈heteroalkyle, C₁₋C₈heterocycloalkyle, heteroaryle et NHCR_{A}R_{A'}-C(O)R₁₂.

Dans une variante particulièrement préférée du premier mode de réalisation, le composé de l'invention est choisi parmi les composés de formule I-B à I-J :

**[Tableau 1]**

| **Composés (anomères)** | **Structure** |
|---|---|
| I-B (alpha) | 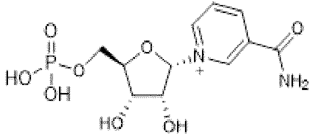 |
| I-C (beta) | 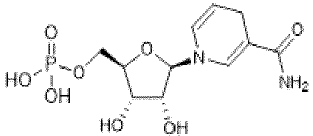 |
| I-D (alpha) | 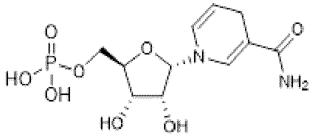 |
| I-E (beta) | 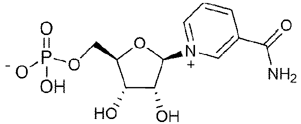 |
| I-F (alpha) | 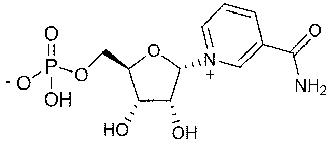 |
| I-G (beta) | 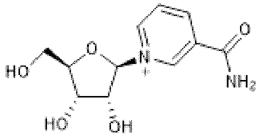 |
| I-H (alpha) | 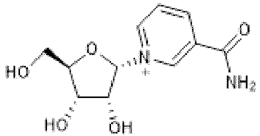 |
| I-I (beta) | 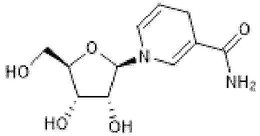 |
| I-J (alpha) | 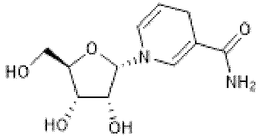 |

Avantageusement, le dérivé pharmaceutiquement acceptable du NMN peut être l'alpha-NMN (composés IB ou I-F).

Avantageusement, le dérivé pharmaceutiquement acceptable du NMN peut être le dihydronicotinamide mononucléotide (NMN-H) (composés IC ou ID).

Dans un deuxième mode de réalisation préféré, le dérivé pharmaceutiquement acceptable est le composé de formule (la).

Dans une variante du deuxième mode de réalisation, X'1 et X'2 représentent chacun indépendamment un oxygène.

Dans une variante du deuxième mode de réalisation, R'7 et R'14 représentent chacun indépendamment un NH₂.

Dans une variante du deuxième mode de réalisation, R'1 et/ou R'13 représentent chacun indépendamment un hydrogène.

Dans une variante du deuxième mode de réalisation, R'6 et/ou R'8 représentent chacun indépendamment un hydrogène.

Dans une variante du deuxième mode de réalisation, R'2, R'3, R'4, R'5, R'9, R'10, R'11 et R'12 représentent chacun indépendamment un hydrogène.

Dans une variante du deuxième mode de réalisation, R'2, R'3, R'4, R'5, R'9, R'10, R'11 et R'12 représentent chacun indépendamment un OH.

Dans une variante du deuxième mode de réalisation, Y'1 et Y'2 représentent chacun indépendamment un CH.

Dans une variante du deuxième mode de réalisation, Y'1 et Y'2 représentent chacun indépendamment un CH2.

Dans une variante du deuxième mode de réalisation, le composé selon l'invention est choisi parmi les composés de formule la-A à Ia-I :

**[Tableau 2]**

| **Composés (anomères)** | **Structure** |
|---|---|
| Ia-A (beta, beta) | 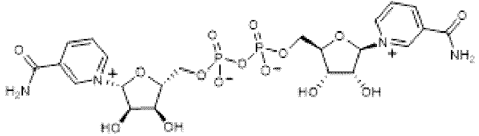 |
| Ia-B (beta, alpha) | 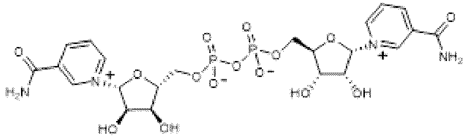 |
| Ia-C (alpha, alpha) | 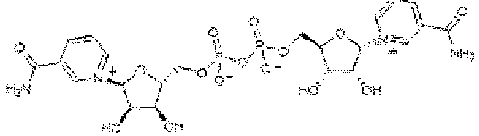 |
| Ia-D (beta, beta) | 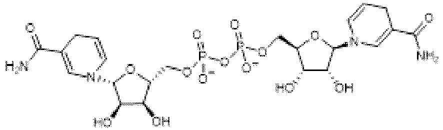 |
| Ia-E (beta, alpha) | 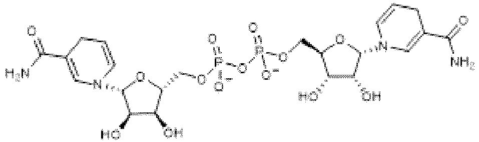 |
| Ia-F (alpha, alpha) | 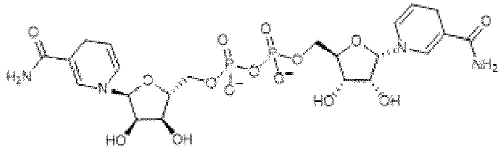 |
| Ia-G (beta, beta) | 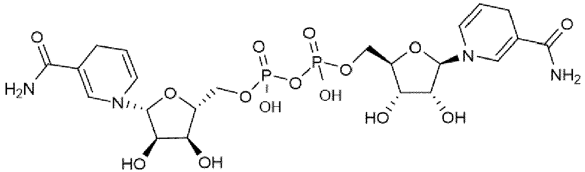 |
| Ia-H (beta, alpha) | 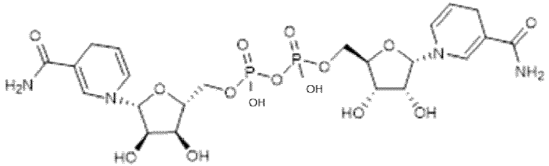 |
| Ia-I (alpha, alpha) | 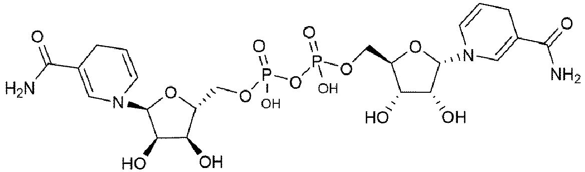 |

De préférence, le composé de formule la est choisi parmi les composés de formule Ia-B, Ia-C, Ia-E, la-F, la-G, la-H et Ia-I et leurs combinaisons.

Avantageusement, le NMN, un de ses dérivés pharmaceutiquement acceptables ou un de ses sels pharmaceutiquement acceptables, peut être utilisé dans le traitement et/ou la prévention d'une douleur articulaire induite par l'activité physique chez des mammifères, de préférence des humains.

Avantageusement, la douleur articulaire concerne la nuque, l'épaule, l'omoplate, le coude, le poignet, les articulations de la main, la hanche, l'articulation sacro-iliaque, le genou, la cheville, les articulations du pied ou leurs combinaisons.

De préférence, la douleur articulaire concerne le genou.

Avantageusement, la douleur articulaire n'est pas due à l'une des pathologies choisies parmi une tumeur, une arthrite, la goutte, une arthrose, une déformation articulaire, une maladie du tissu conjonctif, une dorsopathie, une maladie neurodégénérative, une neuropathie, une maladie génétique, une maladie auto-immune, une myopathie, une osthéopathie, une ostéoporose, une chondropathie, une vasculopathie, une infection virale, une infection fongique, une infection bactérienne, un parasite, l'effet secondaire d'un médicament, un acte chirurgical, un examen médical, une calcification, un traumatisme sauf si induit par une activité physique, une malformation ou leurs combinaisons.

Avantageusement, la douleur articulaire peut être classée dans l'une des catégories M22 à M25, de préférence dans la catégorie M25.5 de la Classification Internationale des Maladies CIM-10.

Dans un mode de réalisation préféré, l'activité physique est la pratique d'un sport.

Avantageusement, le NMN, un de ses dérivés pharmaceutiquement acceptables ou un de ses sels pharmaceutiquement acceptables, peut être administré entre 1 et 10 fois par jour, de préférence entre 1 et 5 fois par jour, de manière davantage préférée entre 1 et 3 fois par jour.

Dans un mode de réalisation préféré, le NMN, un de ses dérivés pharmaceutiquement acceptables ou un de ses sels pharmaceutiquement acceptables, est administré 2 fois par jour.

Avantageusement, le NMN, un de ses dérivés pharmaceutiquement acceptables ou un de ses sels pharmaceutiquement acceptables, peut être utilisé en combinaison avec au moins un autre agent thérapeutique.

Avantageusement, l'au moins un agent thérapeutique peut être un antalgique, un anti-inflammatoire non stéroïdien, la cortisone, un dérivé de la cortisone ou leurs combinaisons.

Avantageusement, l'antalgique peut être choisi parmi le paracétamol, le néfopam, la kétanine, le tetrahydrocannabinol, les cannabinoïdes, l'aspirine, le salicylate de méthyle, diflunisal, salicylamide, la codéine, l'alfentanil, le carfentanil, la dihydrocodéine, la codeinone, le tramadol, la morphine, la morphinone, la buprénorphine, le fentanyl, l'acétyl fentanyl, le remifentanil, le sufentanil, l'héroine, l'hydromorphone, la nalbuphine, l'oxycodone, l'hydroxycodone, l'oxymorphone, le laudanum, la méthadone, la péthidine, le dextropropoxyphène, l'endorphine, le tapentadol, la thébaïne, la vicodin et leurs combinaisons.

Avantageusement, l'anti-inflammatoire non stéroïdien peut être choisi parmi l'ibuprofène, le kétoprofène, le naproxène, le kétorolac, l'alminoprofène, l'acéclofénac, l'acide méfénamique, l'acide niflumique, l'acide tiaprofénique, le célécoxib, le rofécoxib, le valdécoxib, le parécoxib, le dexkétoprofène, le diclofénac, l'étodolac, l'étoricoxib, le fénoprofène, le flurbiprofène, l'indométacine, le méloxicam, le nabumétone, le piroxicam, le sulindac, le ténoxicam, le nimésulide et leurs combinaisons.

Avantageusement, le dérivé de la cortisone peut être choisi parmi la bétaméthasone, la ciprofloxacine, le cortivazol, la dexaméthasone, la fludrocortisone, le methylprednisolone, le prednisolone, la triamcinolone et leurs combinaisons.

Avantageusement, le nicotinamide mononucléotide (NMN), un de ses dérivés pharmaceutiquement acceptables ou un de ses sels pharmaceutiquement acceptables, permet de réduire la raideur articulaire.

Avantageusement, le nicotinamide mononucléotide (NMN), un de ses dérivés pharmaceutiquement acceptables ou un de ses sels pharmaceutiquement acceptables, permet d'améliorer la fonction articulaire.

La présente invention porte également sur une composition comprenant du nicotinamide mononucléotide (NMN), un de ses dérivés pharmaceutiquement acceptables ou un de ses sels pharmaceutiquement acceptables, et au moins un excipient pharmaceutiquement acceptable pour son utilisation dans la prévention et/ou le traitement d'une douleur articulaire induite par l'activité physique, administrée par voie topique.

Avantageusement, la composition selon l'invention peut se présenter sous la forme d'un gel, d'une solution, d'une émulsion eau dans huile, d'une émulsion huile dans eau, d'une huile, d'une crème, d'une pommade ou d'un liniment.

Dans un mode de réalisation préféré, la composition selon l'invention se présente sous la forme d'une émulsion eau dans huile ou d'une émulsion huile dans eau, de manière davantage préférée d'une émulsion huile dans eau.

Dans un mode de réalisation davantage préféré, la composition selon l'invention se présente sous la forme d'un gel hydrophile ou lipophile, de manière encore plus préférée sous la forme d'un gel hydrophile.

Avantageusement, la composition selon l'invention peut être une composition pharmaceutique.

Avantageusement, la composition selon l'invention peut comprendre le NMN, un de ses sels ou un de ses dérivés pharmaceutiquement acceptables, en une quantité comprise entre 0.05% et 15% en poids, de préférence entre 1 à 10% en poids, de manière davantage préférée entre 3 et 5% en poids par rapport au poids total de la composition.

Avantageusement, le NMN, un de ses dérivés pharmaceutiquement acceptables ou un de ses sels pharmaceutiquement acceptables, peut être administré entre 1 et 10 fois par jour, de préférence entre 1 et 5 fois par jour, de manière davantage préférée entre 1 et 3 fois par jour.

Dans un mode de réalisation préféré, le NMN, un de ses dérivés pharmaceutiquement acceptables ou un de ses sels pharmaceutiquement acceptables, peut être administré 2 fois par jour.

Avantageusement, la composition selon l'invention peut en outre comprendre au moins un agent thérapeutique supplémentaire tel que défini ci-dessus pour son utilisation dans la prévention et/ou le traitement d'une douleur articulaire induite par l'activité physique tel qu'exposé ci-dessus.

### DÉFINITIONS

Dans la présente invention, les termes suivants ont la signification suivante.

Sauf indication contraire, la nomenclature des substituants qui ne sont pas explicitement définis dans la présente invention est obtenue en nommant la partie terminale de la fonctionnalité suivie de la fonctionnalité adjacente vers le point d'attache.

« Alkyle » par lui-même ou en tant que partie d'un autre substituant, désigne un radical hydrocarbyle de formule CnH2n+1 dans laquelle n'est un nombre supérieur ou égal à 1. En général, les groupes alkyles de cette invention comprennent de 1 à 12 atomes de carbone, de préférence de 1 à 8 atomes de carbone, plus préférablement de 1 à 6 atomes de carbone, encore plus préférablement de 1 à 2 atomes de carbone. Les groupes alkyles peuvent être linéaires ou ramifiés et peuvent être substitués comme indiqué dans la présente invention. Les alkyles convenant à la mise en oeuvre de l'invention peuvent être choisis parmi methyle, ethyle, n-propyle, i-propyle, n- butyle, i-butyle, s-butyle and t-butyle, pentyle et ses isomères tels que n-pentyle et iso-pentyle, et hexyl eet ses isomères tels que n-hexyle et iso-hexyle, heptyle et ses isomères (par exemple n-heptyle, iso-heptyle), octyle et ses isomères (par exemple n-octyle, iso-octyle), nonyle et ses isomères (par exemple n-nonyle, iso-nonyle), décyle et ses isomères (par exemple n-décyle, iso-décyle), undécyle et ses isomères, dodécyle et ses isomères. De préférence, les groupes alkyles peuvent être choisis parmi methyle, ethyle, n-propyle, i-propyle, n-butyle, i-butyle, s-butyle, t-butyle, n-pentyle, n-hexyle, n-heptyle, n-octyle, n-nonyle et n-decyle. Les groupes alkyles saturés et ramifiés peuvent être choisis, de manière non limitative, parmi isopropyle, sec-butyle, isobutyle, tert-butyle, isopentyle, 2-methylbutyle, 3-methylbutyle, 2-methylpentyle, 3-methylpentyle, 4-methylpentyle, 2-methylhexyle, 3-methylhexyle, 4-methylhexyle, 5-methylhexyle, 2,3-dimethylbutyle, 2,3-dimethylpentyle, 2,4-dimethylpentyle, 2,3-dimethylhexyle, 2,4-dimethylhexyle, 2,5-dimethylhexyle, 2,2-dimethylpentyle, 2,2-dimethylhexyle, 3,3-dimtheylpentyle, 3,3-dimethylhexyle, 4,4-dimethylhexyle, 2-ethylpentyle, 3-ethylpentyle, 2-ethylhexyle, 3-ethylhexyle, 4-ethylhexyle, 2-methyl-2-ethylpentyle, 2-methyl-3-ethylpentyle, 2-methyl-4-ethylpentyle, 2-methyl-2-ethylhexyle, 2-methyl-3-ethylhexyle, 2-methyl-4-ethylhexyle, 2,2-diethylpentyle, 3,3-diethylhexyle, 2,2-diethylhexyle, et 3,3-diethylhexyle. Les groupes alkyle préférés sont les suivants : méthyle, éthyle, n-propyle, i-propyle, n-butyle, i-butyle, s-butyle et t-butyle. Les Cx-Cy-alkyles désignent les groupes alkyles qui comprennent de x à y atomes de carbone.

Lorsque le suffixe "ene" ("alkylène") est utilisé en conjonction avec un groupe alkyle, cela signifie que le groupe alkyle tel que défini ici a deux liaisons simples comme points d'attache à d'autres groupes. Le terme "alkylène" comprend le méthylène, l'éthylène, le méthylméthylène, le propylène, l'éthyléthylène et le 1,2-diméthyléthylène.

Le terme "alcényle" tel qu'il est utilisé ici se réfère à un groupe hydrocarbyle insaturé, qui peut être linéaire ou ramifié, comprenant une ou plusieurs doubles liaisons carbone-carbone. Les groupes alcényles appropriés comprennent entre 2 et 12 atomes de carbone, de préférence entre 2 et 8 atomes de carbone, et encore plus préférablement entre 2 et 6 atomes de carbone. Des exemples de groupes alcényles sont l'éthényle, le 2-propényle, le 2-butényle, le 3-butényle, le 2-pentényle et ses isomères, le 2-hexényle et ses isomères, le 2,4-pentadiényle et les groupes similaires.

Le terme "alcynyle", tel qu'il est utilisé ici, désigne une classe de groupes hydrocarbyle insaturés monovalents, dans lesquels l'insaturation résulte de la présence d'une ou plusieurs triple(s) liaison(s) carbone-carbone. Les groupes alcynyle ont généralement, et de préférence, le même nombre d'atomes de carbone que celui décrit ci-dessus pour les groupes alcényle. Des exemples non limitatifs de groupes alcynyle sont l'éthynyle, le 2-propynyle, le 2-butynyle, le 3-butynyle, le 2-pentynyle et ses isomères, le 2-hexynyle et ses isomères, etc.

« Alcoxy » désigne un groupe alkyle tel que défini ci-dessus, qui est attaché à une autre partie par un atome d'oxygène. Les exemples de groupes alcoxy comprennent les groupes méthoxy, isopropoxy, éthoxy, tert-butoxy, et autres. Les groupes alcoxy peuvent être éventuellement substitués par un ou plusieurs substituants. Les groupes alcoxy inclus dans les composés de cette invention peuvent être éventuellement substitués par un groupe solubilisant.

« Aryle », tel qu'il est utilisé ici, désigne un groupe hydrocarbyle aromatique polyinsaturé ayant un seul cycle (par exemple phényle) ou plusieurs cycles aromatiques fusionnés ensemble (par exemple naphtyle) ou liés par covalence, contenant généralement 5 à 18 atomes, de préférence 5 à 12, de manière davantage préférée de 6 à 10, dont au moins un cycle est aromatique. Le cycle aromatique peut éventuellement comprendre un ou deux cycles supplémentaires (cycloalkyle, hétérocyclyle ou hétéroaryle) qui y sont fusionnés. L'aryle est également destiné à inclure les dérivés partiellement hydrogénés des systèmes carbocycliques énumérés ici. Les exemples d'aryle comprennent le phényle, le biphénylyle, le biphénylényle, le 5- ou 6-tétralinyle, le naphtalène-1- ou -2-yle, le 4-, 5-, 6 ou 7-indényle, le 1- 2-, 3-, 4- ou 5-acénaphtylényle, le 3-, 4- ou 5-acénaphtényle, 1- ou 2-pentalényle, 4- ou 5-indanyle, 5-, 6-, 7- ou 8-tétrahydronaphtyle, 1,2,3,4-tétrahydronaphtyle, 1,4-dihydronaphtyle, 1-, 2-, 3-, 4- ou 5-pyrényle.

Lorsqu'au moins un atome de carbone dans un groupe aryle est remplacé par un hétéroatome, le cycle résultant est appelé ici cycle « hétéroaryle ».

« Alkylaryle » désigne un groupe aryle substitué par un groupe alkyle.

« Acide aminé » désigne un acide carboxylique alpha-aminé, c'est-à-dire une molécule comprenant un groupe fonctionnel acide carboxylique et un groupe fonctionnel amine en position alpha du groupe acide carboxylique, par exemple un acide aminé protéinogène ou un acide aminé non protéinogène.

« Acide aminé protéinogène » désigne un acide aminé qui est incorporé dans les protéines lors de la traduction de l'ARN messager par les ribosomes dans les organismes vivants, c'est-à-dire Alanine (ALA), Arginine (ARG), Asparagine (ASN), Aspartate (ASP), Cystéine (CYS), Glutamate (acide glutamique) (GLU), Glutamine (GLN), Glycine (GLY), Histidine (HIS), Isoleucine (ILE), Leucine (LEU), Lysine (LYS), Méthionine (MET), Phénylalanine (PHE), Proline (PRO), Pyrrolysine (PYL), Sélénocystéine (SEL), Sérine (SER), Thréonine (THR), Tryptophane (TRP), Tyrosine (TYR) ou Valine (VAL).

« Acide aminé non protéinogène » tel qu'il est utilisé ici fait référence à un acide aminé qui n'est pas naturellement codé ou trouvé dans le code génétique d'un organisme vivant. Des exemples non limitatifs d'acide aminé non protéinogène sont l'ornithine, la citrulline, l'argininosuccinate, l'homosérine, l'homocystéine, l'acide cystéine-sulfinique, l'acide 2-aminomuconique, δ-aminolevulinic acid, β-alanine, cystathionine, γ-aminobutyrate, DOPA, 5-hydroxytryptophane, D-sérine, acide iboténique, α-aminobutyrate, 2-aminoisobutyrate, D-leucine, D-valine, D-alanine ou D-glutamate

Le terme "cycloalkyle" tel qu'il est utilisé ici est un groupe alkyle cyclique, c'est-à-dire un groupe hydrocarbyle monovalent, saturé ou insaturé, ayant 1 ou 2 structures cycliques. Le terme "cycloalkyle" inclut les groupes hydrocarbyle monocycliques ou bicycliques. Les groupes cycloalkyle peuvent comprendre 3 atomes de carbone ou plus dans le cycle et généralement, selon la présente invention, comprendre de 3 à 10, plus préférablement de 3 à 8 atomes de carbone et encore plus préférablement de 3 à 6 atomes de carbone. Les exemples de groupes cycloalkyle comprennent, sans s'y limiter, le cyclopropyle, le cyclobutyle, le cyclopentyle, le cyclohexyle, le cyclopropyle étant particulièrement préféré.

Par « excipient pharmaceutiquement acceptable », il est fait référence à un véhicule ou un support inerte utilisé en tant que solvant ou diluant dans lequel le principe actif est formulé et/ou administré, et qui ne produit pas une réaction indésirable, allergique ou autre lorsqu'il est administré à un animal, de préférence un être humain. Cela comprend tous les solvants, milieux de dispersion, revêtements, agents antibactériens et antifongiques, agents isotoniques, retardants d'absorption et autres ingrédients similaires. Pour l'administration humaine, les préparations doivent répondre à des normes de stérilité, de sécurité générale et de pureté, telles que requises par les offices de régulation, tels que par exemple la FDA ou l'EMA. Au sens de l'invention, « excipient pharmaceutiquement acceptable » inclut tous les excipients pharmaceutiquement acceptables ainsi que tous les supports, diluants, et/ou adjuvants pharmaceutiquement acceptables.

« Halogène » ou « halo » signifie fluoro, chloro, bromo ou iodo. Les groupes halo préférés sont le fluoro et le chloro.

« Haloalkyle » seul ou en combinaison, désigne un radical alkyle ayant la signification telle que définie ci-dessus, dans lequel un ou plusieurs atomes d'hydrogène sont remplacés par un halogène tel que défini ci-dessus. Parmi les exemples de tels radicaux halogénoalkyle, on peut citer le chlorométhyle, le 1-bromoéthyle, le fluorométhyle, le difluorométhyle, le trifluorométhyle, le 1,1,1-trifluoroéthyle et les radicaux similaires. Cx-Cy-haloalkyle et Cx-Cy-alkyle désignent des groupes alkyles qui comprennent de x à y atomes de carbone. Les groupes halogénoalkyle préférés sont le difluorométhyle et le trifluorométhyle.

« Hétéroalkyle » désigne un groupe alkyle tel que défini ci-dessus, dans lequel un ou plusieurs atomes de carbone sont remplacés par un hétéroatome choisi parmi les atomes d'oxygène, d'azote et de soufre. Dans les groupes hétéroalkyle, les hétéroatomes sont liés le long de la chaîne alkyle uniquement à des atomes de carbone, c'est-à-dire que chaque hétéroatome est séparé de tout autre hétéroatome par au moins un atome de carbone. Toutefois, les hétéroatomes d'azote et de soufre peuvent éventuellement être oxydés et les hétéroatomes d'azote peuvent éventuellement être quaternisés. Un hétéroalkyle est lié à un autre groupe ou à une autre molécule uniquement par un atome de carbone, c'est-à-dire que l'atome de liaison n'est pas choisi parmi les hétéroatomes inclus dans le groupe hétéroalkyle.

Le terme "hétéroaryle" tel qu'il est utilisé ici, seul ou en tant que partie d'un autre groupe, désigne, sans s'y limiter, des cycles aromatiques de 5 à 12 atomes de carbone ou des systèmes cycliques contenant 1 ou 2 cycles qui sont fusionnés ou liés par covalence, contenant généralement 5 ou 6 atomes ; dont au moins un est aromatique, dans lequel un ou plusieurs atomes de carbone dans un ou plusieurs de ces cycles sont remplacés par des atomes d'oxygène, d'azote et/ou de soufre, les hétéroatomes d'azote et de soufre pouvant éventuellement être oxydés et les hétéroatomes d'azote pouvant éventuellement être quaternisés. Ces cycles peuvent être fusionnés à un cycle aryle, cycloalkyle, hétéroaryle ou hétérocyclyle. Parmi les exemples non limitatifs de tels hétéroaryles, on peut citer furanyle, thiophényle, pyrazolyle, imidazolyle, oxazolyle, isoxazolyle, thiazolyle, isothiazolyle, triazolyle, oxadiazolyle, thiadiazolyle, tétrazolyle, oxatriazolyle, thiatriazolyle, pyridinyle, pyrimidyle, pyrazinyle, pyridazinyle, oxazinyle, dioxinyle, thiazinyle, triazinyle, imidazo [2, 1 -b] [1,3] thiazolyle, thiéno [3 ,2-b] furanyle, thiéno [3 ,2-b] thiophényle, thiéno [2,3-d] [l,3] thiazolyle, thiéno [2,3-d] imidazolyle, tétrazolo [l,5-a] pyridinyle, indolyle, indolizinyle, isoindolyle, benzofuranyle, isobenzofuranyle, benzothiophényle, isobenzothiophényle, indazolyle, benzimidazolyle, 1,3-benzoxazolyle, 1,2- benzisoxazolyle, 2,1-benzisoxazolyle, 1,3-benzothiazolyle, 1,2-benzoisothiazolyle, 2,1-benzoisothiazolyle, benzotriazolyle, 1,2,3-benzoxadiazolyle, 2,1,3- benzoxadiazolyle, 1,2,3-benzothiadiazolyle, 2,1,3-benzothiadiazolyle, thiénopyridinyle, purinyle, imidazo[l,2-a]pyridinyl, 6-oxo-pyridazin-l(6H)-yl, 2- oxopyridin-l(2H)-yl, 6-oxo-pyridazin-l(6H)-yl, 2-oxopyridin-l(2H)-yl, 1,3-benzodioxolyl, quinolinyl, isoquinolinyl, cinnolinyl, quinazolinyl, quinoxalinyl.

Lorsqu'au moins un atome de carbone dans un groupe cycloalkyle est remplacé par un hétéroatome, le cycle résultant est appelé ici "hétérocycloalkyle" ou "hétérocyclyle".

Les termes "hétérocyclyle", "hétérocycloalkyle" ou "hétérocyclo", tels qu'ils sont utilisés ici par eux-mêmes ou en tant que partie d'un autre groupe, désignent des groupes cycliques non aromatiques, totalement saturés ou partiellement insaturés (par exemple, monocyclique de 3 à 7 chaînons, bicyclique de 7 à 11 chaînons ou contenant un total de 3 à 10 atomes de cycle) qui ont au moins un hétéroatome dans au moins un cycle contenant un atome de carbone. Chaque cycle du groupe hétérocyclique contenant un hétéroatome peut avoir 1, 2, 3 ou 4 hétéroatomes choisis parmi les atomes d'azote, d'oxygène et/ou de soufre, où les hétéroatomes d'azote et de soufre peuvent éventuellement être oxydés et les hétéroatomes d'azote peuvent éventuellement être quaternisés. N'importe lequel des atomes de carbone du groupe hétérocyclique peut être substitué par un oxo (par exemple pipéridone, pyrrolidinone). Le groupe hétérocyclique peut être attaché à n'importe quel hétéroatome ou atome de carbone du cycle ou du système cyclique, lorsque la valence le permet. Les cycles des hétérocycles à plusieurs cycles peuvent être fusionnés, pontés et/ou reliés par un ou plusieurs atomes spiro. Les groupes hétérocycliques exemplaires non limitatifs comprennent les groupes oxétanyle, pipéridinyle, azétidinyle, 2-imidazolinyle, pyrazolidinyle, imidazolidinyle, isoxazolinyle, oxazolidinyle, isoxazolidinyle, thiazolidinyle, isothiazolidinyle, pipéridinyle, 3H- indolyle, indolinyle, isoindolinyle, 2-oxopipérazinyle, pipérazinyle, homopipérazinyle, 2-pyrazolinyle, 3-pyrazolinyle, tétrahydro-2H-pyranyle, 2H-pyranyle, 4H-pyranyle, 3,4-dihydro-2H-pyranyle, 3-dioxolanyle, 1,4-dioxanyle, 2, 5-dioximidazolidinyle, 2-oxopipéridinyle, 2-oxopyrrolodinyle, indolinyle, tétrahydropyranyle, tétrahydrofuranyle, tétrahydroquinolinyle, tétrahydroisoquinolin-1-yle, tétrahydroisoquinolin-2-yle, tétrahydroisoquinolin-3-yle, tétrahydroisoquinoléine-4-yl, thiomorpholine-4-yl, oxyde de thiomorpholine-4-ylsulf, thiomorpholine-4-ylsulfone, 1,3- dioxolanyl, 1,4-oxathianyl, 1H-pyrrolizinyl, tétrahydro-l,l-dioxothiophényl, N- formylpipérazinyl et morpholine-4-yl.

Le terme "précurseur" tel qu'il est utilisé ici désigne également les dérivés pharmacologiquement acceptables des composés de formule (I) ou (la) tels que les esters dont le produit de biotransformation in vivo est le médicament actif. Les précurseurs sont caractérisés par une biodisponibilité accrue et sont facilement métabolisés en composés actifs in vivo. Les précurseurs appropriés aux fins de l'invention comprennent notamment les esters carboxyliques, en particulier les esters alkyliques, les esters aryliques, les esters acyloxyalkyliques et les esters carboxyliques de dioxolène ; les esters d'acide ascorbique.

« Pharmaceutiquement acceptable » signifie approuvé ou susceptible d'être approuvé par un organisme de réglementation ou inscrit dans une pharmacopée reconnue pour l'utilisation chez les animaux, et plus préférablement chez l'homme. Il peut s'agir d'une substance qui n'est pas indésirable sur le plan biologique ou autre, c'est-à-dire que la substance peut être administrée à un individu sans provoquer d'effets biologiques indésirables ou d'interactions délétères avec l'un des composants de la composition dans laquelle elle est contenue. De préférence, un sel ou un excipient « pharmaceutiquement acceptable » désigne tout sel ou tout excipient autorisé par la Pharmacopée européenne (notée « Ph. Eur. ») et la pharmacopée américaine (désignée par « United States Pharmacopeia (USP) » en anglais).

Le terme "principe actif" ou « agent thérapeutique » désigne une molécule ou une substance dont l'administration à un sujet ralentit ou arrête la progression, l'aggravation ou la détérioration d'un ou de plusieurs symptômes d'une maladie ou d'un état ; soulage les symptômes d'une maladie ou d'un état ; guérit une maladie ou un état. Selon l'un de ces modes de réalisation, l'ingrédient thérapeutique est une petite molécule, naturelle ou synthétique. Selon une autre, l'ingrédient thérapeutique est une molécule biologique comme par exemple un oligonucléotide, un siRNA, un miRNA, un fragment d'ADN, un aptamère, un anticorps et autres. Les « sels pharmaceutiquement acceptables » comprennent les sels d'addition d'acides et de bases de ces sels. Les sels d'addition d'acide appropriés sont formés à partir d'acides qui forment des sels non toxiques. Il s'agit par exemple de l'acétate, l'adipate, l'aspartate, le benzoate, le bésylate, le bicarbonate/carbonate, le bisulfate/sulfate, le borate, le camsylate, le citrate, le cyclamate, l'édisylate, l'esylate, le formiate, le fumarate, le gluceptate, le gluconate, le glucuronate, l'hexafluorophosphate, l'hibenzate, le chlorhydrate/chlorure, le bromhydrate/bromure, l'hydroiodure/iodure, iséthionate, lactate, malate, maléate, malonate, mésylate, méthylsulfate, naphtylate, 2-napsylate, nicotinate, nitrate, orotate, oxalate, palmitate, pamoate, phosphate/hydrogénophosphate/dihydrogénophosphate, pyroglutamate, saccharate, stéarate, succinate, tannate, tartrate, tosylate, trifluoroacétate et sels de xinofoate. Les sels basiques appropriés sont formés à partir de bases qui forment des sels non toxiques. On peut citer comme exemples les sels d'aluminium, d'arginine, de benzathine, de calcium, de choline, de diéthylamine, de diolamine, de glycine, de lysine, de magnésium, de méglumine, d'olamine, de potassium, de sodium, de trométhamine, de 2-(diéthylamino)éthanol, d'éthanolamine, de morpholine, de 4-(2-hydroxyéthyl)morpholine et de zinc. Des hémisels d'acides et de bases peuvent également être formés, par exemple, des hémisulfates et des sels de calcium chimique. Les sels pharmaceutiquement acceptables préférés sont le chlorhydrate/chlorure, le bromure/hydrobromure, le bisulfate/sulfate, le nitrate, le citrate et l'acétate.

Les sels pharmaceutiquement acceptables peuvent être préparés par une ou plusieurs de ces méthodes :
- en faisant réagir le composé avec l'acide souhaité ;
- en faisant réagir le composé avec la base souhaitée ;
- en éliminant un groupe protecteur labile en milieu acide ou basique d'un précurseur approprié du composé ou en ouvrant le cycle d'un précurseur cyclique approprié, par exemple une lactone ou un lactame, en utilisant l'acide désiré ; ou
- en transformant un sel du composé en un autre par réaction avec un acide approprié ou au moyen d'une colonne d'échange d'ions appropriée.

Toutes ces réactions sont généralement effectuées en solution. Le sel peut précipiter de la solution et être recueilli par filtration ou peut être récupéré par évaporation du solvant. Le degré d'ionisation du sel peut varier de complètement ionisé à presque non ionisé.

« Solvate » est utilisé ici pour décrire un complexe moléculaire comprenant le composé de l'invention et une ou plusieurs molécules de solvant pharmaceutiquement acceptables, par exemple, l'éthanol.

Le terme "substituant" ou "substitué" signifie qu'un radical hydrogène sur un composé ou un groupe est remplacé par tout groupe souhaité qui est substantiellement stable dans les conditions de la réaction sous une forme non protégée ou lorsqu'il est protégé par un groupe protecteur. Les exemples de substituants préférés comprennent, sans s'y limiter, un halogène (chloro, iodo, bromo ou fluoro) ; un alkyle ; un alcényle ; un alcynyle, tel que décrit ci-dessus ; un hydroxy ; un alcoxy ; un nitro ; un thiol ; un thioéther ; une imine ; un cyano ; un amido ; un phosphonato ; une phosphine ; un carboxyle ; un thiocarbonyle ; un sulfonyle ; un sulfonamide ; une cétone ; un aldéhyde ; un ester ; un oxygène (-O) ; un haloalkyle (par ex, trifluorométhyle) ; un cycloalkyle, qui peut être monocyclique ou polycyclique condensé ou non condensé (par exemple, cyclopropyle, cyclobutyle, cyclopentyle ou cyclohexyle), ou un hétérocycloalkyle, qui peut être monocyclique ou polycyclique condensé ou non condensé (par exemple, pyrrolidinyle, pipéridinyle, pipérazinyle, morpholinyle ou thiazinyle), monocyclique ou polycyclique fusionné ou non fusionné, aryle ou hétéroaryle (par exemple, aryle, hétéroaryle, pyrrolidinyle, pipéridinyle, pipérazinyle, morpholinyle ou thiazinyle), monocyclique ou polycyclique fusionné ou non fusionné (par exemple, aryle, hétéroaryle, pyrrolidinyle, pipéridinyle, pipérazinyle, morpholinyle ou thiazinyle), phényle, naphtyle, pyrrolyle, indolyle, furanyle, thiophényle, imidazolyle, oxazolyle, isoxazolyle, thiazolyle, triazolyle, tétrazolyle, pyrazolyle, pyridyle, quinolinyle, isoquinolinyle, acridinyle, pyrazinyle, pyridazinyle, pyrimidinyle, benzimidazolyle, benzothiophényle ou benzofuranyle) ; amino (primaire, secondaire ou tertiaire) ; CO₂CH₃ ; CONH2 ; OCH₂CONH₂ ; NH₂ ; SO₂NH₂ ; OCHF₂ ; CF₃ ; OCF₃ ; et ces groupements peuvent également être éventuellement substitués par une structure ou un pont annulaire fusionné, par exemple -OCH₂O-. Ces substituants peuvent éventuellement être encore substitués par un substituant choisi parmi ces groupes. Dans certaines représentations, le terme "substituant" ou l'adjectif "substitué" désigne un substituant choisi dans le groupe constitué par un alkyle, un alcényle, un alcynyle, un cycloalkyle, un cycloalcényle, un hétérocycloalkyle, un aryle, un hétéroaryle, un arylalkyle, un hétéroarylalkyle, un haloalkyle, - C(O)NR₁₁R₁₂, -NR₁₃C(O)R₁₄, un halo, -OR₁₃, cyano, nitro, un haloalcoxy, -C(O)R₁₃, -NR₁₁R₁₂, -SR₁₃, - C(O)OR'₁₃, -OC(O)R₁₃, -NR₁₃C(O)NR₁₁R₁₂, -OC(O)NR₁₁R₁₂, -NR₁₃C(O)OR₁₄, -S(O)rR13, -NR₁₃S(O)rR₁₄, - OS(O)rR₁₄, S(O)rNR₁₁R₁₂, -O, -S, et -N-R₁₃, où r est 1 ou 2 ; R₁₁ et R₁₂, pour chaque occurrence, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocycloalkyle éventuellement substitué, un aryle éventuellement substitué, un hétéroaryle éventuellement substitué, un arylalkyle éventuellement substitué, ou un hétéroarylalkyle éventuellement substitué ; ou R₁₁ et R₁₂ pris ensemble avec l'azote auquel ils sont attachés sont un hétérocycloalkyle éventuellement substitué ou un hétéroaryle éventuellement substitué ; et R₁₃ et R₁₄ pour chaque occurrence sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocycloalkyle éventuellement substitué, un aryle éventuellement substitué, un hétéroaryle éventuellement substitué, un arylalkyle éventuellement substitué, ou un hétéroarylalkyle éventuellement substitué. Dans certaines variantes, le terme "substituant" ou l'adjectif "substitué" désigne un groupe solubilisant.

Le terme « administration », ou une variante de ce terme (par exemple, « administrer »), signifie fournir le principe actif, seul ou dans le cadre d'une composition pharmaceutiquement acceptable, au patient chez qui/à qui l'état, le symptôme ou la maladie doit être traité ou prévenu.

« Traiter », « soigner » et « traitement », tels qu'ils sont utilisés dans la présente invention, sont censés inclure le soulagement, l'atténuation ou l'ablation d'un état ou d'une maladie et/ou de ses symptômes associés.

« Prévenir », « empêcher » et « prévention », tels qu'ils sont utilisés dans la présente invention, font référence à une méthode permettant de retarder ou d'empêcher l'apparition d'un état ou d'une maladie et/ou de ses symptômes connexes, d'empêcher un patient de contracter un état ou une maladie, ou de réduire le risque qu'un patient contracte un état ou une maladie.

Les liaisons d'un carbone asymétrique peuvent être représentées ici en utilisant un triangle plein ( 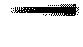 ), un triangle pointillé ( 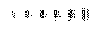 ) ou une ligne en zigzag ( 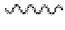 ).

### DESCRIPTION DETAILLEE DE L'INVENTION

La présente invention a pour objet le nicotinamide mononucléotide (NMN), un de ses dérivés pharmaceutiquement acceptables ou un de ses sels pharmaceutiquement acceptables, pour son utilisation par voie topique dans la prévention et/ou le traitement d'une douleur articulaire induite par l'activité physique ainsi que des compositions le comprenant.

Le nicotinamide adénine dinucléotide (NAD) est une coenzyme présente dans toutes les cellules vivantes. Le NAD existe dans la cellule soit sous sa forme oxydée NAD+, soit sous sa forme réduite NADH. Le rôle de NAD est celui d'un transporteur d'électrons intervenant dans les réactions d'oxydoréduction du métabolisme. Le NAD intervient en outre dans de nombreux processus cellulaires tels que la ribosylation de l'ADP dans le cadre de modifications post-traductionnelles des protéines.

Le NAD peut être synthétisé de novo par la cellule à partir d'acides aminés comme le tryptophane ou l'aspartate. Cependant, cette synthèse est marginale car la principale voie de synthèse de NAD est la voie de sauvetage par laquelle la cellule, et principalement le noyau cellulaire, recycle des composés pour reformer le NAD à partir de précurseurs. Les précurseurs du NAD comprennent la niacine, le nicotinamide riboside, le nicotinamide mononucléotide et le nicotinamide.

Le NMN est un des composés permettant la synthèse de NAD par la voie de sauvetage et a pour formule :

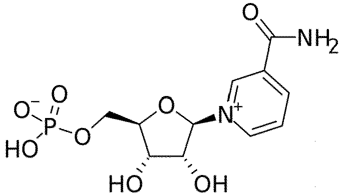

Les inventeurs ont en effet démontré que l'utilisation du NMN, de ses sels et/ou dérivés pharmaceutiquement acceptables et de la composition selon l'invention permettait de soulager les douleurs articulaires résultant de l'activité physique, et notamment de la pratique d'un sport. De préférence, la douleur articulaire est la gonalgie, c'est-à-dire une douleur ressentie au niveau du genou.

Les inventeurs ont en effet démontré que l'utilisation de NMN, d'un de ses dérivés pharmaceutiquement acceptables ou d'un de ses sels pharmaceutiquement acceptables, ainsi que les compositions les comprenant se montre particulièrement efficace pour réduire la douleur articulaire, en particulier la gonalgie, induite par une activité physique, notamment une activité sportive. Plus exactement, les inventeurs ont démontré que l'utilisation de NMN, d'un de ses dérivés pharmaceutiquement acceptables ou d'un de ses sels pharmaceutiquement acceptables, ainsi que les compositions les comprenant permet de réduire la douleur articulaire, en particulier la gonalgie, de manière suffisamment efficace pour éviter le recours aux thérapies conventionnelles.

Selon l'OMS, on entend par activité physique tout mouvement produit par les muscles squelettiques, responsable d'une augmentation de la dépense énergétique. L'activité physique peut être liée à l'activité professionnelle. Les activités physiques de loisirs peuvent inclure le sport mais également les activités réalisées sans encadrement : par exemple, les promenades à pied, à vélo, en trottinette, dans des parcs et espaces verts, à la campagne ; des équipements spécialisés en accès libre peuvent être utilisés pour réaliser des activités physiques (parcours santé aménagés, terrains multisports, espaces de fitness en plein air, pistes cyclables, circuits de randonnée...). Les activités domestiques concernent les activités physiques réalisées chez soi, à l'intérieur ou à l'extérieur (montées et descentes d'escaliers, travaux ménagers - passer l'aspirateur, porter des courses-, bricolage, jardinage). Enfin, dans un mode de réalisation préféré, l'invention trouve son application aux douleurs articulaires résultant de la pratique d'un sport.

Selon l'OMS, pratiquer un sport ou « faire de l'exercice » correspond à une sous-catégorie de l'activité physique plus délibérée, structurée, répétitive, et qui vise à améliorer ou à entretenir un ou plusieurs aspects de la condition physique. Dans le contexte de la présente invention, on désigne indifféremment la pratique d'un sport, le sport et l'activité sportive. Selon l'ANSES, le sport peut également être défini comme une activité physique où les participants adhèrent à un ensemble commun de règles et où un objectif de performance est défini (par exemple : sports collectifs, gymnastique, gymnastique aquatique, course à pied, marche nordique, cyclisme, ski de fond, aviron, natation).

Les recommandations mondiales en matière d'activité physique pour la santé sont formulées par l'OMS en tenant compte de l'âge de la population cible et de l'intensité de l'activité physique. Par exemple, selon les recommandations concernant l'activité physique de l'OMS, les enfants et les adolescents de 5 à 17 ans devraient pratiquer au moins 60 minutes quotidiennes d'activité physique, d'intensité modérée à forte, sachant qu'une activité physique d'une durée supérieure à 60 minutes par jour leur apportera des bienfaits supplémentaires en matière de santé, et ils devraient inclure des activités qui renforcent les muscles et les os à raison d'au moins trois fois par semaine. Selon les recommandations concernant l'activité physiques de l'OMS, les adultes de 18 à 64 ans devraient pratiquer au moins 150 minutes hebdomadaires d'une activité physique d'intensité modérée, ou au moins 75 minutes hebdomadaires d'une activité physique intense, ou une combinaison équivalente d'activité physique d'intensité modérée à forte. Pour en retirer des bienfaits supplémentaires en matière de santé, les adultes devraient porter à 300 minutes par semaine la pratique d'une activité physique d'intensité modérée ou l'équivalent et devraient également pratiquer des activités de renforcement musculaire mettant en jeu les principaux groupes de muscles devraient être pratiquées deux jours par semaine ou plus. Selon les recommandations concernant l'activité physiques de l'OMS, les adultes de 65 ans et plus devraient pratiquer 150 minutes d'activité physique d'intensité modérée par semaine, ou au moins 75 minutes d'activité physique de forte intensité par semaine, ou une combinaison équivalente d'activité physique d'intensité modérée à forte. Pour obtenir des bienfaits supplémentaires en matière de santé, ils devraient porter à 300 minutes par semaine la pratique d'une activité physique d'intensité modérée, ou l'équivalent. Les personnes à mobilité réduite devraient pratiquer une activité physique pour améliorer leur équilibre et prévenir les chutes à raison de trois jours par semaine ou plus. Les activités de renforcement musculaire mettant en jeu les principaux groupes de muscles devraient être pratiquées deux fois par semaine ou plus.

De plus, l'utilisation de NMN, molécule naturellement présente dans le corps, présente de nombreux avantages. Notamment, le NMN ne pose aucun problème de tolérance chez les patients. L'utilisation de NMN et de la composition selon l'invention n'induit en effet aucune allergie. De plus, l'utilisation de NMN et de la composition selon l'invention ne provoque pas les effets secondaires fréquemment rencontrés avec les traitements conventionnels.

Le NMN n'induit notamment aucun phénomène de dépendance physique ou psychologique, contrairement aux antalgiques comprenant de la morphine ou les dérivés de l'opium. Le NMN n'induit en outre aucune fragilité osseuse ou vulnérabilité aux infections comme cela est observé avec l'administration chronique de cortisone ou de ses dérivés. L'utilisation de NMN et de la composition selon l'invention pour prévenir et/ou traiter la douleur articulaire telle que la gonalgie résultant de l'activité physique, et notamment la pratique d'un sport, est donc sûre pour les patients.

Le NMN et la composition selon l'invention peuvent également être utilisés chez l'enfant et l'adulte. Le NMN est en effet bien toléré par les enfants. Dans le contexte de l'invention, on considère qu'un patient est un enfant lorsque son âge est inférieur à 18 ans et qu'il est un adulte à partir de 18 ans. Par conséquent, l'invention trouve également son intérêt pour traiter la gonalgie chez les enfants.

Dans un mode de réalisation particulièrement préféré, le NMN se trouve sous la forme d'un zwitterion. On entend par « zwitterion » une espèce chimique moléculaire possédant des charges électriques de signe opposé et situées, en général, sur des atomes non adjacents de la molécule.

Le dérivé pharmaceutiquement acceptable de NMN est choisi parmi un composé de formule (I) : 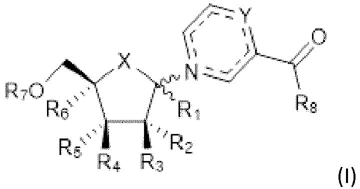 ou un de ses stéréoisomères, un de ses sels, un de ses hydrates, un de ses solvates ou un de ses cristaux pharmaceutiquement acceptable de celui-ci, dans lequel :
- X est choisi parmi O, CH₂, S, Se, CHF, CF₂ et C=CH₂;
- R₁ est choisi parmi H, azido, cyano, alkyle en C₁-C₈, thio-alkyle en C₁-C₈, hétéroalkyle en C₁-C₈ et OR ; dans lequel R est choisi parmi H et alkyle en C₁-C₈ ;
- R₂, R₃, R₄ et R₅ sont choisis indépendamment l'un de l'autre parmi H, halogène, azido, cyano, hydroxyle, alkyle en C₁-C₁₂, thio-alkyle en C₁-C₁₂, hétéroalkyle en C₁-C₁₂, haloalkyle en C₁-C₁₂ et OR ; dans lequel R est choisi parmi H, alkyle en C₁-C₁₂, C(O)(C₁-C₁₂)alkyle, C(O)NH(C₁-C₁₂)alkyle, C(O)O(C₁-C₁₂)alkyle, C(O)aryle, C(O)(C₁-C₁₂)alkyle aryle, C(O)NH(C₁-C₁₂)alkyle aryle, C(O)O(C₁-C₁₂)alkyle aryle et C(O)CHR_{AA}NH2 ; dans lequel R_{AA} est une chaîne latérale choisie parmi un acide aminé protéinogène ;
- R₆ est choisi parmi H, azido, cyano, C₁-C₈ alkyle, C₁-C₈ thio-alkyle, C₁-C₈ hétéroalkyle et OR ; dans lequel R est choisi parmi H et C₁-C₈ alkyle;
- R₇ est choisi parmi H, P(O)R₉R₁₀ et P(S)R₉R₁₀ et 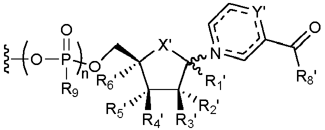 où n est un entier choisi parmi 1, 2 ou 3; dans lequel
- R₉ et R₁₀ sont choisis indépendamment l'un de l'autre parmi OH, OR₁₁, NHR₁₃, NR₁₃R₁₄, un alkyle en C₁-C₈, un alcényle en C₂-C₈, un alcynyl en C₂-C₈, un cycloalkyl en C₃-C₁₀, un aryle en C₅-C₁₂, (C₁-C₈)alkyle aryle, (C₁-C₈)aryle alkyle, (C₁-C₈) heteroalkyle, (C₁-C₈) heterocycloalkyle, un heteroaryle et NHCHR_{A}R_{A}-C(O)R_{I2} ; dans lequel :
   - R₁₁ est choisi parmi un groupe C₁-C₁₀alkyle, C₃₋C₁₀cycloalkyle, C₅-C₁₈ aryle, C₁₋C₁₀ alkylaryle, C₅-C₁₂ aryle substitué, C₁₋C₁₀ heteroalkyle, C₃₋C₁₀ heterocycloalkyle, C₁₋C₁₀ haloalkyle, un heteroaryle, - (CH₂)ₙC(O)(C₁-C₁₅)alkyle, -(CH₂)ₙOC(O)(C₁-C₁₅)alkyle, -(CH₂)ₙOC(O)O(C₁-C₁₅)alkyle, -(CH₂)ₙSC(O)(C₁-C₁₅)alkyle, -(CH₂)ₙC(O)O(C₁-C₁₅)alkyle and -(CH₂)ₙC(O)O(C₁-C₁₅)alkyle aryle; dans lesquels n est un entier choisi de 1 à 8; P(O)(OH)OP(O)(OH)₂; halogène, nitro, cyano, C₁-C₆ alkoxy, C₁-C₆ haloalkoxy, -N(R₁₁ₐ)₂, C₁-C₆ acylamino, -COR_{11b}, -O COR_{11b}; NHSO₂(C₁-C₆ alkyl), - SO₂N(R₁₁ₐ)₂ SO₂ dans lequel chacun de R₁₁ₐ est indépendamment choisi parmi H et un C₁-C₆ alkyle et R_{11b} est indépendamment choisi parmi OH, C₁-C₆ alkoxy, NH₂, NH(C₁-C₆alkyle) ou N(C₁-C₆alkyle)₂ ;
   - R₁₂ est choisi parmi H, C₁₋C₁₀ alkyle, C₂-C₈ alcényle, C₂-C₈ alcynyle, C₁₋C₁₀ haloalkyle, C₃₋C₁₀ cycloalkyle, C₃₋C₁₀ heterocycloalkyle, C₅-C₁₈ aryle, C₁-C₄ alkylaryle and C₅-C₁₂ heteroaryle; dans lesquels lesdits groupes aryle ou heteroaryle sont optionnellement substitué parmi un ou deux groupes choisi parmi un halogène, trifluoromethyl, C₁-C₆ alkyle, C₁-C₆ alkoxy et cyano; et
   - R_{A} et R_{A'} sont indépendamment choisi parmi H, un C₁₋C₁₀ alkyle, C₂-C₁₀ alcényle, C₂-C₁₀ alcynyle, C₃₋C₁₀ cycloalkyle, C₁₋C₁₀ thio-alkyle, C₁₋C₁₀ hydroxylalkyle, C₁₋C₁₀ alkylaryle and C₅-C₁₂ aryle, C₃₋C₁₀ heterocycloalkyle, un heteroaryle, -(CH₂)₃NHC(=NH)NH₂, (1H-indol-3-yl)methyl, (1H-imidazol-4-yl)methyl et une chaine latérale choisie parmi un acide aminé protéinogène ou un acide aminé non protéinogène; dans lesquels lesdits groupes aryle sont optionnellement substitué avec un groupe choisi parmi un hydroxyle, C₁₋C₁₀alkyl, C₁₋C₆ alkoxy, un halogène, un nitro et un cyano; ou
   - R₉ et R₁₀ forment ensemble avec les atomes de phosphore auxquels ils sont attaché un cycle à 6 membres dans lequel -R₉-R₁₀- représente -CH₂-CH₂-CHR-; dans lequel R est choisi parmi H, un groupe (C₅-C₆) aryle et (C₅-C₆) heteroaryle, dans lequel lesdits groupes aryle ou heteroaryle sont optionnellement substitués par un halogène, trifluoromethyl, un C₁-C₆ alkyle, un C₁-C₆ alkoxy et cyano; ou
   R₉ et R₁₀ forment ensemble avec les atomes de phosphore auxquels ils sont attaché un cycle à 6 membres dans lequel -R₉-R₁₀- représente -O-CH₂-CH₂-CHR-O-; dans lequel R est choisi parmi H, un groupe (C₅-C₆) aryle et (C₅-C₆) heteroaryle, dans lequel lesdits groupes aryle ou heteroaryle sont optionnellement substitués par un halogène, trifluoromethyl, un (C₁-C₆) alkyle, un (C₁-C₆) alkoxy et cyano;
   - R₈ est choisi parmi H, OR, NHR₁₃, NR₁₃R₁₄, NH-NHR₁₃, SH, CN, N₃ et halogène ; dans lequel R₁₃ et R₁₄ sont choisis indépendamment l'un de l'autre parmi H, (Cᵢ-C_{g}) alkyle, (C₁-C₈) alkyle aryle, et -CR_{B}R_{C}-C(O)-OR_{D} dans lequel R_{B} et R_{C} sont indépendamment un atome d'hydrogène, un (C₁-C₆) alkyle, un (C₁-C₆) alkoxy, benzyle, indolyle ou imidazolyle, où le (C₁-C₆) alkyle et le (C₁-C₆)) alkoxy peuvent être éventuellement et indépendamment l'un de l'autre substitué par un ou plusieurs des groupes halogène, amino, amido, guanidyle, hydroxyle, thiol ou carboxyle, et le groupe benzyle est éventuellement substitué par un ou plusieurs des groupes halogène ou hydroxyle, ou R_{B} et Reforment ensemble avec l'atome de carbone auquel ils sont attachés un groupe cycloalkyle en C₃-C₆ éventuellement substitué par un ou plusieurs halogènes, amino, amido, guanidyle, hydroxyle, thiol et carboxyle et R_{D} est un hydrogène, un (C₁-C₆) alkyle, un (C₂-C₆) alcényle, un (C₂-C₆) alcynyle ou un (C₃-C₆) cycloalkyle ;
   - Y est choisi parmi CH, CH₂, C(CH₃)₂ et CCH₃ ;
   - -̅ -̅ -̅ représente une simple ou une double liaison selon Y ; et
   - 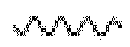 représente l'anomère alpha ou bêta selon la position de R₁
   ou
   un composé de formule (la) : 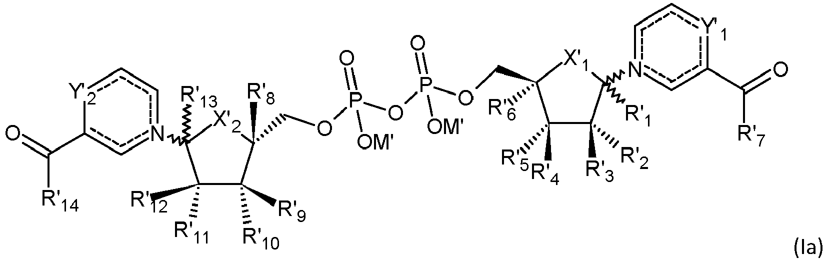
   ou un de ses stéréoisomères, un de ses sels, un de ses hydrates, un de ses solvates ou un de ses cristaux, dans lequel
   X'₁ et X'₂ sont indépendamment choisis parmi O, CH₂, S, Se, CHF, CF₂, et C=CH₂ ;
   R'₁ et R'13 sont indépendamment choisis parmi H, azido, cyano, un alkyl en C1-C8, un thio-alkyl en C1-C8, un hétéroalkyl en C1-C8, et OR, dans lequel R est choisi parmi H et un alkyl en C1-C8,
   R'₂, R'₃, R'₄, R'₅, R'₉, R'₁₀, R'₁₁, R'₁₂ sont indépendamment choisis parmi H, un halogène, un azido, un cyano, un hydroxyl, un alkyl en C₁-C₁₂, un thioalkyl en C₁-C₁₂, un hétéro-alkyl en C₁-C₁₂, un haloalkyl en C₁-C₁₂ et OR, dans lequel R peut être choisi parmi H, un alkyl en C₁-C₁₂, un C(O)( C₁-C₁₂) alkyle, un C(O)NH(C₁-C₁₂) alkyle, un C(O)O(C₁-C₁₂) alkyle, un C(O) aryle, un C(O)( C₁-C₁₂) aryle, un C(O)NH(C₁-C₁₂) alkyle aryle, un C(O)O(C₁-C₁₂) alkyle aryle ou un groupe C(O)CHR_{AA}NH2 dans lequel R_{AA} est une chaine latérale choisi parmi un acide aminé protéinogène ;
   R'₆ et R'₈ sont indépendamment choisis parmi H, un azido, un cyano, un alkyl en C₁-C₈ et OR, dans lequel R est choisi parmi H et un alkyl en C₁-C₈ ;
   R'₇ et R'₁₄ sont indépendamment choisis parmi H, OR, NHR, NRR', NH-NHR, SH, CN, N₃ et un halogène, dans lequel R et R' sont indépendamment choisit parmi H et un (C₁-C₈) alkyle aryle ;
   Y'₁ et Y'₂ sont indépendamment choisis parmi CH, CH₂, C(CH₃)₂ ou CCH₃ ;
   M' est choisi parmi H ou un contre-ion adapté ;
   -̅ -̅ -̅ représente une liaison simple ou double en fonction de Y'₁ et Y'₂ ; et 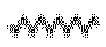 représente un anomère alpha ou béta en fonction de la position de R'₁ et R'₁₃;
   et leurs combinaisons.

Au sens de l'invention, M' peut être un contre-ion interne ou externe.

Dans un premier mode de réalisation préféré, le dérivé pharmaceutiquement acceptable est le composé de formule (I).

Dans une variante du premier mode de réalisation, X représente un oxygène.

Dans une variante du premier mode de réalisation, R₁ et R₆ représentent chacun indépendamment l'un de l'autre un hydrogène.

Dans une variante du premier mode de réalisation, R₂, R₃, R₄ et R₅ représentent chacun indépendamment l'un de l'autre un hydrogène ou un OH.

Dans une variante du premier mode de réalisation, Y représente un CH.

Dans une variante du premier mode de réalisation, Y représente un CH₂.

Dans une variante du premier mode de réalisation, R₇ représente un hydrogène.

Dans une variante du premier mode de réalisation, R₇ représente P(O)(OH)₂.

Dans une variante du premier mode de réalisation,
X représente un oxygène ; et/ou
R₁ et R₆ représente chacun indépendamment un hydrogène ; et/ou
R₂, R₃, R₄ et R₅ représente chacun indépendamment un hydrogène ou R₂, R₃, R₄ et R₅ représente indépendamment OH ; et/ou
Y représente un CH ou un CH₂; et/ou
R₇ représente P(O)R₉R₁₀, dans lequel R₉ et R₁₀ sont indépendamment choisis parmi OH, OR₁₁, NHR₁₃, NR₁₃R₁₄, C₁-C₈alkyle, C₂-C₈ alcényle, C₂-C₈ alcynyle, C₃₋C₁₀cycloalkyle, C₅-C₁₂ aryle, C₁-C₈ aryle alkyle, C₁-C₈ alkyle aryle, C₁-C₈heteroalkyle, C₁₋C₈heterocycloalkyle, heteroaryle et NHCR_{A}R_{A'}-C(O)R₁₂.

Dans une variante particulièrement préférée du premier mode de réalisation, le composé de l'invention est choisi parmi les composés de formule I-B à I-J :

**[Tableau 1]**

| **Composés (anomères)** | **Structure** |
|---|---|
| I-B (alpha) | 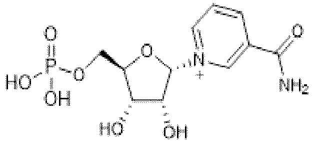 |
| I-C (beta) | 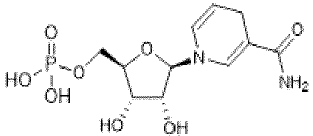 |
| I-D (alpha) | 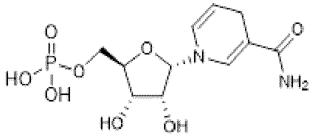 |
| I-E (beta) | 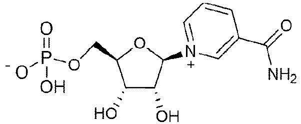 |
| I-F (alpha) | 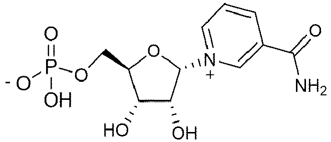 |
| I-G (beta) | 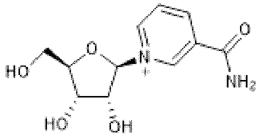 |
| I-H (alpha) | 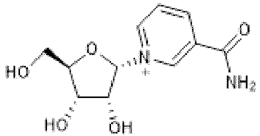 |
| I-I (beta) | 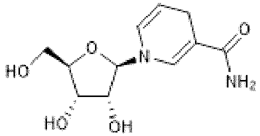 |
| I-J (alpha) | 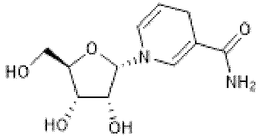 |

Le dérivé pharmaceutiquement acceptable du NMN peut être l'alpha-NMN (composés I-B ou I-F) ou le dihydronicotinamide mononucléotide (NMN-H) (composés I-D ou I-C) et leurs combinaisons.

Dans un deuxième mode de réalisation préféré, le dérivé pharmaceutiquement acceptable est le composé de formule (la).

Dans une variante du deuxième mode de réalisation, X'1 et X'2 représentent chacun indépendamment un oxygène.

Dans une variante du deuxième mode de réalisation, R'7 et R'14 représentent chacun indépendamment un NH₂.

Dans une variante du deuxième mode de réalisation, R'1 et/ou R'13 représentent chacun indépendamment un hydrogène.

Dans une variante du deuxième mode de réalisation, R'6 et/ou R'8 représentent chacun indépendamment un hydrogène.

Dans une variante du deuxième mode de réalisation, R'2, R'3, R'4, R'5, R'9, R'10, R'11 et R'12 représentent chacun indépendamment un hydrogène.

Dans une variante du deuxième mode de réalisation, R'2, R'3, R'4, R'5, R'9, R'10, R'11 et R'12 représentent chacun indépendamment un OH.

Dans une variante du deuxième mode de réalisation, Y'1 et Y'2 représentent chacun indépendamment un CH.

Dans une variante du deuxième mode de réalisation, Y'1 et Y'2 représentent chacun indépendamment un CH2.

Dans une variante du deuxième mode de réalisation, le composé selon l'invention est choisi parmi les composés de formule la-A à Ia-I :

**[Tableau 2]**

| **Composés (anomères)** | **Structure** |
|---|---|
| Ia-A (beta, beta) | 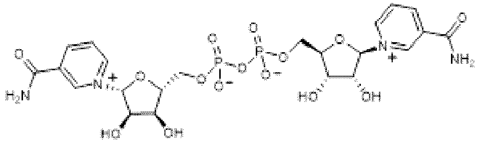 |
| Ia-B (beta, alpha) | 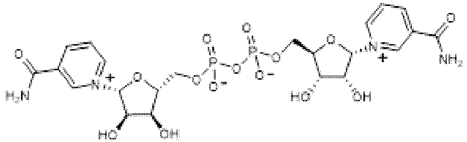 |
| Ia-C (alpha, alpha) | 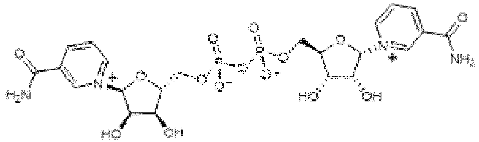 |
| Ia-D (beta, beta) | 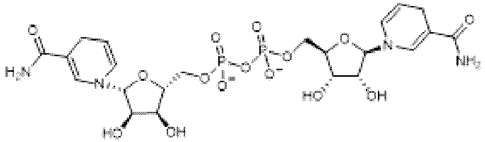 |
| Ia-E (beta, alpha) | 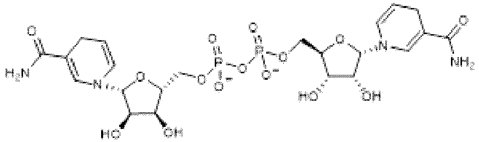 |
| Ia-F (alpha, alpha) | 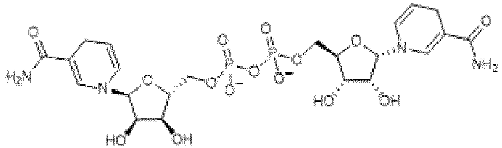 |
| Ia-G (beta, beta) | 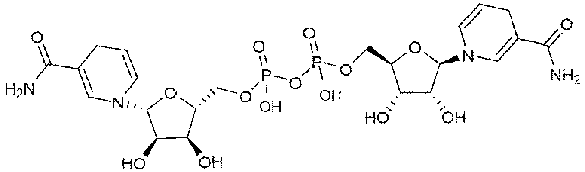 |
| Ia-H (beta, alpha) | 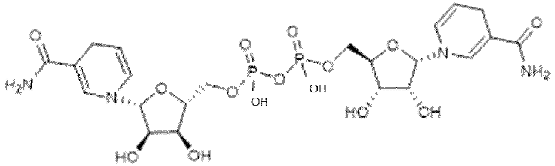 |
| Ia-I (alpha, alpha) | 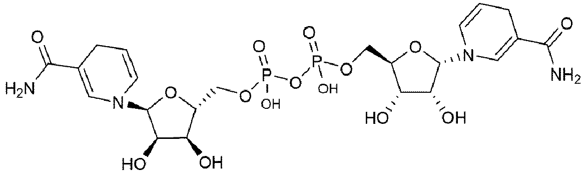 |

De préférence, le composé de formule la est choisi parmi les composés Ia-B, Ia-C, IaE, Ia-F, Ia-G, Ia-H et Ia-I et leurs combinaisons.

Le NMN, d'un de ses dérivés pharmaceutiquement acceptables ou d'un de ses sels pharmaceutiquement acceptables, ainsi que de compositions le comprenant selon l'invention peuvent être utilisés premièrement pour traiter les douleurs articulaires induites par une activité physique, en particulier la pratique d'un sport.

En réduisant le recours aux thérapies utilisées de manière conventionnelles, voire en les remplaçant, la présente invention permet donc d'éviter, ou à tout le moins de réduire, l'utilisation des traitements conventionnels des douleurs articulaires et donc d'éviter, ou à tout le moins de réduire, l'apparition d'effets secondaires liés à ces thérapies.

Dans le contexte de la présente invention, la douleur articulaire résulte uniquement de l'activité physique, de préférence de la pratique d'un sport. De telles douleurs sont localisées ou diffuses et ne nécessitent pas d'intervention chirurgicale pour être soignées. Notamment, de telles douleurs peuvent résulter de la pratique excessive d'une activité physique, et notamment d'un sport. Elles peuvent également résulter d'un faux mouvement ou d'un mauvais équipement lors d'une activité physique, et notamment lors de la pratique d'un sport.

La douleur articulaire peut être classée dans l'une des catégories M22 à M25, de préférence dans la catégorie M25.5 de la Classification Internationale des Maladies CIM-10. Autrement dit, la douleur articulaire peut être classée dans l'une des catégories M22, M23, M24 ou M25, de manière préférée dans la catégorie M25.5 de la Classification Internationale des Maladies CIM-10.

Cependant, dans le contexte de la présente invention, la douleur articulaire n'est pas due à l'une des pathologies choisies parmi une tumeur, une arthrite (classes M00 à M09 et M11 à M14 de la CIM-10), la goutte (M10 de la CIM-10), une arthrose (classes M15 à M19 CIM-10), une déformation articulaire (classes M20 et M21 de la CIM-10), une maladie du tissu conjonctif (M30 à M36 de la CIM-10), une dorsopathie (classes M40 à M54 de la CIM-10), une maladie neurodégénérative, une neuropathie, une maladie génétique, une maladie auto-immune, une myopathie (classes M60-M63 de la CIM-10), une osthéopathie (classes M80 à M90 de la CIM-10), une ostéoporose, une chondropathie (classes M91 à M94 de la CIM-10), une vasculopathie, une infection virale, une infection fongique, une infection bactérienne, un parasite, l'effet secondaire d'un médicament, un acte chirurgical, un examen médical, une calcification, un traumatisme sauf si induit par une activité physique, une malformation ou leurs combinaisons.

On entend par « maladie neurodégénérative » une pathologie progressive qui affecte le cerveau ou plus globalement le système nerveux, entraînant la mort des cellules nerveuses. On entend par « neuropathie » l'ensemble des affections essentiellement du système nerveux périphérique, c'est-à-dire des nerfs moteurs et sensitifs et des membres, des nerfs du système nerveux autonome qui commandent les organes ainsi que plus rarement du système nerveux central. Les neuropathies peuvent être causées, de manière non exhaustive, par un abus d'alcool, des médicaments, le diabète, une infection virale, une blessure ayant endommagé un nerf ou résulter d'une cause inconnue.

On entend par « maladie génétique » une maladie due à une ou plusieurs anomalies sur un ou plusieurs chromosomes qui entraînent un défaut de fonctionnement de certaines cellules de l'organisme. Une maladie génétique peut être due à une délétion ou une mutation sur un gène conduisant à la formation d'une protéine non active ou mal formée.

Une maladie auto-immune est une maladie dans laquelle le système immunitaire est suractivé et s'attaque aux cellules normales de l'Homme.

Une myopathie est une maladie musculaire dégénérative caractérisée par une diminution de la force des muscles atteints et un degré variable d'atrophie. Ce sont le plus souvent des maladies héréditaires. On entend par « chondropathie » une maladie affectant le cartilage et peut être due à des pressions excessives ou mal réparties sur le cartilage. Elle peut se manifester par un ramollissement du cartilage. On entend par « ostéoporose » une raréfaction du tissu osseux et peut faire suite à la ménopause, l'âge ou être idiopathique. On entend par « vasculopathie » est une pathologie affectant les vaisseaux, artériels ou veineux.

On entend par « traumatisme » une fracture, une entorse, une luxation, une subluxation, un déchirement musculaire, une lésion ligamentaire ou tendineuse, ou leurs combinaisons.

Il existe différentes échelles pour mesurer la douleur, et notamment la douleur articulaire. De telles échelles de mesure sont par exemple listées dans le document fourni par la Haute Autorité de Santé (https://www.has-sante.fr/upload/docs/application/pdf/2019-02/liste echelles douleur 2019.pdf).

Parmi ces échelles, on peut notamment citer l'Echelle Visuelle Analogique de douleur, l'échelle numérique, l'échelle verbale simple. Certaines échelles sont notamment développées pour des catégories particulières de la population. Par exemple, les échelles Doloplus et Algoplus sont spécifiquement développées pour les personnes âgées.

Concernant la douleur articulaire, différentes échelles ont été proposées dont l'indice WOMAC (pour *Western Ontario and McMaster Universities Arthritis Index*)*.* Ces échelles peuvent être utilisées pour évaluer tous types de douleurs articulaires, notamment la douleur articulaire du genou, quelle qu'en soit l'étiologie.

Plus exactement, le score de WOMAC est calculé en fonction des réponses aux questions ci-dessous :
1) Douleur (5 items noté de 0-100) : Quelle est l'importance de la douleur ?
   a) Item n°1. Lorsque vous marchez sur une surface plane ?
   b) Item n°2. Lorsque vous montez ou descendez les escaliers ?
   c) Item n°3. La nuit, lorsque vous êtes au lit ?
   d) Item n°4. Lorsque vous vous levez d'une chaise ou vous vous asseyez ?
   e) Item n°5. Lorsque vous vous tenez debout ?
2) Raideur articulaire (2 items noté de 0-100) : Quelle est l'importance de la raideur de votre articulation ?
   a) Item n°1. Lorsque vous vous levez le matin ?
   b) Item n°2. Lorsque vous bougez après vous être assis, couché ou reposé durant la journée ?
3) Fonction articulaire (17 items noté de 0-100) : Quelle est l'importance de la difficulté que vous éprouvez à :
   a) Item n°1. Descendre les escaliers ?
   b) Item n°2. Monter les escaliers ?
   c) Item n°3. Vous relever de la position assise ?
   d) Item n°4. Vous tenir debout ?
   e) Item n°5. Vous pencher en avant ?
   f) Item n°6. Marcher en terrain plat ?
   g) Item n°7. Entrer et sortir d'une voiture ?
   h) Item n°8. Faire vos courses ?
   i) Item n°9. Enfiler collants ou chaussettes ?
   j) Item n°10. Sortir du lit ?
   k) Item n°11. Enlever vos collants ou vos chaussettes ?
   l) Item n°12. Vous étendre sur le lit ?
   m) Item n°13. Entrer ou sortir d'une baignoire ?
   n) Item n°14. Vous asseoir ?
   o) Item n°15. Vous asseoir et vous relever des toilettes ?
   p) Item n°16. Faire le ménage " à fond " de votre domicile ?
   q) Item n°17. Faire l'entretien quotidien de votre domicile ?

Le score total correspond à la moyenne des 24 items. Il en est de même pour le score de chaque domaine. Le score total mesurant la douleur articulaire reflète la composante de ces trois sous-domaines : douleur, raideur et fonction.

Quant au score de Lequesne, il varie de 0 à 22 : plus le score est élevé plus le handicap est extrême voire insupportable. De 8 à 10, le handicap est qualifié d'important et pour un indice supérieur ou égal à 10, le handicap est qualifié de très important.

En particulier, le NMN, d'un de ses dérivés ou de ses sels pharmaceutiquement acceptables, ou les compositions les comprenant peuvent être utilisés pour améliorer les paramètres douleur articulaire, la raideur articulaire et/ou améliorer la fonction articulaire tels qu'évalués par de l'indice WOMAC.

On entend par « fonction articulaire » l'utilisation de l'articulation. Par exemple, l'évaluation de la fonction articulaire d'un genou comprend des questions relatives à la marche sans douleur, la montée d'escaliers, s'asseoir, entrer et sortir d'un véhicule, se pencher en avant, se relever d'une chaise, se tenir debout sans difficulté, faire ses courses, s'habiller, s'allonger sans douleur, faire le ménage.

### Utilisation

Selon la présente invention, le NMN, ses dérivés pharmaceutiquement acceptables ou ses sels pharmaceutiquement acceptables, ainsi que les compositions le comprenant sont utilisés pour prévenir et/ou traiter une douleur articulaire résultant d'une activité physique.

L'activité physique pouvant provoquer une douleur articulaire dépend d'une part de la condition physique de la personne et de la nature de l'activité physique.

L'activité physique peut être soit à but récréatif, à but professionnel ou une pratique sportive. On peut citer à titre d'exemples non limitatifs d'une activité physique à but récréatif la marche, faire les boutiques, le bricolage, monter des meubles, le jardinage, la pêche, la cuisine. L'activité physique pouvant entrainer une douleur articulaire peut par exemple être un travail à la chaine, un travail nécessitant des manipulations physiques tels que kinésithérapeute, ostéopathe, infirmier, aide-soignant, brancardier, pompier, secouriste, technicien de surface, magasinier, vendeur, vigile et autres. L'intensité des différentes formes d'activité physique varie d'une personne à l'autre. Pour qu'elle soit profitable du point de vue de l'endurance cardiorespiratoire, toute activité doit être pratiquée par tranche d'au moins 10 minutes. Dans son Questionnaire Global sur l'Activité Physique (ou « Global Physical Activity Questionnaire » en anglais), l'OMS fournit des exemples d'activité physique intense ou modérée, dans le cadre du travail ou des loisirs. Par exemple, une activité physique de forte intensité nécessite une augmentation conséquente de la respiration ou du rythme cardiaque, comme soulever des charges lourdes, travailler sur un chantier, effectuer du travail de maçonnerie, courir ou jouer au football pendant au moins 10 minutes d'affilée. Une activité physique d'intensité modérée peut être une marche rapide ou soulever une charge légère, nager, faire du vélo ou jouer au volley-ball durant au moins 10 min d'affilée. L'OMS prend également en compte le mode de déplacement d'un endroit à un autre, comme la marche ou le vélo, dans l'activité physique. Une activité physique d'intensité faible peut être par exemple de la marche lente, ou faire la vaisselle, une activité physique d'intensité modérée peut être une marche rapide ou de l'aquagym, et une activité physique d'intensité élevée peut être un jogging ou du tennis.

On distingue 4 niveaux d'intensité d'activité, l'intensité physique étant dépendante du ressenti de chaque individu :
Les activités de faible intensité - comme par exemple, conduire une voiture, faire du rangement, préparer la cuisine - ne provoquent pas d'essoufflement et pas de transpiration. On estime l'effort ressenti à 3 ou 4 sur une échelle de 0 à 10.

Les activités d'intensité modérée - comme par exemple marcher d'un bon pas, courir (moins de 8 km/h), faire du vélo (environ 15 km/h) ou monter les escaliers - provoquent un essoufflement modéré et une légère transpiration. On estime l'effort ressenti à 5 ou 6 sur une échelle de 0 à 10. Tenir une conversation est possible.

Les activités d'intensité élevée - comme par exemple marcher rapidement ou en côte, courir, faire du vélo (environ 20 km/h) ou déplacer des charges lourdes - provoquent un essoufflement marqué et une transpiration abondante. On estime l'effort ressenti à 7 ou 8 sur une échelle de 0 à 10. Tenir une conversation est difficile.

Les activités d'intensité très élevée - comme par exemple la course à pied (de 9 à 18 km/h), faire du cyclisme (plus de 25 km/h), sauter à la corde - provoquent un essoufflement très important et une transpiration très abondante. On estime l'effort ressenti à plus de 8 sur une échelle de 0 à 10. Tenir une conversation est impossible.

De préférence, la douleur articulaire résulte de la pratique d'un sport.

On peut citer, de manière non exhaustive ou limitative, comme exemple de sport provoquant des douleurs articulaires la randonnée, la marche nordique, la marche sportive, la course à pied, l'équitation, l'athlétisme, la danse, la gymnastique, les sports de raquettes, les sports de combat, les sports collectifs, les sports nautiques, les sports extrêmes. A titre d'exemples de sport de raquettes, on peut citer de manière non exhaustive le tennis, le badminton, le squash, le ping-pong et le jeu de paume. A titre d'exemples de sport de combat, les arts martiaux, la boxe française, la boxe anglaise, la boxe thaïlandaise, l'escrime, la lutte, la capoeira, le catch, la savate. A titre d'exemples de sports collectifs, on peut citer de manière non exhaustive le football, le handball, le volley-ball, le basket-ball, le rugby, le polo et le waterpolo. A titre d'exemples de sports nautiques, le kayak, la voile, la planche à voile, la plongée, le canoë. A titre d'exemples de sports extrêmes, on peut citer de manière non exhaustive le parachutisme, le parapente, le kitesurf, le surf, le wakeboard, le canyoning, l'alpinisme et l'escalade.

Le NMN, d'un de ses dérivés ou de ses sels, ainsi que les compositions le comprenant selon l'invention peuvent en effet être utilisés pour soulager les douleurs articulaires, notamment la gonalgie, liées à la pratique sportive, sans avoir recours à l'utilisation de thérapies conventionnelles.

Notamment, le NMN, un de ses dérivés pharmaceutiquement acceptables ou un de ses sels pharmaceutiquement acceptables, ainsi que les compositions les comprenant peuvent être utilisés dans le traitement et/ou la prévention de la douleur articulaire, de préférence la gonalgie, induite par l'activité physique chez des mammifères, de préférence des humains.

La douleur peut concerner toutes les articulations du corps humain et notamment la nuque, l'épaule, l'omoplate, le coude, le poignet, les articulations de la main, la hanche, l'articulation sacro-iliaque, le genou, la cheville, les articulations du pied ou leurs combinaisons.

De préférence, la douleur articulaire concerne le genou (gonalgie). L'utilisation selon la présente invention se trouve en effet particulièrement efficace pour soulager les douleurs du genou.

### Mode d'administration et forme galénique

Le NMN, un de ses sels pharmaceutiquement acceptables, un de ses dérivés pharmaceutiquement acceptables et les compositions le comprenant sont administrés par voie topique. On entend par « voie topique » la forme d'administration d'une composition ou d'une substance sur un point ou une surface externe du corps, telle que la peau ou les muqueuses.

Les formes galéniques convenant à la mise en oeuvre de l'invention sont un gel, une solution, une émulsion eau dans huile, une émulsion huile dans eau, une huile, une crème, une pommade ou un liniment.

On entend par « solution » une forme galénique liquide utilisée pour l'administration d'au moins un principe actif obtenue par la dissolution des différents ingrédients dans une phase liquide et ne formant qu'une phase homogène.

On entend par « émulsion » un mélange hétérogène de deux substances liquides non miscibles, l'une étant dispersée sous forme de petites gouttelettes dans l'autre. Une émulsion permet de mélanger deux liquides qui ne se mélangent pas spontanément (non miscibles), comme l'eau et l'huile. Une émulsion peut être obtenue grâce à des opérations spécifiques (agitation, mélange, ajout de quelques principes actifs). Une émulsion présente un aspect macroscopiquement homogène, mais microscopiquement hétérogène. L'une des substances est en effet dispersée dans la seconde substance sous forme de gouttelettes. Le mélange peut rester stable grâce à un troisième ingrédient appelé émulsifiant (vitesse ou cinétique d'évolution du mélange quasi nulle). Une « émulsion eau dans huile » notée « eau/huile » est composée d'une phase aqueuse dispersée dans une phase huileuse. Une « émulsion huile dans eau » notée « huile/eau » est composée d'une phase huileuse dispersée dans une phase aqueuse.

On entend par « crème » une préparation semi-solide destinée à être administrée en usage topique.

On entend par « pommade » une préparation semi-solide destinée à être appliquée sur la peau.

On appelle « liniment » une forme pharmaceutique liquide, comprenant classiquement des corps gras tels que les huiles, destinée à être utilisé en friction.

On entend par « gel » un matériau solide, éventuellement ductile, constitué d'un réseau tridimensionnel de macromolécules entourées de liquide. Une composition sous forme de gel pénètre bien et rapidement dans la peau et permet en outre d'apporter une sensation de fraicheur anesthésiante.

Dans un mode de réalisation préféré, le gel peut être un gel hydrophobe ou un gel hydrophile. De préférence, le gel est un gel hydrophile.

Dans un mode de réalisation particulièrement préféré, la composition selon l'invention se présente sous la forme d'une émulsion eau dans huile ou d'une émulsion huile dans eau, de manière davantage préférée sous la d'une émulsion huile dans eau (notée huile/eau ou H/E).

Le NMN est très hydrophile et se solubilise donc mieux dans des phases aqueuses.

La composition selon l'invention peut comprendre le NMN, un de ses sels ou un de ses dérivés pharmaceutiquement acceptables, en une quantité comprise entre 0.05% et 15% en poids, de préférence entre 1 à 10% en poids, de manière davantage préférée entre 3 et 5% en poids par rapport au poids total de la composition.

Le NMN, un de ses dérivés pharmaceutiquement acceptables ou un de ses sels pharmaceutiquement acceptables, peut être administré entre 1 et 10 fois par jour, de préférence entre 1 et 5 fois par jour, de manière davantage préférée entre 1 et 3 fois par jour.

Dans un mode de réalisation préféré, le NMN, un de ses dérivés pharmaceutiquement acceptables ou un de ses sels pharmaceutiquement acceptables, ou la composition les comprenant peut être administré 2 fois par jour.

### Combinaisons thérapeutiques

Le NMN, un de ses dérivés pharmaceutiquement acceptables, un de ses sels pharmaceutiquement acceptables et les compositions les comprenant peuvent également être utilisés en combinaison avec au moins un autre agent thérapeutique, en particulier les agents thérapeutiques utilisés de manière conventionnelle pour soulager les douleurs articulaires résultant d'une activité physique.

Parmi les agents thérapeutiques pouvant être combinés à l'invention, on peut citer un antalgique, un anti-inflammatoire non stéroïdien, la cortisone, un dérivé de la cortisone et leurs combinaisons.

L'antalgique peut être choisi parmi le paracétamol, le néfopam, la kétanine, le tetrahydrocannabinol, les cannabinoïdes, l'aspirine, le salicylate de méthyle, diflunisal, salicylamide, la codéine, l'alfentanil, le carfentanil, la dihydrocodéine, la codeinone, le tramadol, la morphine, la buprénorphine, le fentanyl, l'acétyl fentanyl, le remifentanil, le sufentanil, l'héroine, l'hydromorphone, la nalbuphine, l'oxycodone, l'hydroxycodone, l'oxymorphone, le laudanum, la méthadone, la péthidine le dextropropoxyphène, l'endorphine, le tapentadol, la thébaïne, la vicodine et leurs combinaisons.

L'anti-inflammatoire non stéroïdien (AINS) peut être choisi parmi l'ibuprofène, le kétoprofène, le naproxène, le kétorolac, l'alminoprofène l'acéclofénac, l'acide méfénamique, l'acide niflumique, l'acide tiaprofénique, le célécoxib, le rofécoxib, le valdécoxib, le parécoxib, le dexkétoprofène, le diclofénac, l'étodolac, l'étoricoxib, le fénoprofène, le flurbiprofène, l'indométacine, le méloxicam, le nabumétone, le piroxicam, le sulindac, le ténoxicam, le nimésulide et leurs combinaisons.

Le dérivé de la cortisone peut être choisi parmi la bétaméthasone, la ciprofloxacine, le cortivazol, la dexaméthasone, la fludrocortisone, le methylprednisolone, le prednisolone et la triamcinolone et leurs combinaisons.

L'au moins un autre agent thérapeutique supplémentaire peut être administré soit par voie topique, soit par voie injectable, soit par voie orale. Plus exactement, l'au moins un autre agent thérapeutique peut être administré par la voie par laquelle il est administré classiquement.

L'au moins un autre agent thérapeutique peut également être administré concomitamment ou à des moments différents du NMN, un de ses sels ou un de ses dérivés pharmaceutiquement acceptables, ou de la composition selon l'invention.

### Compositions

Les compositions selon l'invention peuvent comprendre le nicotinamide mononucléotide, un de ses dérivés pharmaceutiquement acceptables ou un de ses sels pharmaceutiquement acceptables, et au moins un excipient pharmaceutiquement acceptable pour la prévention et/ou le traitement d'une douleur articulaire induite par l'activité physique, administrée par voie topique.

De telles compositions trouvent notamment leur utilité pour soulager des douleurs articulaires induites par une activité physique, en particulier résultant de la pratique d'un sport comme exposé dans la description.

Dans le contexte de la présente invention, un « excipient » désigne toute substance autre que le NMN dans la composition et n'ayant pas d'effet thérapeutique. L'excipient n'interagit pas sur le plan chimique avec le NMN ou tout autre agent thérapeutique supplémentaire.

L'excipient peut être choisi parmi un agent de charge, un lubrifiant, un parfum, un colorant, un émulsifiant, un agent de compression, un diluant, un conservateur, un gélifiant, un plastifiant, un tensioactif ou leurs combinaisons. L'homme de l'art sait quel excipient choisir en fonction de la forme galénique qu'il aura choisie.

La composition selon l'invention peut être une composition pharmaceutique.

Dans une variante intéressante, la composition selon l'invention peut en outre comprendre au moins un autre agent thérapeutique supplémentaire tel que défini ci-dessus pour son utilisation dans la prévention et/ou le traitement des douleurs articulaires induites par l'activité physique, en particulier le sport.

### PROCEDE DE PREPARATION DES COMPOSES DE FORMULE (I) ET (IA)

Les composés de formule (I) ou de formule (la) peuvent être préparés selon toute méthode bien connue de l'homme du métier.

### Procédé de préparation des composés de formule (I)

Les composés de formule (I) peuvent être préparés selon le procédé décrit dans la demande internationale WO 2017/024255A1 ainsi que selon la méthode décrite ci-dessous.

En particulier, les composés de formule (I) divulgués ici peuvent être préparés comme décrit ci-dessous à partir des substrats A-E. Il sera entendu par un homme du métier que ces schémas réactionnels ne sont en aucune façon limitatifs et que des variations peuvent être faites sans s'écarter de l'esprit et de la portée de la présente invention.

Selon un mode de réalisation, l'invention concerne une méthode de préparation des composés de formule (I) tels que décrits ci-dessus.

La méthode implique dans une première étape la mono-phosphorylation d'un composé de formule (A), en présence de chlorure de phosphoryle et d'un phosphate de trialkyle, pour conduire au phosphorodichloridate de formule (B), 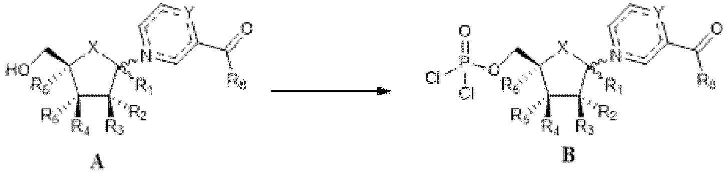 dans lesquels X, R₁, R₂, R₃, R₄, R₅, R₆, R₈, Y, -̅ -̅ -̅ et 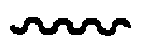 sont tels que définis ci-dessus pour les composés de formule (I).

Dans une seconde étape, le phosphorodichloridate de formule (B) est hydrolysé pour conduire au phosphate de formule (C), 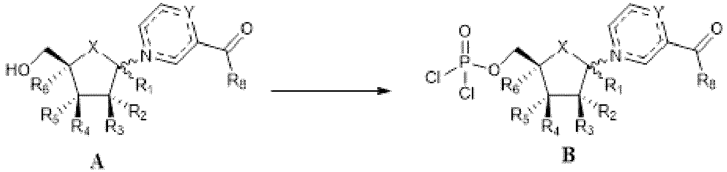
dans lesquels X, R₁, R₂, R₃, R₄, R₅, R₆, R₈, Y, -̅ -̅ -̅ et 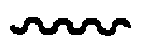 sont tels que définis ci-dessus pour les composés de formule (I).
Selon un mode de réalisation, le composé de formule (A) est synthétisé à l'aide de diverses méthodes connues de l'homme du métier.

Selon un mode de réalisation, le composé de formule (A) est synthétisé par réaction du pentose de formule (D) avec un dérivé azoté de formule (E), dans lequel R, R₂, R₃, R₄, R₅, R₆, R₇, Y sont tels que décrits ci-dessus pour les composés de formule I, conduisant au composé de formule (A-1) qui est ensuite déprotégé sélectivement pour donner le composé de formule (A), 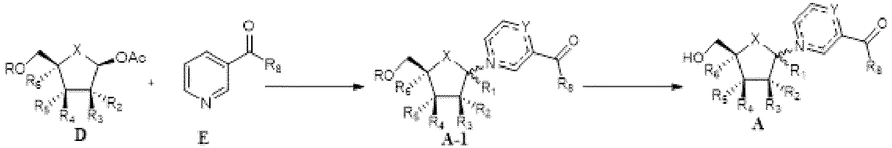 dans lesquels X, R₁, R₂, R₃, R₄, R₅, R₆, R₈, Y, -̅ -̅ -̅ et 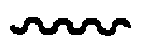 sont tels que définis ci-dessus pour les composés de formule (I).

Selon un mode de réalisation, R est un groupe protecteur approprié connu de l'homme du métier. Dans un mode de réalisation, le groupe protecteur est choisi parmi les triarylméthyles et/ou silyles. Des exemples non limitatifs de triarylméthyle comprennent les groupes trityle, monométhoxytrityle, 4,4'-diméthoxytrityle et 4,4',4"-triméthoxytrityle. Des exemples non limitatifs de groupes silyle comprennent les groupes triméthylsilyle, tert-butyldiméthylsilyle, triisopropylsilyle, tert-butyldiphénylsilyle, tri-iso-propylsilyloxyméthyle et [2-(triméthylsilyl)éthoxy]méthyle.

Selon un mode de réalisation, tout groupe hydroxyle attaché au pentose est protégé par un groupe protecteur approprié connu de l'homme du métier.

Le choix et l'échange des groupes protecteurs relèvent de la compétence de l'homme du métier. Les groupes protecteurs peuvent également être éliminés par des méthodes bien connues de l'homme du métier, par exemple, avec un acide (par exemple, un acide minéral ou organique), une base ou une source de fluorure.

Dans un mode de réalisation préféré, le dérivé azoté de formule (E) est couplé au pentose de formule (D) par une réaction en présence d'un acide de Lewis conduisant au composé de formule (A-1). Des exemples non limitatifs d'acides de Lewis comprennent le TMSOTf, BF₃.OEt₂, TiCl₄ et FeCl₃.

Dans un mode de réalisation, la méthode de la présente invention comprend en outre une étape de réduction du composé de formule (A) par diverses méthodes bien connues de l'homme du métier conduisant au composé de formule (A') dans lequel est CH₂ et R₁, R₂, R₃, R₄, R₅, R₆, R₈, Y, -̅ -̅ -̅ et 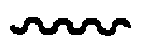 sont tels que définis ci-dessus pour les composés de formule (I).

Dans un mode de réalisation particulier, la présente invention concerne une méthode de préparation des composés de formule I-A, I-C, I-E, I-G.

Dans une première étape, le nicotinamide de formule E est couplé au ribose tétraacétate de formule D par une réaction de couplage en présence d'un acide de Lewis, conduisant au composé de formule A-1 :

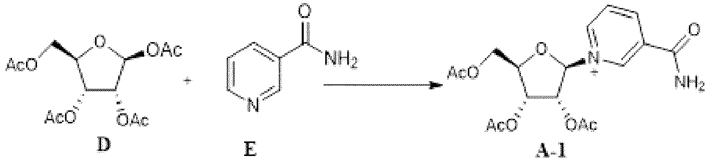

Dans une seconde étape, un traitement ammoniacal du composé de formule A-1 est réalisé, conduisant au composé de formule I-A :

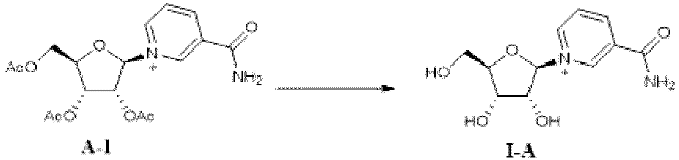

Dans une troisième étape, la mono-phosphorylation du composé de formule I-A, en présence de chlorure de phosphoryle et d'un phosphate de trialkyle, conduit au phosphorodichloridate de formule I-A' :

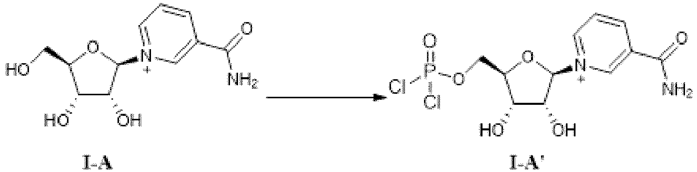

Dans une quatrième étape, le phosphorodichloridate de formule B est hydrolysé pour conduire au composé de formule I-C :

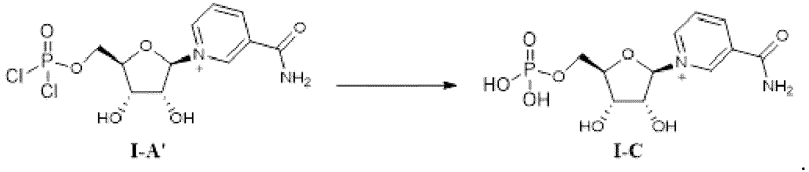

Dans un mode de réalisation, une étape de réduction du composé de formule I-A est réalisée, conduisant au composé de formule I-E.

Le composé de formule I-E est ensuite mono-phosphorylé comme décrit à la quatrième étape et hydrolysé pour conduire au composé de formule I-G.

Selon un mode de réalisation, les composés de formule (I) sont sélectionnés parmi les composés I-A à I-J du tableau ci-dessous :

**[Tableau 1]**

| **Composés (anomères)** | **Structure** |
|---|---|
| I-A (beta) | 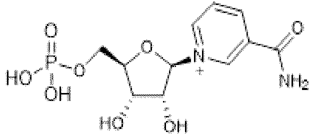 |
| I-B (alpha) | 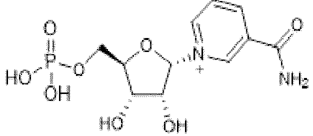 |
| I-C (beta) | 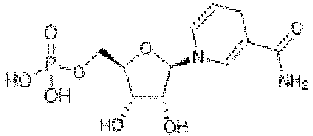 |
| I-D (alpha) | 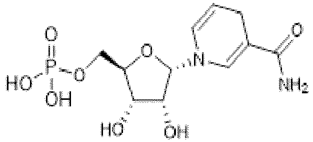 |
| I-E (beta) | 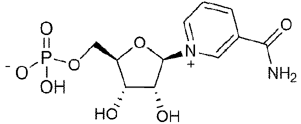 |
| I-F (alpha) | 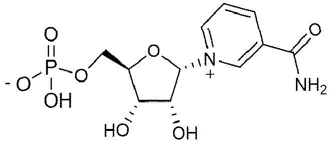 |
| I-G (beta) | 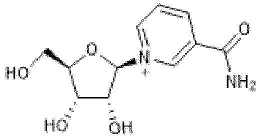 |
| I-H (alpha) | 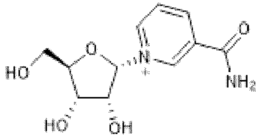 |
| I-I (beta) | 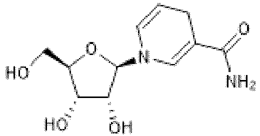 |
| I-J (alpha) | 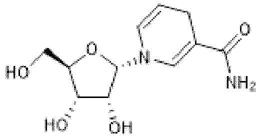 |

De préférence, le composé de formule (I) est choisi parmi le composé I-A, le composé I-B, le composé I-C, , le composé I-D, le composé I-E, le composé I-F, le composé I-G, le composé I-H, le composé I-I, le composé I-J, de préférence le composé I-C, le composé I-D ou le composé I-F, et leurs combinaisons. De manière davantage préférée, le composé de formule (I) est choisi parmi le composé IB, le composé IC, le composé ID, le composé IF et leurs combinaisons.

### Procédé de préparation des dérivés de formule (la)

En particulier, les composés de formule la présentés ici peuvent être préparés comme décrit ci-dessous à partir des substrats X-XIII. Il sera entendu par un homme du métier ordinaire que ces schémas ne sont en aucune façon limitatifs et que des variations de détail peuvent être faites sans s'écarter de l'esprit et de la portée de la présente invention.

Selon un mode de réalisation, l'invention porte sur une méthode de préparation du composé de formule I décrit ci-dessus.

La méthode consiste d'abord à mono-phosphoryler un composé de formule X, en présence de chlorure de phosphoryle dans un phosphate de trialkyle, pour obtenir le composé phosphorodichloridate XI, 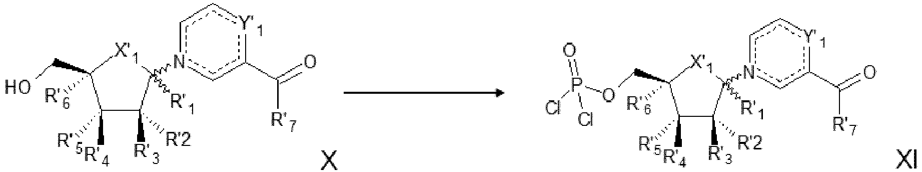 dans laquelle X'₁, R'₁, R'₂, R'₃, R'₄, R'₅, R'₆, R'₇, Y'₁, -̅ -̅ -̅ et 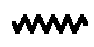 sont tels que définis ci-dessus.

Dans une deuxième étape, l'hydrolyse du phosphorodichloridate XI obtenu dans la première étape donne le composé de phosphate de formule XII, 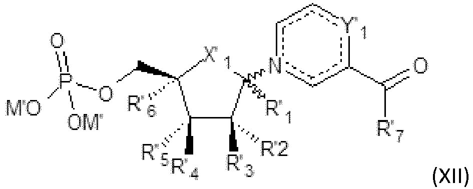 dans laquelle X'₁, R'₁, R'₂, R'₃, R'₄, R'₅, R'₆, R'₇, Y'₁,M', -̅ -̅ -̅, et 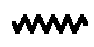 sont tels que définis ci-dessus.

Le composé de phosphate de formule XII obtenu dans la deuxième étape est ensuite mis à réagir avec un composé de phophorodichloridate de formule XIII obtenu comme décrit dans la première étape, 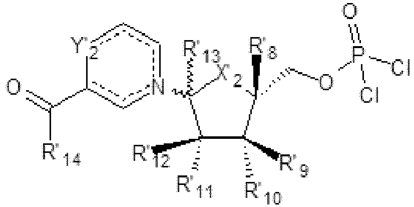 dans laquelle X'₂, R'₈, R'₉, R'₁₀, R'₁₁, R'₁₂, R'₁₃, R'₁₄, Y'₂, -̅ -̅ -̅ et 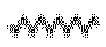 sont tels que décrits ici pour la formule la, pour donner le composé de formule la tel que décrit ici.

Selon un mode de réalisation, le procédé comprend en outre une étape de réduction du composé de formule la, en utilisant diverses méthodes connues des spécialistes, pour donner le composé de formule la, où Y'₁ et Y'₂ sont identiques et représentent chacun CH₂ et où X'₁, X'₂, R'₁, R'₂, R'₃, R'₄, R'₅, R'₆, R'₇, R'₈, R'₉, R'₁₀, R'₁₁, R'₁₂, R'₁₃, R'₁₄, Y'₁, Y'₂ et -̅ -̅ -̅, 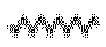 sont tels que décrits ici pour la formule la.

Dans une variante, R est un groupe de protection approprié connu de ceux qui sont habiles dans cet art. Les groupes triarylméthyle et/ou silyle sont des exemples de groupes protecteurs appropriés. Les exemples non limitatifs de triarylméthyle comprennent le trityle, le monométhoxytrityle, le 4,4'-diméthoxytrityle et le 4,4',4"-triméthoxytrityle. Les exemples non limitatifs de groupes silyle comprennent le triméthylsilyle, le tert-butyldiméthylsilyle, le triisopropylsilyle, le tert-butyldiphénylsilyle, le tri-iso-propylsilyloxyméthyle et le [2-(triméthylsilyl)éthoxy]méthyle.

Selon l'une des représentations, tout groupe hydroxy attaché à l'anneau pentose est protégé par un groupe de protection approprié connu des spécialistes de cet art.

La sélection et l'échange des groupes de protection relèvent de la compétence de ceux qui sont compétents dans ce domaine. Tout groupe de protection peut également être éliminé par des méthodes connues dans l'art, par exemple, avec un acide (par exemple, un minéral ou un acide organique), une base ou une source de fluorure.

Selon un mode de réalisation préféré, les dérivés azotés de formule XV sont ajoutés au pentose XIV par une réaction de couplage en présence d'un acide de Lewis pour donner le composé de formule X-1. Parmi les exemples non limitatifs d'acides de Lewis appropriés, on peut citer le TMSOTf, le BF3.OEt2, le TiCl4 et le FeCl3.

Selon une réalisation spécifique, l'invention porte sur une méthode de préparation du composé de formule VIII, 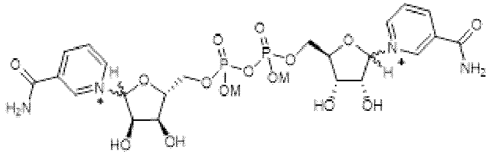 ou leurs sels et/ou solvates pharmaceutiquement acceptables.

Dans une première étape, le nicotinamide de formule XV est ajouté au tétraacétate de ribose XIV, par une réaction de couplage en présence d'un acide de Lewis, pour donner le composé de formule X-1 :

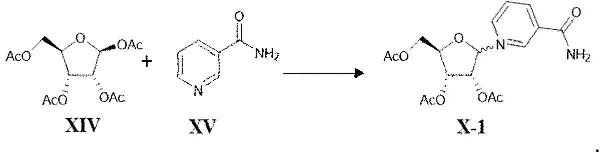

Dans une deuxième étape, un traitement ammoniacal du composé de formule X-1 donne le composé de formule X :

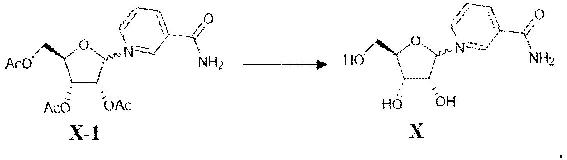

Dans une troisième étape, la mono-phosphorylation d'un composé de formule X, en présence de chlorure de phosphoryle dans un phosphate de trialkyle, donne le composé phosphorodichloridate XI :

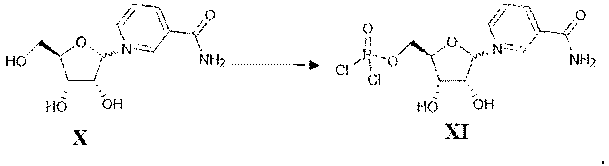

Dans une quatrième étape, le composé phosphorodichloridate XI obtenu dans la troisième étape est partiellement hydrolysé pour donner le composé phosphate de formule XII :

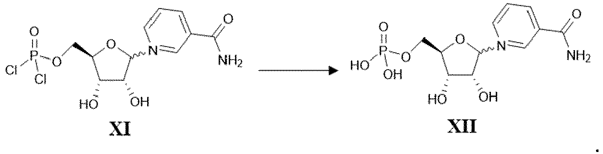

Dans une cinquième étape, on fait ensuite réagir le composé phosphate de formule XII obtenu dans la quatrième étape, avec le composé phosphorodichloridate de formule XI obtenu comme décrit dans la troisième étape, pour obtenir le composé de formule VIII.

Selon une autre concrétisation spécifique, l'invention porte sur une méthode de préparation du composé de formule IX, 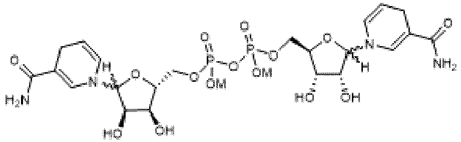 ou leurs sels et/ou solvates pharmaceutiquement acceptables.

D'après une variante, le composé de formule IX est obtenu à partir du composé de formule VIII, préalablement synthétisé comme décrit ci-dessus.

Dans cette réalisation, le composé de formule IX est obtenu en réduisant le composé de formule VIII, à l'aide d'un agent réducteur approprié connu des spécialistes, pour donner le composé de formule IX.

Selon un mode de réalisation, les composés préférés de l'invention sont les composés la-A à Ia-I du tableau 2 :

**[Tableau 2]**

| **Composés (anomères)** | **Structure** |
|---|---|
| Ia-A (beta, beta) | 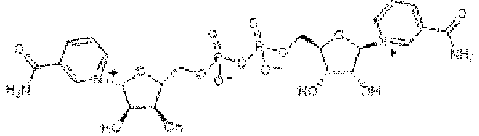 |
| Ia-B (beta, alpha) | 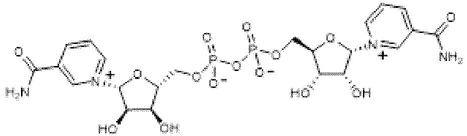 |
| Ia-C (alpha, alpha) | 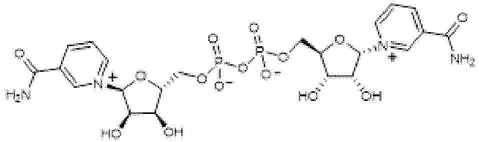 |
| Ia-D (beta, beta) | 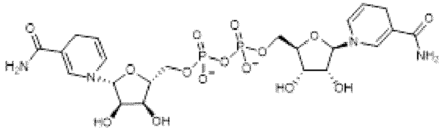 |
| Ia-E (beta, alpha) | 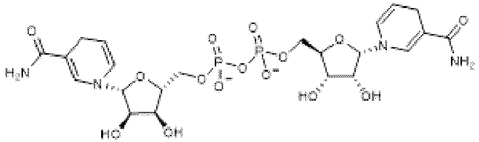 |
| Ia-F (alpha, alpha) | 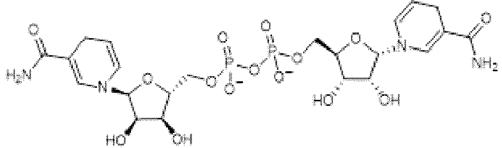 |
| Ia-G (beta, beta) | 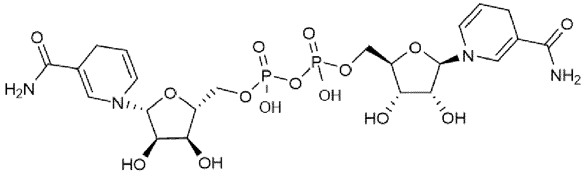 |
| Ia-H (beta, alpha) | 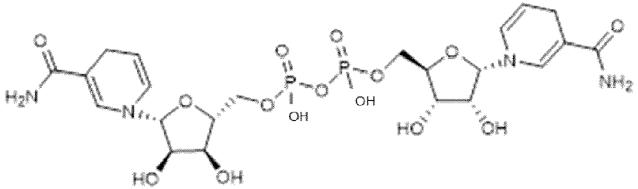 |
| Ia-I (alpha, alpha) | 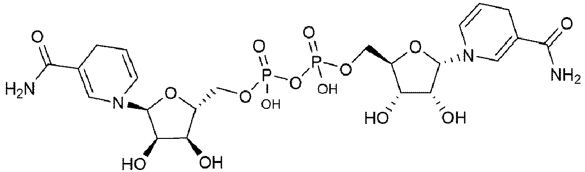 |

De préférence, le composé de formule (Ia) est choisi parmi le composé de formule Ia-B, le composé de formule la-C, le composé de formule la-E, le composé de formule la-F, le composé de formule la-H, le composé de formule Ia-I et le composé de formule la-G ainsi que leurs combinaisons.

### FIGURES

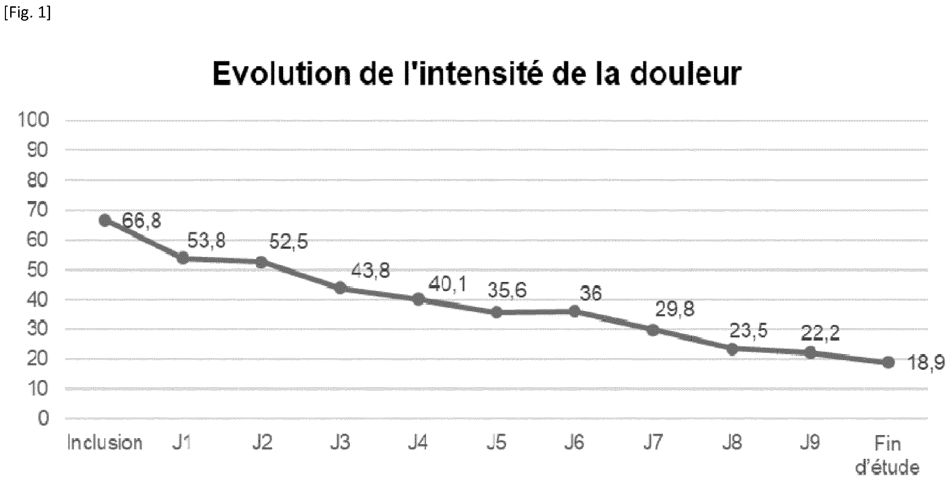

[Fig. 1] est un graphique montrant l'évolution de l'intensité de la douleur.

### EXEMPLE

Dans la suite de la présente description, les exemples sont fournis à titre illustratif de la présente invention et ne visent en aucun cas à en limiter la portée.

Une étude de satisfaction a été menée chez un groupe de 12 volontaires, âgés de 37 ± 9 ans, composé de huit hommes et quatre femmes. L'objectif principal de cette étude est d'évaluer la satisfaction des personnes au regard de l'évolution de leur douleur aiguë au genou lors de l'application matin et/ou soir d'un gel contenant 5% en poids de NMN.

L'IMC moyen des participants était de 26.0 ± 3.9. Plus exactement, six participants avaient un poids normal, quatre participants étaient en surpoids et deux participants étaient en obésité. Aucun de ces patients ne présentait de pathologie chronique telle que l'arthrose, une pathologie inflammatoire altérant leur cartilage, leurs muscles, leurs tendons, leurs ligaments ou leurs os, ou nécessitant un acte chirurgical.

L'ancienneté de l'existence des douleurs au niveau du genou au moment de l'étude était en moyenne de 6.3 ± 1.9 jours, avec une médiane à 7 jours, tandis que les douleurs actuelles des sujets remontaient à 6 ± 2 jours avant l'inclusion. Ces douleurs étaient survenues majoritairement suite à la pratique d'une activité physique (91.7%). Une personne présentait une douleur spontanée. Les 11 autres volontaires présentaient une douleur consécutive à la pratique d'un sport ou d'une activité physique.

Une composition sous forme d'une émulsion huile dans eau comprenant 5% de NMN a été formulée comme suit, les ingrédients étant désignés par leur nom INCI : Aqua, Paraffinum liquidum, Cetyl alcohol, Glyceryl stéarate, Benzyl PCA, Ceteareth-20, Ceteareth-12, Cetyl Palmitate, Cocoglycerides, Cetearyl alcohol, Sodium Hydroxide, NMN.

Les pourcentages massiques sont calculés en rapportant la masse de l'ingrédient par rapport à la masse totale de la composition, puis en multipliant par 100.

L'étude s'est déroulée sur 10 jours. A l'inclusion (J0), les sujets sélectionnés fournissent leurs caractéristiques démographiques (âge, poids, taille), indiquent l'ancienneté et l'intensité de la douleur sur une Echelle Visuelle Analogique, et remplissent les questionnaires WOMAC et Lequesne. Bien qu'aucun des patients ne présente d'arthrose ou d'arthrite, les questionnaires de Lequesne et WOMAC ont été utilisés, afin d'évaluer par différentes manières l'effet du NMN sur la douleur articulaire au genou.

Le score WOMAC est une échelle validée dans l'évaluation de la gonalgie et reconnue par la Haute Autorité de Santé et la Société Française de Rhumatologie. Le score WOMAC se compose de 24 questions, dont les réponses sont des Echelles Visuelles Analogiques variant de 0 (minimum) à 100 (maximum), dans 3 domaines d'évaluation : la douleur avec 5 questions, la raideur avec 2 questions et la fonction quotidienne avec 17 questions. Le score total est calculé par la moyenne des 24 réponses et est évalué de 0 (retentissement nul) à 100 (retentissement maximal). Plus le score WOMAC est élevé, plus le retentissement fonctionnel est important.

La dimension « douleur » du WOMAC était la plus importante à l'inclusion et s'élevait à 57.1 ± 7.4, la dimension « raideur » à 54.3 ± 19.1 et la dimension « fonction » à 41.5 ± 17.9. Le score total WOMAC s'élevait à 45.8 ± 14.3 à l'inclusion.

L'indice algo-fonctionnel de Lequesne est utilisé pour le suivi clinique de la gonalgie et comme un outil d'évaluation pour l'indication d'une prothèse. Le score de Lequesne varie de 0 à 22, plus le score est élevé plus le handicap est extrême voire insupportable. De 8 à 10 le handicap est qualifié d'important et pour un indice supérieur ou égal à 10 une prothèse peut être indiquée.

Le score de Lequesne à l'inclusion était de 8.0 ± 2.4, et 2 des sujets avaient un score supérieur ou égal à 10 (16.7%).

Durant les 9 jours suivants, les personnes remplissent chaque soir l'Echelle Visuelle Analogique de douleur et notent la survenue d'éventuels désagréments ou la prise d'antalgique.

Au 10ème jour, les volontaires remplissent le questionnaire WOMAC, le questionnaire de Lequesne, l'Echelle Visuelle Analogique (EVA) de douleur, l'amélioration perçue de la douleur mesurée par l'indice PGI-I (pour impression globale d'amélioration du patient ou Patient Global Improvement Impression en anglais), la satisfaction à l'égard de l'évolution de la gonalgie sur une échelle de Lickert ainsi que la facilité d'application et de pénétration du gel, l'appréciation de la texture et de l'odeur du gel, son utilisation à nouveau si une douleur identique réapparaissait et la recommandation à des tiers qui présenteraient une douleur de même nature. L'indice PGI-I est un indice permettant d'évaluer la réponse à un traitement. L'échelle de Likert est un outil psychométrique permettant de mesurer une attitude chez des individus, et qui consiste en une ou plusieurs affirmations pour lesquelles la personne interrogée exprime son degré d'accord ou de désaccord.

L'observance du traitement était de 95.8 ± 8.2% en moyenne, au cours de l'étude.

La douleur, mesurée par l'échelle EVA, a diminué de manière constante au cours des 10 jours d'application du produit passant de 66.8 ± 5.0 à l'inclusion à 18.9 ± 18.8 soit une réduction significative de 47.9 ± 20.1 points (p<0.0001). Le délai moyen d'obtention d'une réduction de 50% de la douleur par rapport à l'inclusion était de 5 ± 2 jours. Les résultats exprimés en moyenne et écart-type, jour par jour et pour l'ensemble des volontaires, sont résumés dans le tableau 3 ci-dessous :

**[Tableau 3]**

| Douleur | N | Moyenne | Écart-type | Médiane | Minimum | Maximum |
|---|---|---|---|---|---|---|
| Inclusion J0 | 12 | 66.8 | 5.0 | 65.5 | 61.0 | 80.0 |
| J1 | 12 | 53.8 | 12.8 | 57.5 | 22.0 | 64.0 |
| J2 | 12 | 52.5 | 12.0 | 50.0 | 25.0 | 71.0 |
| J3 | 12 | 43.8 | 9.8 | 43.5 | 29.0 | 60.0 |
| J4 | 12 | 40.1 | 14.1 | 39.0 | 11.0 | 61.0 |
| J5 | 12 | 35.6 | 13.3 | 36.5 | 9.0 | 60.0 |
| J6 | 12 | 36.0 | 15.7 | 34.5 | 12.0 | 72.0 |
| J7 | 12 | 29.8 | 15.7 | 24.5 | 14.0 | 69.0 |
| J8 | 12 | 23.5 | 14.5 | 19.0 | 0.0 | 55.0 |
| J9 | 12 | 22.2 | 17.7 | 19.5 | 0.0 | 51.0 |
| Fin d'étude J10 | 12 | 18.9 | 18.8 | 18.0 | 0.0 | 56.0 |

Ces résultats sont en outre représentés par le graphique de la figure 1. Comme on peut le voir sur la figure 1 et au vu des résultats du tableau 3, la douleur ressentie par les patients a diminué en moyenne de 71.7%.

Au terme de 10 jours d'application de la composition selon l'invention, la dimension « douleur » du WOMAC est passé de 57.1 ± 7.4 à l'inclusion à 14.3 ± 13.8 en fin d'étude soit une réduction significative de 42.7 ± 17.1 points (p<0.0001). Les réductions pour les autres dimensions étaient également significatives passant de 54.3 ± 19.1 à 20.8 ± 21.8 pour la dimension « raideur » du WOMAC (différence de 33.5 ± 27.8, p<0.01) et de 41.5 ± 17.9 à 11.0 ± 15.9 pour la dimension « fonction » du WOMAC (différence de 30.4 ± 21.7, p<0.001). Le score total WOMAC diminuait également significativement passant de 45.8 ± 14.3 à 12.5 ± 15.0 soit une diminution 33.2 ± 20.2 points (p<0.001) et une réduction de 72.7% du score.

Le score algo-fonctionnel de Lequesne a diminué significativement entre l'inclusion et la fin de l'étude diminuant de 8.0 ± 2.4 à 3.8 ± 2.9 (p<0.001) soit une réduction de 52.5% du score. A la fin de l'étude, les deux tiers des patients (66.7%) n'avaient plus de handicap ou seulement un handicap modeste.

A la fin de l'étude, 91.7% des volontaires ont ressenti une amélioration de leur douleur, dont 2 considérablement (16.7%), 8 beaucoup (66.7%) et 1 légèrement (8.3%). 91.7% des volontaires ont été satisfaits de l'évolution de leur gonalgie dont 41.7% très satisfaits.

Sur le plan organoleptique, tous les patients ont trouvé que le gel était facile à appliquer (dont 83.3% très facile), pénétrait facilement dans la peau (dont 75% très facilement), avait une texture agréable (dont 50% très agréable) et une odeur agréable (66.7% agréable et 33.3% assez agréable).

Tous les patients seraient enclins à réutiliser le gel si une douleur articulaire identique survenait (dont 41.7% très certainement) et à le conseiller à un proche qui aurait une douleur identique au niveau du genou (dont 41.7% très certainement).

Aucun volontaire n'a ressenti d'effet secondaire suite à l'utilisation de la composition selon l'invention ni développé d'allergie.

Le NMN, un de ses dérivés pharmaceutiquement acceptables ou un de ses sels pharmaceutiquement acceptables, ainsi que de compositions les comprenant sont donc efficaces pour réduire la douleur articulaire induite par une activité physique ou sportive.

En effet, quelle que soit l'échelle de mesure de la douleur utilisée, une réduction importante de la douleur induite par l'activité physique a été mesurée chez les participants à l'étude.

Bien que ces résultats aient été obtenus par la mesure de la douleur articulaire au niveau du genou, le NMN, un de ses dérivés pharmaceutiquement acceptables ou de ses sels pharmaceutiquement acceptables, ainsi que des compositions le comprenant peuvent être utilisés pour traiter ou prévenir d'autres types de douleurs articulaires telles que, par exemple, des douleurs à l'épaule, au coude, à la cheville, à la nuque ou autres articulations.

## Revendications

1. Nicotinamide mononucléotide (NMN), un de ses dérivés pharmaceutiquement acceptables ou un de ses sels pharmaceutiquement acceptable, pour son utilisation par voie topique dans la prévention et/ou le traitement d'une douleur articulaire induite par l'activité physique, dans lequel le dérivé pharmaceutiquement acceptable du NMN est choisi parmi le dihydronicotinamide mononucléotide (NMN-H), l'alpha-NMN, un composé de formule (I) : 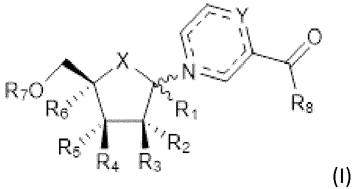 ou un de ses stéréoisomères, un de ses sels, un de ses hydrates, un de ses solvates ou un de ses cristaux pharmaceutiquement acceptable de celui-ci, dans lequel :
- X est choisi parmi O, CH₂, S, Se, CHF, CF₂ et C=CH₂;
- R₁ est choisi parmi H, azido, cyano, alkyle en C₁-C₈, thio-alkyle en C₁-C₈, hétéroalkyle en C₁-C₈ et OR ; dans lequel R est choisi parmi H et alkyle en C₁-C₈ ;
- R₂, R₃, R₄ et R₅ sont choisis indépendamment l'un de l'autre parmi H, halogène, azido, cyano, hydroxyle, alkyle en C₁-C₁₂, thio-alkyle en C₁-C₁₂, hétéroalkyle en C₁-C₁₂, haloalkyle en C₁-C₁₂ et OR ; dans lequel R est choisi parmi H, alkyle en C₁-C₁₂, C(O)(C₁-C₁₂)alkyle, C(O)NH(C₁-C₁₂)alkyle, C(O)O(C₁-C₁₂)alkyle, C(O)aryle, C(O)(C₁-C₁₂)alkyle aryle, C(O)NH(C₁-C₁₂)alkyle aryle, C(O)O(C₁-C₁₂)alkyle aryle et C(O)CHR_{AA}NH₂ ; dans lequel R_{AA} est une chaîne latérale choisie parmi un acide aminé protéinogène ;
- R₆ est choisi parmi H, azido, cyano, C₁-C₈ alkyle, C₁-C₈ thio-alkyle, C₁-C₈ hétéroalkyle et OR ; dans lequel R est choisi parmi H et C₁-C₈ alkyle;
- R₇ est choisi parmi H, P(O)R₉R₁₀ et P(S)R₉R₁₀ et 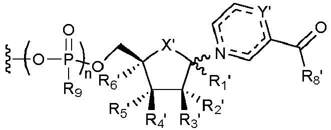 où n est un entier choisi parmi 1, 2 ou 3 ; dans lequel
- R₉ et R₁₀ sont choisis indépendamment l'un de l'autre parmi OH, OR₁₁, NHR₁₃, NR₁₃R₁₄, un alkyle en C₁-C₈, un alcényle en C₂-C₈, un alcynyl en C₂-C₈, un cycloalkyl en C₃-C₁₀, un aryle en C₅-C₁₂, (C₁-C₈)alkyle aryle, (C₁-C₈)aryle alkyle, (C₁-C₈) heteroalkyle, (C₁-C₈) heterocycloalkyle, un heteroaryle et NHCHR_{A}R_{A}-C(O)R_{I2} ; dans lequel :
- R₁₁ est choisi parmi un groupe C₁-C₁₀ alkyle, C₃₋C₁₀ cycloalkyle, C₅-C₁₈ aryle, C₁₋C₁₀ alkylaryle, C₅-C₁₂ aryle substitué, C₁₋C₁₀ heteroalkyle, C₃₋C₁₀ heterocycloalkyle, C₁₋C₁₀ haloalkyle, un heteroaryle, - (CH₂)ₙC(O)(C₁-C₁₅)alkyle, -(CH₂)ₙOC(O)(C₁-C₁₅)alkyle, -(CH₂)ₙOC(O)O(C₁-C₁₅)alkyle, -(CH₂)ₙSC(O)(C₁-C₁₅)alkyle, -(CH₂)ₙC(O)O(C₁-C₁₅)alkyle and -(CH₂)ₙC(O)O(C₁-C₁₅)alkyle aryle; dans lesquels n est un entier choisi de 1 à 8; P(O)(OH)OP(O)(OH)₂, halogène, nitro, cyano, C₁-C₆ alkoxy, C₁-C₆ haloalkoxy, -N(R₁₁ₐ)₂, C₁-C₆ acylamino, -COR_{11b}, -O COR_{11b}; NHSO₂(C₁-C₆ alkyl), - SO₂N(R₁₁ₐ)₂ SO₂ dans lequel chacun de R₁₁ₐ est indépendamment choisi parmi H et un C₁-C₆alkyle et R_{11b} est indépendamment choisi parmi OH, C₁-C₆ alkoxy, NH₂, NH(C₁-C₆alkyle) ou N(C₁-C₆alkyle)₂ ;
- R₁₂ est choisi parmi H, C₁₋C₁₀ alkyle, C₂-C₈ alcényle, C₂-C₈ alcynyle, C₁₋C₁₀ haloalkyle, C₃₋C₁₀ cycloalkyle, C₃₋C₁₀ heterocycloalkyle, C₅-C₁₈ aryle, C₁-C₄ alkylaryle and C₅-C₁₂ heteroaryle; dans lesquels lesdits groupes aryle ou heteroaryle sont optionnellement substitué parmi un ou deux groupes choisi parmi un halogène, trifluoromethyl, C₁-C₆ alkyle, C₁-C₆ alkoxy et cyano; et
- R_{A} et R_{A'} sont indépendamment choisi parmi H, un C₁₋C₁₀alkyle, C₂-C₁₀ alcényle, C₂-C₁₀ alcynyle, C₃₋C₁₀ cycloalkyle, C₁₋C₁₀ thio-alkyle, C₁₋C₁₀ hydroxylalkyle, C₁₋C₁₀ alkylaryle and C₅-C₁₂ aryle, C₃₋C₁₀ heterocycloalkyle, un heteroaryle, -(CH₂)₃NHC(=NH)NH₂, (1H-indol-3-yl)methyl, (1H-imidazol-4-yl)methyl et une chaine latérale choisie parmi un acide aminé protéinogène ou un acide aminé non protéinogène; dans lesquels lesdits groupes aryle sont optionnellement substitué avec un groupe choisi parmi un hydroxyle, C₁₋C₁₀alkyl, C₁₋C₆ alkoxy, un halogène, un nitro et un cyano; ou
- R₉ et R₁₀ forment ensemble avec les atomes de phosphore auxquels ils sont attaché un cycle à 6 membres dans lequel -R₉-R₁₀- représente -CH₂-CH₂-CHR-; dans lequel R est choisi parmi H, un groupe (C₅-C₆) aryle et (C₅-C₆) heteroaryle, dans lequel lesdits groupes aryle ou heteroaryle sont optionnellement substitués par un halogène, trifluoromethyl, un C₁-C₆ alkyle, un C₁-C₆ alkoxy et cyano; ou
R₉ et R₁₀ forment ensemble avec les atomes de phosphore auxquels ils sont attaché un cycle à 6 membres dans lequel -R₉-R₁₀- représente -O-CH₂-CH₂-CHR-O-; dans lequel R est choisi parmi H, un groupe (C₅-C₆) aryle et (C₅-C₆) heteroaryle, dans lequel lesdits groupes aryle ou heteroaryle sont optionnellement substitués par un halogène, trifluoromethyl, un (C₁-C₆) alkyle, un (C₁-C₆) alkoxy et cyano;
- R₈ est choisi parmi H, OR, NHR₁₃, NR₁₃R₁₄, NH-NHR₁₃, SH, CN, N₃ et halogène ;
- dans lequel R₁₃ et R₁₄ sont choisis indépendamment l'un de l'autre parmi H, (C₁-C₈) alkyle et (C₁-C₈) alkyle aryle, et -CR_{B}R_{C}-C(O)-OR_{D} dans lequel R_{B} et R_{C} sont indépendamment un atome d'hydrogène, un (C₁-C₆) alkyle, un (C₁-C₆) alkoxy, benzyle, indolyle ou imidazolyle, où le (C₁-C₆) alkyle et le (C₁-C₆)) alkoxy peuvent être éventuellement et indépendamment l'un de l'autre substitué par un ou plusieurs des groupes halogène, amino, amido, guanidyle, hydroxyle, thiol ou carboxyle, et le groupe benzyle est éventuellement substitué par un ou plusieurs des groupes halogène ou hydroxyle, ou R_{B} et Reforment ensemble avec l'atome de carbone auquel ils sont attachés un groupe cycloalkyle en C₃-C₆ éventuellement substitué par un ou plusieurs halogènes, amino, amido, guanidyle, hydroxyle, thiol et carboxyle et R_{D} est un hydrogène, un (C₁-C₆) alkyle, un (C₂-C₆) alcényle, un (C₂-C₆) alcynyle ou un (C₃-C₆) cycloalkyle ;
- Y est choisi parmi CH, CH₂, C(CH₃)₂ et CCH₃ ;
- -̅ -̅ -̅ représente une simple ou une double liaison selon Y ; et
- 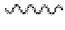 représente l'anomère alpha ou bêta selon la position de R₁ ou
un composé de formule (la) : 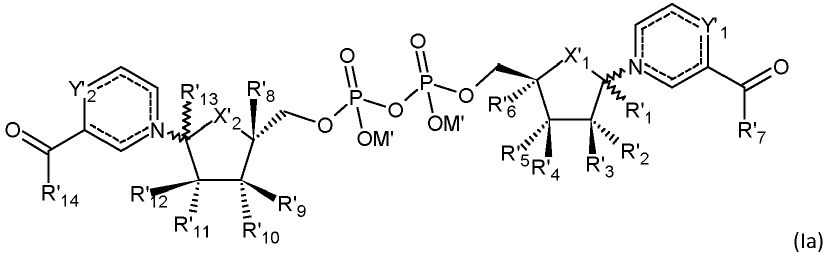 ou un de ses stéréoisomères, un de ses sels, un de ses hydrates, un de ses solvates ou un de ses cristaux, dans lequel
- X'₁ et X'₂ sont indépendamment choisis parmi O, CH₂, S, Se, CHF, CF₂, et C=CH₂ ;
- R'₁ et R'13 sont indépendamment choisis parmi H, azido, cyano, un alkyl en C1-C8, un thio-alkyl en C1-C8, un hétéroalkyl en C1-C8, et OR, dans lequel R est choisi parmi H et un alkyl en C1-C8,
- R'₂, R'₃, R'₄, R'₅, R'₉, R'₁₀, R'₁₁, R'₁₂ sont indépendamment choisis parmi H, un halogène, un azido, un cyano, un hydroxyl, un alkyl en C₁-C₁₂, un thioalkyl en C₁-C₁₂, un hétéro-alkyl en C₁-C₁₂, un haloalkyl en C₁-C₁₂ et OR, dans lequel R peut être choisi parmi H, un alkyl en C₁-C₁₂, un C(O)( C₁-C₁₂) alkyle, un C(O)NH(C₁-C₁₂) alkyle, un C(O)O(C₁-C₁₂) alkyle, un C(O) aryle, un C(O)( C₁-C₁₂) aryle, un C(O)NH(C₁-C₁₂) alkyle aryle, un C(O)O(C₁-C₁₂) alkyle aryle ou un groupe C(O)CHR_{AA}NH2 dans lequel R_{AA} est une chaine latérale choisi parmi un acide aminé protéogène ;
- R'₆ et R'₈ sont indépendamment choisis parmi H, un azido, un cyano, un alkyl en C₁-C₈ et OR, dans lequel R est choisi parmi H et un alkyl en C₁-C₈ ;
- R'₇ et R'₁₄ sont indépendamment choisis parmi H, OR, NHR, NRR', NH-NHR, SH, CN, N₃ et un halogène, dans lequel R et R' sont indépendamment choisit parmi H et un (C₁-C₈) alkyle aryle ;
- Y'1 et Y'2 sont indépendamment choisis parmi CH, CH₂, C(CH₃)₂ ou CCH₃ ;
- M' est choisi parmi H ou un contre-ion adapté ;
- - - - représente une liaison simple ou double en fonction de Y'1 et Y'2 ; et
- 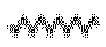 représente un anomère alpha ou béta en fonction de la position de R'₁ et R'₁₃ ; et leurs combinaisons.

2. Nicotinamide mononucléotide (NMN), un de ses dérivés pharmaceutiquement acceptables ou un de ses sels pharmaceutiquement acceptables, pour son utilisation selon la revendication 1 dans lequel le dérivé pharmaceutiquement acceptable du NMN est choisi parmi le composé I-B, le composé I-C, , le composé I-D, le composé I-E, le composé I-F, le composé I-G, le composé I-H, le composé I-I, le composé I-J, de préférence le composé IB, le composé IC, le composé ID, le composé IF et leurs combinaisons.

3. Nicotinamide mononucléotide (NMN), un de ses dérivés pharmaceutiquement acceptables ou un de ses sels pharmaceutiquement acceptables, pour son utilisation selon la revendication 1 ou 2 est choisi parmi les composés Ia-A à Ia-I, de préférence parmi le composé de formule la-B, le composé de formule la-C, le composé de formule la-E, le composé de formule la-F, le composé de formule la-H, le composé de formule Ia-I et le composé de formule la-G.

4. Nicotinamide mononucléotide (NMN), un de ses dérivés pharmaceutiquement acceptables ou un de ses sels pharmaceutiquement acceptables, pour son utilisation selon l'une des revendications précédentes dans laquelle la douleur articulaire concerne la nuque, l'épaule, l'omoplate, le coude, le poignet, les articulations de la main, la hanche, l'articulation sacro-iliaque, le genou, la cheville, les articulations du pied ou leurs combinaisons, de préférence le genou.

5. Nicotinamide mononucléotide (NMN), un de ses dérivés pharmaceutiquement acceptables ou un de ses sels pharmaceutiquement acceptables, pour son utilisation selon l'une des revendications précédentes dans laquelle l'activité physique est la pratique d'un sport.

6. Nicotinamide mononucléotide (NMN), un de ses dérivés pharmaceutiquement acceptables ou un de ses sels pharmaceutiquement acceptables, pour son utilisation selon l'une des revendications précédentes dans laquelle il peut être utilisé en combinaison avec au moins un autre agent thérapeutique.

7. Nicotinamide mononucléotide (NMN), un de ses dérivés pharmaceutiquement acceptables ou un de ses sels pharmaceutiquement acceptables, pour son utilisation selon l'une des revendications précédentes dans laquelle la douleur articulaire n'est pas due à l'une des pathologies choisies parmi une tumeur, une arthrite, la goutte, une arthrose, une déformation articulaire, une maladie du tissu conjonctif, une dorsopathie, une maladie neurodégénérative, une neuropathie, une maladie génétique, une maladie auto-immune, une myopathie, une ostéopathie, une ostéoporose, une chondropathie, une vasculopathie, une infection virale, une infection fongique, une infection bactérienne, un parasite, l'effet secondaire d'un médicament, un acte chirurgical, un examen médical, une calcification, un traumatisme sauf si induit par une activité physique, une malformation et leurs combinaisons.

8. Composition comprenant du nicotinamide mononucléotide (NMN), un de ses dérivés pharmaceutiquement acceptables ou un de ses sels pharmaceutiquement acceptables, et au moins un excipient pharmaceutiquement acceptable pour son utilisation par voie topique dans la prévention et/ou le traitement d'une douleur articulaire induite par l'activité physique, dans laquelle le dérivé pharmaceutiquement acceptable est choisi parmi parmi le dihydronicotinamide mononucléotide (NMN-H), l'alpha-NMN, un composé de formule (I) : 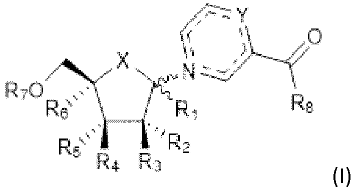 ou un de ses stéréoisomères, un de ses sels, un de ses hydrates, un de ses solvates ou un de ses cristaux pharmaceutiquement acceptable de celui-ci, dans lequel :
- X est choisi parmi O, CH₂, S, Se, CHF, CF₂ et C=CH₂;
- R₁ est choisi parmi H, azido, cyano, alkyle en C₁-C₈, thio-alkyle en C₁-C₈, hétéroalkyle en C₁-C₈ et OR ; dans lequel R est choisi parmi H et alkyle en C₁-C₈ ;
- R₂, R₃, R₄ et R₅ sont choisis indépendamment l'un de l'autre parmi H, halogène, azido, cyano, hydroxyle, alkyle en C₁-C₁₂, thio-alkyle en C₁-C₁₂, hétéroalkyle en C₁-C₁₂, haloalkyle en C₁-C₁₂ et OR ; dans lequel R est choisi parmi H, alkyle en C₁-C₁₂, C(O)(C₁-C₁₂)alkyle, C(O)NH(C₁-C₁₂)alkyle, C(O)O(C₁-C₁₂)alkyle, C(O)aryle, C(O)(C₁-C₁₂)alkyle aryle, C(O)NH(C₁-C₁₂)alkyle aryle, C(O)O(C₁-C₁₂)alkyle aryle et C(O)CHR_{AA}NH₂ ; dans lequel R_{AA} est une chaîne latérale choisie parmi un acide aminé protéinogène ;
- R₆ est choisi parmi H, azido, cyano, C₁-C₈ alkyle, C₁-C₈ thio-alkyle, C₁-C₈ hétéroalkyle et OR ; dans lequel R est choisi parmi H et C₁-C₈ alkyle;
- R₇ est choisi parmi H, P(O)R₉R₁₀ et P(S)R₉R₁₀ et 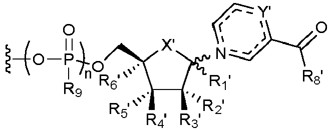 où n est un entier choisi parmi 1, 2 ou 3; dans lequel
- R₉ et R₁₀ sont choisis indépendamment l'un de l'autre parmi OH, OR₁₁, NHR₁₃, NR₁₃R₁₄, un alkyle en C₁-C₈, un alcényle en C₂-C₈, un alcynyl en C₂-C₈, un cycloalkyl en C₃-C₁₀, un aryle en C₅-C₁₂, (C₁-C₈)alkyle aryle, (C₁-C₈)aryle alkyle, (C₁-C₈) heteroalkyle, (C₁-C₈) heterocycloalkyle, un heteroaryle et NHCHR_{A}R_{A'}C(O)R₁₂ ; dans lequel :
- R₁₁ est choisi parmi un groupe C₁-C₁₀ alkyle, C₃-C₁₀ cycloalkyle, C₅-C₁₈ aryle, C₁-C₁₀ alkylaryle, C₅-C₁₂ aryle substitué, C₁-C₁₀ heteroalkyle, C₃-C₁₀ heterocycloalkyle, C₁-C₁₀ haloalkyle, un heteroaryle,-(CH₂)ₙC(O)(C₁-C₁₅)alkyle, -(CH₂)ₙOC(O)(C₁-C₁₅)alkyle, -(CH₂)ₙOC(O)O(C₁-C₁₅)alkyle, -(CH₂)ₙSC(O)(C₁-C₁₅)alkyle, -(CH₂)ₙC(O)O(C₁-C₁₅)alkyle and -(CH₂)ₙC(O)O(C₁-C₁₅)alkyle aryle; dans lesquels n est un entier choisi de 1 à 8; P(O)(OH)OP(O)(OH)₂, halogène, nitro, cyano, C₁-C₆ alkoxy, C₁-C₆ haloalkoxy, -N(R₁₁ₐ)₂, C₁-C₆ acylamino, -COR_{11b}, -O COR_{11b}; NHSO₂(C₁-C₆ alkyl), - SO₂N(R₁₁ₐ)₂ SO₂ dans lequel chacun de R₁₁ₐ est indépendamment choisi parmi H et un C₁-C₆alkyle et R_{11b} est indépendamment choisi parmi OH, C₁-C₆ alkoxy, NH₂, NH(C₁-C₆alkyle) ou N(C₁-C₆alkyle)₂ ;
- R₁₂ est choisi parmi H, C₁-C₁₀ alkyle, C₂-C₈ alcényle, C₂-C₈ alcynyle, C₁-C₁₀ haloalkyle, C₃-C₁₀ cycloalkyle, C₃-C₁₀ heterocycloalkyle, C₅-C₁₈ aryle, C₁-C₄ alkylaryle and C₅-C₁₂ heteroaryle; dans lesquels lesdits groupes aryle ou heteroaryle sont optionnellement substitué parmi un ou deux groupes choisi parmi un halogène, trifluoromethyl, C₁-C₆ alkyle, C₁-C₆ alkoxy et cyano; et
- R_{A} et R_{A'} sont indépendamment choisi parmi H, un C₁₋C₁₀ alkyle, C₂-C₁₀ alcényle, C₂-C₁₀ alcynyle, C₃-C₁₀ cycloalkyle, C₁-C₁₀ thio-alkyle, C₁-C₁₀ hydroxylalkyle, C₁-C₁₀ alkylaryle and C₅-C₁₂ aryle, C₃-C₁₀ heterocycloalkyle, un heteroaryle, -(CH₂)₃NHC(=NH)NH₂, (1H-indol-3-yl)methyl, (1H-imidazol-4-yl)methyl et une chaine latérale choisie parmi un acide aminé protéinogène ou un acide aminé non protéinogène; dans lesquels lesdits groupes aryle sont optionnellement substitué avec un groupe choisi parmi un hydroxyle, C₁₋C₁₀alkyl, C₁₋C₆ alkoxy, un halogène, un nitro et un cyano; ou
- R₉ et R₁₀ forment ensemble avec les atomes de phosphore auxquels ils sont attaché un cycle à 6 membres dans lequel -R₉-R₁₀- représente -CH₂-CH₂-CHR-; dans lequel R est choisi parmi H, un groupe (C₅-C₆) aryle et (C₅-C₆) heteroaryle, dans lequel lesdits groupes aryle ou heteroaryle sont optionnellement substitués par un halogène, trifluoromethyl, un C₁-C₆ alkyle, un C₁-C₆ alkoxy et cyano; ou
R₉ et R₁₀ forment ensemble avec les atomes de phosphore auxquels ils sont attaché un cycle à 6 membres dans lequel -R₉-R₁₀- représente -O-CH₂-CH₂-CHR-O-; dans lequel R est choisi parmi H, un groupe (C₅-C₆) aryle et (C₅-C₆) heteroaryle, dans lequel lesdits groupes aryle ou heteroaryle sont optionnellement substitués par un halogène, trifluoromethyl, un (C₁-C₆) alkyle, un (C₁-C₆) alkoxy et cyano;
- R₈ est choisi parmi H, OR, NHR₁₃, NR₁₃R₁₄, NH-NHR₁₃, SH, CN, N₃ et halogène ;
- dans lequel R₁₃ et R₁₄ sont choisis indépendamment l'un de l'autre parmi H, (C₁-C₈) alkyle et (C₁-C₈) alkyle aryle, et -CR_{B}R_{C}-C(O)-OR_{D} dans lequel R_{B} et R_{C} sont indépendamment un atome d'hydrogène, un (C₁-C₆) alkyle, un (C₁-C₆) alkoxy, benzyle, indolyle ou imidazolyle, où le (C₁-C₆) alkyle et le (C₁-C₆)) alkoxy peuvent être éventuellement et indépendamment l'un de l'autre substitué par un ou plusieurs des groupes halogène, amino, amido, guanidyle, hydroxyle, thiol ou carboxyle, et le groupe benzyle est éventuellement substitué par un ou plusieurs des groupes halogène ou hydroxyle, ou R_{B} et R_{C} forment ensemble avec l'atome de carbone auquel ils sont attachés un groupe cycloalkyle en C₃-C₆ éventuellement substitué par un ou plusieurs halogènes, amino, amido, guanidyle, hydroxyle, thiol et carboxyle et R_{D} est un hydrogène, un (C₁-C₆) alkyle, un (C₂-C₆) alcényle, un (C₂-C₆) alcynyle ou un (C₃-C₆) cycloalkyle ;
- Y est choisi parmi CH, CH₂, C(CH₃)₂ et CCH₃ ;
- -̅ -̅ -̅ représente une simple ou une double liaison selon Y ; et
- 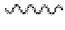 représente l'anomère alpha ou bêta selon la position de R₁ ou
un composé de formule (la) : 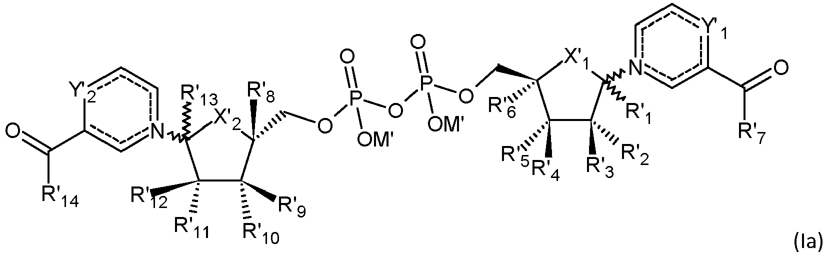 ou un de ses stéréoisomères, un de ses sels, un de ses hydrates, un de ses solvates ou un de ses cristaux, dans lequel
- X'₁ et X'₂ sont indépendamment choisis parmi O, CH₂, S, Se, CHF, CF₂, et C=CH₂ ;
- R'₁ et R'13 sont indépendamment choisis parmi H, azido, cyano, un alkyl en C1-C8, un thio-alkyl en C1-C8, un hétéroalkyl en C1-C8, et OR, dans lequel R est choisi parmi H et un alkyl en C1-C8,
- R'₂, R'₃, R'₄, R'₅, R'₉, R'₁₀, R'₁₁, R'₁₂ sont indépendamment choisis parmi H, un halogène, un azido, un cyano, un hydroxyl, un alkyl en C₁-C₁₂, un thioalkyl en C₁-C₁₂, un hétéro-alkyl en C₁-C₁₂, un haloalkyl en C₁-C₁₂ et OR, dans lequel R peut être choisi parmi H, un alkyl en C₁-C₁₂, un C(O)( C₁-C₁₂) alkyle, un C(O)NH(C₁-C₁₂) alkyle, un C(O)O(C₁-C₁₂) alkyle, un C(O) aryle, un C(O)( C₁-C₁₂) aryle, un C(O)NH(C₁-C₁₂) alkyle aryle, un C(O)O(C₁-C₁₂) alkyle aryle ou un groupe C(O)CHR_{AA}NH2 dans lequel R_{AA} est une chaine latérale choisi parmi un acide aminé protéogène ;
- R'₆ et R'₈ sont indépendamment choisis parmi H, un azido, un cyano, un alkyl en C₁-C₈ et OR, dans lequel R est choisi parmi H et un alkyl en C₁-C₈ ;
- R'₇ et R'₁₄ sont indépendamment choisis parmi H, OR, NHR, NRR', NH-NHR, SH, CN, N₃ et un halogène, dans lequel R et R' sont indépendamment choisit parmi H et un (C₁-C₈) alkyle aryle ;
- Y'1 et Y'2 sont indépendamment choisis parmi CH, CH₂, C(CH₃)₂ ou CCH₃ ;
- M' est choisi parmi H ou un contre-ion adapté ;
- - - - représente une liaison simple ou double en fonction de Y'1 et Y'2 ; et
- 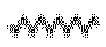 représente un anomère alpha ou bêta en fonction de la position de R'₁ et R'₁₃ ; et leurs combinaisons.

9. Composition selon la revendication 8 comprenant le nicotinamide mononucléotide (NMN), un de ses dérivés pharmaceutiquement acceptables ou un de ses sels pharmaceutiquement acceptables, en une quantité comprise entre 0.05% et 15% en poids, de préférence entre 1 à 10% en poids, de manière davantage préférée entre 3 et 5% en poids par rapport au poids total de la composition.

10. Composition selon la revendication 8 ou 9 se présentant sous la forme d'une émulsion eau dans huile ou d'une émulsion huile dans eau, de préférence sous la forme d'une émulsion huile dans eau.

11. Composition selon l'une des revendications 8 à 10 comprenant en outre au moins un agent thérapeutique supplémentaire.

12. Composition selon la revendication 11 dans laquelle l'au moins un agent thérapeutique supplémentaire est choisi parmi un antalgique, un anti-inflammatoire non stéroïdien, la cortisone, un dérivé de la cortisone ou leurs combinaisons.

13. Composition selon la revendication 12 dans laquelle le dérivé pharmaceutiquement acceptable est choisi parmi le composé I-B, le composé I-C, , le composé I-D, le composé I-E, le composé I-F, le composé I-G, le composé I-H, le composé I-I, le composé I-J, de préférence le composé IB, le composé !C, le composé ID, le composé IF, le composé Ia-A, le composé de formule Ia-B, le composé de formule Ia-C, le composé de formule Ia-E, le composé de formule Ia-F, le composé de formule Ia-H, le composé de formule Ia-I, le composé de formule Ia-G et leurs combinaisons.

## Patentansprüche

1. Nicotinamidmononukleotid (NMN), ein pharmazeutisch annehmbares Derivat davon oder ein pharmazeutisch annehmbares Salz davon zur topischen Verwendung bei der Prävention und/oder Behandlung von Gelenkschmerzen, die durch körperliche Aktivität induziert werden, wobei das pharmazeutisch annehmbare Derivat von NMN ausgewählt ist aus Dihydronicotinamidmononukleotid (NMN-H), alpha-NMN, einer Verbindung von Formel (I): 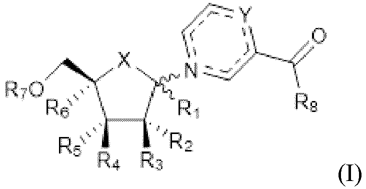 oder einem Stereoisomer davon, einem Salz davon, einem Hydrat davon, einem Solvat davon oder einem pharmazeutisch annehmbaren Kristall davon, wobei:
- X ausgewählt ist aus O, CH₂, S, Se, CHF, CF₂ und C=CH₂;
- R₁ ausgewählt ist aus H, Azido, Cyano, C₁-C₈-Alkyl, C₁-C₈-Thioalkyl, C₁-C₈-Heteroalkyl und OR; wobei R ausgewählt ist aus Hund C₁-C₈-Alkyl;
- R₂, R₃, R₄ und R₅ unabhängig voneinander ausgewählt sind aus H, Halogen, Azido, Cyano, Hydroxyl, C₁-C₁₂-Alkyl, C₁-C₁₂-Thioalkyl, C₁-C₁₂-Heteroalkyl, C₁-C₁₂ Halogenalkyet OR; wobei R ausgewählt ist aus H, C₁-C₁₂-Alkyl, C(O)(C₁-C₁₂)-Alkyl, C(O)NH(C₁-C₁₂)-Alkyl, C(O)O(C₁-C₁₂)-Alkyl, C(O)-Aryl, C(O)( C₁-C₁₂)-Alkylaryl, C(O)NH(C₁-C₁₂)-Alkylaryl, C(O)O(C₁-C₁₂)-Alkylaryl und C(O)CHR_{AA}NH₂; wobei R_{AA} eine Seitenkette ist, die ausgewählt ist aus einer proteinogenen Aminosäure;
- R₆ ausgewählt ist aus H, Azido, Cyano, C₁-C₈-Alkyl, C₁-C₈-Thioalkyl, C₁-C₈-Heteroalkyl und OR; wobei R ausgewählt ist aus Hund C₁-C₈-Alkyl;
- R₇ ausgewählt ist aus H, P(O)R₉R₁₀ und P(S)R₉R₁₀ und 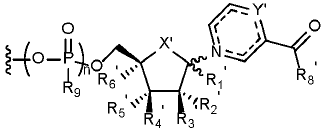 wobei n eine ganze Zahl ist, ausgewählt aus 1, 2 oder 3; wobei:
- R₉ und R₁₀ unabhängig voneinander ausgewählt sind aus OH, OR₁₁, NHR₁₃, NR₁₃R₁₄, C₁-C₈-Alkyl, C₂-C₈-Alkenyl, C₂-C₈-Alkinyl, C₃-C₁₀-Cycloalkyl, C₅-C₁₂-Aryl (C₁-C₈)-Alkylaryl, (C₁-C₈)-Arylalkyl, (C₁-C₈)-Heteroalkyl, (C₁-C₈)-Heterocycloalkyl, Heteroaryl und NHCHR_{A}R_{A'}C(O)R₁₂; wobei:
- R₁₁ ausgewählt ist aus einer Gruppe C₁-C₁₀-Alkyl, C₃-C₁₀-Cycloalkyl, C₅-C₁₈-Aryl, C₁-C₁₀-Alkylaryl, substituiertem C₅-C₁₂-Aryl, C₁-C₁₀-Heteroalkyl, C₃-C₁₀-Heterocycloalkyl, C₁-C₁₀-Haloalkyl, einem Heteroaryl,-(CH₂)ₙC(O)(C₁-C₁₅)-Alkyl, -(CH₂)ₙOC(O)(C₁-C₁₅)-Alkyl,-(CH₂)ₙOC(O)O(C₁-C₁₅)-Alkyl, -(CH₂)ₙSC(O)(C₁-C₁₅)-Alkyle,-(CH₂)nC(O)O(C₁-C₁₅)-Alkyle und -(CH₂)ₙC(O)O(C₁-C₁₅)-Alkylaryl, wobei n eine ganze Zahl ist, ausgewählt aus 1 bis 8; P(O)(OH)OP(O)(OH)₂, Halogen, Nitro, Cyano, C₁-C₆-Alkoxy, C₁-C₆-Halogenalkoxy, -N(R₁₁ₐ)₂, C₁-C₆-Acylamino, -COR_{11b}, -O COR_{11b}; NHSO₂(C₁-C₆-Alkyl), -SO₂N(R₁₁ₐ)₂ SO2, wobei jedes von R₁₁ₐ jeweils unabhängig ausgewählt ist aus H und C₁₋C₆-Alkyl und R_{11b} unabhängig ausgewählt ist aus OH, C₁-C₆-Alkoxy, NH₂, NH(C₁-C₆-Alkyl ) oder N(C₁-C₆-Alkyl)₂,
- R₁₂ ausgewählt ist aus H, C₁-C₁₀-Alkyl, C₂-C₈-Alkenyl, C₂-C₈-Alkinyl, C₁-C₁₀-Haloalkyl, C₃-C₁₀-Cycloalkyl, C₃-C₁₀-Heterocycloalkyl, C₅-C₁₈-Aryl, C₁-C₄-Alkylaryl und C₅-C₁₂-Heteroaryl, wobei die Aryl- oder Heteroarylgruppe optional mit einer oder zwei Gruppen substituiert sind, die ausgewählt sind aus Halogen, Trifluormethyl, C₁-C₆-Alkyl, C₁-C₆-Alkoxy und Cyano; und
- R_{A} und R_{A'} unabhängig voneinander ausgewählt sind aus H, C₁-C₁₀-Alkyl, C₂-C₁₀-Akenyl, C₂-C₁₀-Alkinyl, C₃-C₁₀-Cycloalkyl, C₁-C₁₀-Thioalkyl, C₁-C₁₀-Hydroxylalkyl, C₁-C₁₀-Alkylaryl und C₅-C₁₂-Aryl, C₃-C₁₀-Heterocycloalkyl, einem Heteroaryl, -(CH₂)₃NHC(=NH)NH₂, (1H-Indol-3-yl)methyl, (1H-Imidazol-4-yl)methyl und einer Seitenkette, die ausgewählt ist aus einer proteinogenen Aminosäure oder einer nicht-proteinogenen Aminosäure; wobei die Arylgruppen optional mit einer Gruppe substituiert sind, die ausgewählt ist aus Hydroxyl, C₁-C₁₀-Alkyl, C₁-C₆-Alkoxy, Halogen, Nitro und Cyano; oder
- R₉ und R₁₀ zusammen mit den Phosphoratomen, an die sie gebunden sind, einen 6-gliedrigen Ring bilden, wobei -R₉-R₁₀- -CH₂CH₂CHR-darstellt; wobei R ausgewählt ist aus H, (C₅-C₆)-Aryl und (C₅-C₆)-Heteroaryl, worin die Aryl- oder Heteroarylgruppen optional substituiert sind mit Halogen, Trifluormethyl, C₁-C₆-Alkyl, C₁-C₆-Alkoxy und Cyano; oder
R₉ und R₁₀ zusammen mit den Phosphoratomen, an die sie gebunden sind, einen 6-gliedrigen Ring bilden, wobei -R₉-R₁₀- -O-CH₂-CH₂-CHR-O-darstellt; wobei R ausgewählt ist aus H, (C₅-C₆)-Aryl und (C₅-C₆)-Heteroaryl, wobei die Aryl- oder Heteroarylgruppen optional substituiert sind mit Halogen, Trifluormethyl, (C₁-C₆)-Alkyl, (C₁-C₆)-Alkoxy und Cyano;
- R₈ ausgewählt ist aus H, OR, NHR₁₃, NR₁₃R₁₄, NH-NHR₁₃, SH, CN, N₃ und Halogen; wobei:
- R₁₃ und R₁₄ unabhängig voneinander ausgewählt sind aus H, (C₁-C₈)-Alkyl und (C₁-C₈)-Alkylaryl ausgewählt sind, und -CR_{B}R_{C}-C(O)-OR_{D}, wobei R_{B} und Rc unabhängig Wasserstoff, (C₁-C₆)-Alkyl, (C₁-C₆)-Alkoxy, Benzyl, Indolyl oder Imidazolyl sind, wobei das (C₁-C₆)-Alkyl und das (C₁-C₆)-Alkoxy optional und unabhängig mit einer oder mehreren der Gruppen Halogen, Amino, Amido, Guanidyl, Hydroxyl substituiert sein können, Thiol oder Carboxyl substituiert ist und die Benzylgruppe optional mit einer oder mehreren der Halogen- oder Hydroxylgruppen substituiert ist, oder R_{B} und Rc zusammen mit dem Kohlenstoffatom, an das sie gebunden sind, eine C₃-C₆-Cycloalkylgruppe bilden, die optional mit einem oder mehreren Halogenen, Amino, Amido, Guanidyl, Hydroxyl, Thiol und Carboxyl substituiert ist und R_{D} Wasserstoff, (C₁-C₆)-Alkyl, (C₂-C₆)-Alkenyl, (C₂-C₆)-Alkinyl oder (C₃-C₆)-Cycloalkyl ist;
- Y ausgewählt ist aus CH, CH₂, C(CH₃)₂ und CCH₃;
- -̅ -̅ -̅ eine Einfach- oder Doppelbindung gemäß Y darstellt; und
- 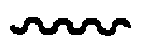 je nach Position von R₁ das alpha- oder beta-Anomer darstellt oder
eine Verbindung von Formel (Ia): 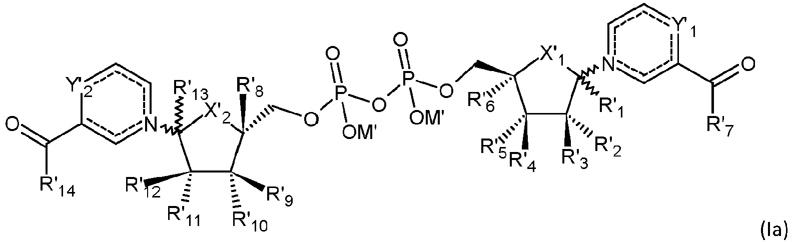 oder ein Stereoisomer davon, ein Salz davon, ein Hydrat davon, ein Solvat davon oder ein Kristall davon, wobei:
- X'₁ und X'₂ unabhängig ausgewählt sind aus O, CH₂, S, Se, CHF, CF₂ und C=CH₂;
- R'₁ und R'₁₃ unabhängig ausgewählt sind aus H, Azido, Cyano, C₁-C₈-Alkyl, C₁-C₈-Thioalkyl, C₁-C₈-Heteroalkyl und OR, wobei R ausgewählt ist aus Hund C₁-C₈-Alkyl,
- R'₂, R'₃, R'₄, R'₅, R'₉, R'₁₀, R'₁₁, R'₁₂ unabhängig ausgewählt sind aus H, Halogen, Azido, Cyano, Hydroxyl, C₁-C₁₂-Alkyl, C₁-C₁₂-Thioalkyl, C₁-C₁₂-Heteroalkyl, C₁-C₁₂-Halogenalkyl und OR, wobei R ausgewählt sein kann aus H, C₁-C₁₂-Alkyl, C(O)( C₁-C₁₂)-Alkyl, C(O)NH(C₁-C₁₂)-Alkyl, C(O)O(C₁-C₁₂)-Alkyl, C(O)-Aryl, C(O)( C₁-C₁₂)-Aryl, C(O)NH(C₁-C₁₂)-Alkylaryl, C(O)O(C₁-C₁₂)-Alkylaryl oder einer Gruppe C(O)CHR_{AA}NH2, wobei R_{AA} eine Seitenkette ist, ausgewählt aus einer proteinogenen Aminosäure;
- R'₆ und R'₈ unabhängig ausgewählt sind aus H, Azido, Cyano, C₁-C₈-Alkyl und OR, wobei R ausgewählt ist aus Hund C₁-C₈-Alkyl;
- R'₇ und R'₁₄ unabhängig ausgewählt sind aus H, OR, NHR, NRR', NH-NHR, SH, CN, N₃ und Halogen, wobei R und R' unabhängig ausgewählt sind aus H und (C₁-C₈)-Alkylaryl;
- Y'₁ und Y'₂ unabhängig ausgewählt sind aus CH, CH₂, C(CH₃)₂ oder CCH₃;
- M' ausgewählt ist aus H oder einem angepassten Gegenion;
- - - - eine Einfach- oder Doppelbindung abhängig von Y'₁ und Y'₂ darstellt; und
- 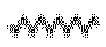 ein Alpha- oder Beta-Anomer abhängig von der Position von R'₁ und R' 13 darstellt; und Kombinationen davon,

2. Nicotinamidmononukleotid (NMN), pharmazeutisch annehmbares Derivat davon oder pharmazeutisch annehmbares Salz davon, zur Verwendung nach Anspruch 1, wobei das pharmazeutisch annehmbare Derivat von NMN ausgewählt ist aus der Verbindung I-B, der Verbindung I-C, der Verbindung I-D, der Verbindung I-E, der Verbindung I-F, der Verbindung I-G, der Verbindung I-H, der Verbindung I-I, der Verbindung I-J, vorzugsweise der Verbindung I-B, der Verbindung I-C, der Verbindung I-D, der Verbindung I-F und Kombinationen davon.

3. Nicotinamidmononukleotid (NMN), pharmazeutisch annehmbares Derivat davon oder pharmazeutisch annehmbares Salz davon, zur Verwendung nach Anspruch 1 oder 2, ausgewählt aus den Verbindungen Ia-A bis Ia-I, vorzugsweise aus der Verbindung von Formel Ia-B, der Verbindung von Formel Ia-C, der Verbindung von Formel Ia-E, der Verbindung von Formel Ia-F, der Verbindung von Formel Ia-H, der Verbindung von Formel Ia-I und der Verbindung von Formel Ia-G.

4. Nicotinamidmononukleotid (NMN), ein pharmazeutisch annehmbares Derivat davon oder pharmazeutisch annehmbares Salz davon, zur Verwendung nach einem der vorherigen Ansprüche, wobei der Gelenkschmerz den Nacken, die Schulter, das Schulterblatt, den Ellbogen, das Handgelenk, die Handgelenke, die Hüfte, das Iliosakralgelenk, das Knie, den Knöchel, die Fußgelenke oder Kombinationen davon betrifft, vorzugsweise das Knie.

5. Nicotinamidmononukleotid (NMN), pharmazeutisch annehmbares Derivat davon oder pharmazeutisch annehmbares Salz davon, zur Verwendung nach einem der vorherigen Ansprüche, wobei die körperliche Aktivität das Ausüben von Sport ist.

6. Nicotinamidmononukleotid (NMN), pharmazeutisch annehmbares Derivat davon oder pharmazeutisch annehmbares Salz davon, zur Verwendung nach einem der vorherigen Ansprüche, wobei es in Kombination mit mindestens einem anderen therapeutischen Mittel verwendet werden kann.

7. Nicotinamidmononukleotid (NMN), pharmazeutisch annehmbares Derivat davon oder pharmazeutisch annehmbares Salz davon zur Verwendung nach einem der vorherigen Ansprüche, wobei der Gelenkschmerz nicht auf eine der Erkrankungen zurückzuführen ist, die ausgewählt ist aus Tumor, Arthritis, Gicht, Arthrose, Gelenkdeformität, Bindegewebserkrankung, Dorsopathie, neurodegenerativer Erkrankung, Neuropathie, genetischer Erkrankung, Autoimmunerkrankung, Myopathie, Osteopathie, Osteoporose, Chondropathie, Vaskulopathie, Virusinfektion, Pilzinfektion, bakterieller Infektion, Parasiten, Nebenwirkung eines Medikaments, chirurgischem Eingriff, medizinischer Untersuchung, Verkalkung, Trauma außer wenn induziert durch körperliche Aktivität, einer Missbildung und Kombinationen davon.

8. Zusammensetzung, umfassend Nicotinamidmononukleotid (NMN), eines seiner pharmazeutisch annehmbaren Derivate oder eines seiner pharmazeutisch annehmbaren Salze und mindestens einen pharmazeutisch annehmbaren Hilfsstoff zur topischen Verwendung bei der Vorbeugung und/oder Behandlung von durch körperliche Aktivität induzierten Gelenkschmerzen, wobei das pharmazeutisch annehmbare Derivat ausgewählt ist aus Dihydronicotinamidmononukleotid (NMN-H), alpha-NMN, einer Verbindung von Formel (I): 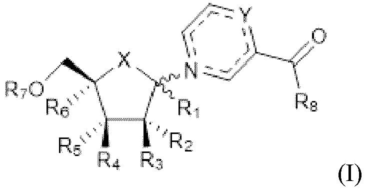 oder einem Stereoisomer davon, einem Salz davon, einem Hydrat davon, einem Solvat davon oder einem pharmazeutisch annehmbaren Kristall davon, wobei:
- X ausgewählt ist aus O, CH₂, S, Se, CHF, CF₂ und C=CH₂;
- R₁ ausgewählt ist aus H, Azido, Cyano, C₁-C₈-Alkyl, C₁-C₈-Thioalkyl, C₁-C₈-Heteroalkyl und OR; wobei R ausgewählt ist aus Hund C₁-C₈-Alkyl;
- R₂, R₃, R₄ und Rs unabhängig voneinander ausgewählt sind aus H, Halogen, Azido, Cyano, Hydroxyl, C₁-C₁₂-Alkyl, C₁-C₁₂-Thioalkyl, C₁-C₁₂-Heteroalkyl, C₁-C₁₂ Halogenalkyet OR; wobei R ausgewählt ist aus H, C₁-C₁₂-Alkyl, C(O)(C₁-C₁₂)-Alkyl, C(O)NH(C₁-C₁₂)-Alkyl, C(0)0(C₁-C₁₂)-Alkyl, C(O)-Aryl, C(O)( C₁-C₁₂)-Alkylaryl, C(O)NH(C₁-C₁₂)-Alkylaryl, C(O)O(C₁-C₁₂)-Alkylaryl und C(O)CHR_{AA}NH₂; wobei R_{AA} eine Seitenkette ist, die ausgewählt ist aus einer proteinogenen Aminosäure;
- R₆ ausgewählt ist aus H, Azido, Cyano, C₁-C₈-Alkyl, C₁-C₈-Thioalkyl, C₁-C₈-Heteroalkyl und OR; wobei R ausgewählt ist aus Hund C₁-C₈-Alkyl;
- R₇ ausgewählt ist aus H, P(O)R₉R₁₀ und P(S)R₉R₁₀ und 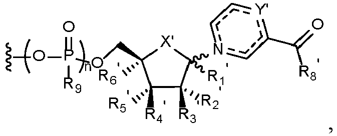 wobei n eine ganze Zahl ist, ausgewählt aus 1, 2 oder 3; wobei
- R₉ und R₁₀ unabhängig voneinander ausgewählt sind aus OH, OR₁₁, NHR₁₃, NR₁₃R₁₄, C₁-C₈-Alkyl, C₂-C₈-Alkenyl, C₂-C₈-Alkinyl, C₃-C₁₀-Cycloalkyl, C₅-C₁₂-Aryl (C₁-C₈)-Alkylaryl, (C₁-C₈)-Arylalkyl, (C₁-C₈)-Heteroalkyl, (C₁-C₈)-Heterocycloalkyl, Heteroaryl und NHCHR_{A}R_{A'}C(O)R₁₂; wobei:
- R₁₁ ausgewählt ist aus einer Gruppe C₁-C₁₀-Alkyl, C₃-C₁₀-Cycloalkyl, C₅-C₁₈-Aryl, C₁-C₁₀-Alkylaryl, substituiertem C₅-C₁₂-Aryl, C₁-C₁₀-Heteroalkyl, C₃-C₁₀-Heterocycloalkyl, C₁-C₁₀-Haloalkyl, einem Heteroaryl,-(CH₂)ₙC(O)(C₁-C₁₅)-Alkyl, -(CH₂)ₙOC(O)(C₁-C₁₅)-Alkyl,-(CH₂)ₙOC(O)O(C₁-C₁₅)-Alkyl, -(CH₂)ₙSC(O)(C₁-C₁₅)-Alkyle,-(CH₂)ₙC(O)O(C₁-C₁₅)-Alkyle und -(CH₂)ₙC(O)O(C₁-C₁₅)-Alkylaryl, wobei n eine ganze Zahl ist, ausgewählt aus 1 bis 8; P(O)(OH)OP(O)(OH)z, Halogen, Nitro, Cyano, C₁-C₆-Alkoxy, C₁-C₆-Halogenalkoxy, -N(R₁₁ₐ)₂, C₁-C₆-Acylamino, -COR_{11b}, -O COR_{11b}; NHSO₂(C₁-C₆-Alkyl),-SO₂N(R₁₁ₐ)₂SO₂, wobei jedes von R₁₁ₐ jeweils unabhängig ausgewählt ist aus H und C₁-C₆-Alkyl und R_{11b} unabhängig ausgewählt ist aus OH, C₁-C₆-Alkoxy, NH₂, NH(C₁-C₆-Alkyl ) oder N(C₁-C₆-Alkyl)₂,
- R₁₂ ausgewählt ist aus H, C₁-C₁₀-Alkyl, C₂-C₈-Alkenyl, C₂-C₈-Alkinyl, C₁-C₁₀-Haloalkyl, C₃-C₁₀-Cycloalkyl, C₃-C₁₀-Heterocycloalkyl, C₅-C₁₈-Aryl, C₁-C₄-Alkylaryl und C₅-C₁₂-Heteroaryl, wobei die Aryl- oder Heteroarylgruppe optional mit einer oder zwei Gruppen substituiert sind, die ausgewählt sind aus Halogen, Trifluormethyl, C₁-C₆-Alkyl, C₁-C₆-Alkoxy und Cyano; und
- R_{A} und R_{A'} unabhängig ausgewählt sind aus H, C₁-C₁₀-Alkyl, C₂-C₁₀-Akenyl, C₂-C₁₀-Alkinyl, C₃-C₁₀-Cycloalkyl, C₁-C₁₀-Thioalkyl, C₁-C₁₀-Hydroxylalkyl, C₁-C₁₀-Alkylaryl und C₅-C₁₂-Aryl, C₃-C₁₀-Heterocycloalkyl, einem Heteroaryl, -(CH₂)₃NHC(=NH)NH₂, (1H-Indol-3-yl)methyl, (1H-Imidazol-4-yl)methyl und einer Seitenkette, die ausgewählt ist aus einer proteinogenen Aminosäure oder einer nicht-proteinogenen Aminosäure; wobei die Arylgruppen optional mit einer Gruppe substituiert sind, die ausgewählt ist aus Hydroxyl, C₁-C₁₀-Alkyl, C₁-C₆-Alkoxy, Halogen, Nitro und Cyano; oder
- R₉ und R₁₀ zusammen mit den Phosphoratomen, an die sie gebunden sind, einen 6-gliedrigen Ring bilden, wobei -R₉-R₁₀- -CH₂CH₂CHR-darstellt; wobei R ausgewählt ist aus H, (C₅-C₆)-Aryl und (C₅-C₆)-Heteroaryl, worin die Aryl- oder Heteroarylgruppen optional substituiert sind mit Halogen, Trifluormethyl, C₁-C₆-Alkyl, C₁-C₆-Alkoxy und Cyano; oder
R₉ und R₁₀ zusammen mit den Phosphoratomen, an die sie gebunden sind, einen 6-gliedrigen Ring bilden, wobei -R₉-R₁₀--O-CH₂-CH₂-CHR-O- darstellt; wobei R ausgewählt ist aus H, (C5-C6)-Aryl und (C5-C6)-Heteroaryl, wobei die Aryl- oder Heteroarylgruppen optional substituiert sind mit Halogen, Trifluormethyl, (C₁-C₆)-Alkyl, (C₁-C₆)-Alkoxy und Cyano;
- R₈ ausgewählt ist aus H, OR, NHR₁₃, NR₁₃R₁₄, NH-NHR₁₃, SH, CN, N₃ und Halogen; wobei:
- R₁₃ und R₁₄ unabhängig voneinander ausgewählt sind aus H, (C₁-C₈)-Alkyl und (C₁-C₈)-Alkylaryl ausgewählt sind, und -CR_{B}R_{C}-C(O)-OR_{D}, wobei R_{B} und Rc unabhängig Wasserstoff, (C₁-C₆)-Alkyl, (C₁-C₆)-Alkoxy, Benzyl, Indolyl oder Imidazolyl sind, wobei das (C₁-C₆)-Alkyl und das (C₁-C₆)-Alkoxy optional und unabhängig mit einer oder mehreren der Gruppen Halogen, Amino, Amido, Guanidyl, Hydroxyl substituiert sein können, Thiol oder Carboxyl substituiert ist und die Benzylgruppe optional mit einer oder mehreren der Halogen- oder Hydroxylgruppen substituiert ist, oder R_{B} und Rc zusammen mit dem Kohlenstoffatom, an das sie gebunden sind, eine C₃-C₆-Cycloalkylgruppe bilden, die optional mit einem oder mehreren Halogenen, Amino, Amido, Guanidyl, Hydroxyl, Thiol und Carboxyl substituiert ist und R_{D} Wasserstoff, (C₁-C₆)-Alkyl, (C₂-C₆)-Alkenyl, (C₂-C₆)-Alkinyl oder (C₃-C₆)-Cycloalkyl ist;
- Y ausgewählt ist aus CH, CH₂, C(CH₃)₂ und CCH₃;
- -̅ -̅ -̅ eine Einfach- oder Doppelbindung gemäß Y darstellt; und
- 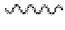 je nach Position von R₁ das alpha- oder beta-Anomer darstellt oder
eine Verbindung von Formel (Ia): 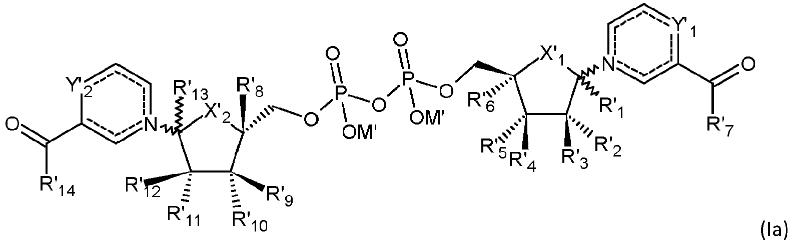 oder ein Stereoisomer davon, ein Salz davon, ein Hydrat davon, ein Solvat davon oder ein Kristall davon, wobei
- X'₁ und X'₂ unabhängig ausgewählt sind aus O, CH₂, S, Se, CHF, CF₂ und C=CH₂;
- R'₁ und R'₁₃ unabhängig ausgewählt sind aus H, Azido, Cyano, C₁-C₈-Alkyl, C₁-C₈-Thioalkyl, C₁-C₈-Heteroalkyl und OR, wobei R ausgewählt ist aus Hund C₁-C₈-Alkyl,
- R'₂, R'₃, R'₄, R'₅, R'₉, R'₁₀, R'₁₁, R'₁₂ unabhängig ausgewählt sind aus H, Halogen, Azido, Cyano, Hydroxyl, C₁-C₁₂-Alkyl, C₁-C₁₂-Thioalkyl, C₁-C₁₂-Heteroalkyl, C₁-C₁₂-Halogenalkyl und OR, wobei R ausgewählt sein kann aus H, C₁-C₁₂-Alkyl, C(O)( C₁-C₁₂)-Alkyl, C(O)NH(C₁-C₁₂)-Alkyl, C(O)O(C₁-C₁₂)-Alkyl, C(O)-Aryl, C(O)( C₁-C₁₂)-Aryl, C(O)NH(C₁-C₁₂)-Alkylaryl, C(O)O(C₁-C₁₂)-Alkylaryl oder einer Gruppe C(O)CHR_{AA}NH2, wobei R_{AA} eine Seitenkette ist, ausgewählt aus einer proteinogenen Aminosäure;
- R'₆ und R'₈ unabhängig ausgewählt sind aus H, Azido, Cyano, C₁-C₈-Alkyl und OR, wobei R ausgewählt ist aus Hund C₁-C₈-Alkyl;
- R'₇ und R'₁₄ unabhängig ausgewählt sind aus H, OR, NHR, NRR', NH-NHR, SH, CN, N₃ und Halogen, wobei R und R' unabhängig ausgewählt sind aus H und (C₁-C₈)-Alkylaryl;
- Y'₁ und Y'₂ unabhängig ausgewählt sind aus CH, CH₂, C(CH₃)₂ oder CCH₃;
- M' ausgewählt ist aus H oder einem angepassten Gegenion;
- - - - eine Einfach- oder Doppelbindung abhängig von Y'₁ und Y'₂ darstellt; und
- 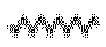 ein Alpha- oder Beta-Anomer abhängig von der Position von R'₁ und R'₁₃ darstellt; und Kombinationen davon.

9. Zusammensetzung nach Anspruch 8, umfassend Nicotinamidmononukleotid (NMN), ein pharmazeutisch annehmbares Derivat davon oder ein pharmazeutisch annehmbares Salz davon in einer Menge von 0,05 bis 15 Gewichts-%, vorzugsweise von 1 bis 10 Gewichts-%, bevorzugter von 3 bis 5 Gewichts-%, bezogen auf das Gesamtgewicht der Zusammensetzung.

10. Zusammensetzung nach Anspruch 8 oder 9 in Form einer Wasser-in-Öl-Emulsion oder einer Öl-in-Wasser-Emulsion, vorzugsweise in Form einer Öl-in-Wasser-Emulsion.

11. Zusammensetzung nach einem der Ansprüche 8 bis 10, ferner umfassend mindestens ein weiteres therapeutisches Mittel.

12. Zusammensetzung nach Anspruch 11, wobei das mindestens eine zusätzliche therapeutische Mittel ausgewählt ist aus einem Schmerzmittel, einem nicht-steroidalen entzündungshemmenden Mittel, Kortison, einem Kortisonderivat oder Kombinationen davon.

13. Zusammensetzung nach Anspruch 12, wobei das pharmazeutisch annehmbare Derivat ausgewählt ist aus Verbindung I-B, Verbindung I-C,, Verbindung I-D, Verbindung I-E, Verbindung I-F, Verbindung I-G, Verbindung I-H, Verbindung I-I, Verbindung I-J, vorzugsweise Verbindung IB, Verbindung IC, Verbindung ID, Verbindung IF, Verbindung Ia-A, Verbindung von Formel Ia-B, Verbindung von Formel Ia-C, Verbindung von Formel Ia-E, Verbindung von Formel Ia-F, Verbindung von Formel Ia-H, Verbindung von Formel Ia-I, Verbindung von Formel Ia-G und Kombinationen davon.

## Claims

1. Nicotinamide mononucleotide (NMN), a pharmaceutically acceptable derivative thereof or a pharmaceutically acceptable salt thereof, for use via topical route in the prevention and/or treatment of a joint pain induced by physical activity, wherein the pharmaceutically acceptable derivative of NMN is chosen from among dihydronicotinamide mononucleotide (NMN-H), alpha-NMN, a compound of formula (I): 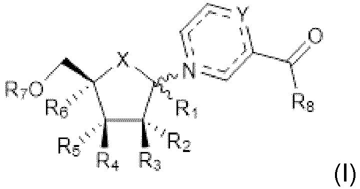 or a pharmaceutically acceptable stereoisomer, salt, hydrate, solvate, or crystal thereof, wherein:
- X is chosen from among O, CH₂, S, Se, CHF, CF₂ and C=CH₂;
- R₁ is chosen from among H, azido, cyano, C₁-C₈ alkyl, C₁-C₈ thioalkyl, C₁-C₈ heteroalkyl and OR; wherein R is chosen from among H and C₁-C₈ alkyl;
- R₂, R₃, R₄, and R₅ are each independently chosen from among H, halogen, azido, cyano, hydroxyl, C₁-C₁₂ alkyl, C₁-C₁₂ thioalkyl, C₁-C₁₂ heteroalkyl, C₁-C₁₂ haloakyl and OR; wherein R is chosen from among H, C₁-C₁₂ alkyl, C(O)(C₁-C₁₂)alkyl, C(O)NH(C₁-C₁₂)alkyl, C(O)O(C₁-C₁₂)alkyl, C(O)aryl, C(O)(C₁-C₁₂)alkyl aryl, C(O)NH(C₁-C₁₂)alkyl aryl, C(O)O(C₁-C₁₂)alkyl aryl and C(O)CHR_{AA}NH₂; wherein R_{AA} is a side chain chosen from among a proteinogenic amino acid;
- R₆ is chosen from among H, azido, cyano, C₁-C₈ alkyl, C₁-C₈ thioalkyl, C₁-C₈ heteroalkyl and OR; wherein R is chosen from among H and C₁-C₈ alkyl;
- R₇ is chosen from among H, P(O)R₉R₁₀ and P(S)R₉R₁₀ and 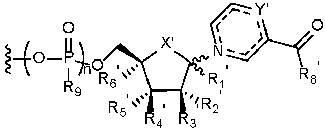 wherein n is an integer chosen to be 1, 2, or 3; wherein:
- R₉ and R₁₀ are each independently chosen from among OH, OR₁₁, NHR₁₃, NR₁₃R₁₄, C₁-C₈ alkyl, C₂-C₈ alkenyl, C₂-C₈ alkynyl, C₃-C₁₀ cycloalkyl, C₅-C₁₂ aryl, (C₁-C₈) alkyl aryl, (C₁-C₈) aryl alkyl, (C₁-C₈) heteroalkyl, (C₁-C₈) heterocycloalkyl, a heteroaryl and NHCHR_{A}R_{A'}C(O)R₁₂; wherein:
- R₁₁ is chosen from among the groups C₁-C₁₀ alkyl, C₃-C₁₀ cycloalkyl, C₅-C₁₈ aryl, C₁-C₁₀ alkylaryl, substituted C₅-C₁₂ aryl, C₁-C₁₀ heteroalkyl, C₃-C₁₀ heterocycloalkyl, C₁-C₁₀ haloalkyl, a heteroaryl,-(CH₂)ₙC(O)(C₁-C₁₅)alkyl, -(CH₂)ₙOC(O)C₁-C₁₅)alkyl,-(CH₂)ₙOC(O)O(C₁-C₁₅)alkyl, -(CH₂)ₙSC(O)(C₁-C₁₅) alkyl,-(CH₂)ₙC(O)O(C₁-C₁₅) alkyl and -(CH₂)ₙC(O)O(C₁-C₁₅) alkyl aryl; wherein n is an integer chosen from 1 to 8; P(O)(OH)OP(O)(OH)₂, halogen, nitro, cyano, C₁-C₆ alkoxy, C₁-C₆ haloalkoxy, -N(R₁₁ₐ)₂, C₁-C₆ acylamino, -COR_{11b}, -O COR_{11b}; NHSO₂(C₁-C₆ alkyl), - SO₂N(R₁₁ₐ)₂SO₂ wherein each R₁₁ₐ is independently chosen from among H and C₁-C₆ alkyl, and R_{11b} is independently chosen from among OH, C₁-C₆ alkoxy, NH₂, NH(C₁-C₆ alkyl) or N(C₁-C₆ alkyl)₂;
- R₁₂ is chosen from among H, C₁-C₁₀ alkyl, C₂-C₈ alkenyl, C₂-C₈ alkynyl, C₁-C₁₀ haloalkyl, C₃-C₁₀ cycloalkyl, C₃-C₁₀ heterocycloalkyl, C₅-C₁₈ aryl, C₁₋C₄ alkylaryl and C₅-C₁₂ heteroaryl; wherein said aryl or heteroaryl groups are optionally substituted by one or two groups chosen from among halogen, trifluoromethyl, C₁-C₆ alkyl, C₁-C₆ alkoxy and cyano; and
- R_{A} and R_{A'} are each independently chosen from among H, C₁-C₁₀ alkyl, C₂-C₁₀ alkenyl, C₂-C₁₀ alkynyl, C₃-C₁₀ cycloalkyl, C₁-C₁₀ thioalkyl, C₁-C₁₀ hydroxyalkyl, C₁-C₁₀ alkylaryl and C₅-C₁₂ aryl, C₃-C₁₀ heterocycloalkyl, a heteroaryl, -(CH₂)₃NHC(=NH)NH₂, (1H-indol-3-yl)methyl, (1H-imidazol-4-yl)methyl and a side chain chosen from among a proteinogenic amino acid or non-proteinogenic amino acid; wherein said aryl groups are optionally substituted by a group chosen from among a hydroxyl, C₁-C₁₀ alkyl, C₁-C₆ alkoxy, a halogen, nitro and cyano; or
- R₉ and R₁₀, together with the phosphorus atoms to which they are attached, form a 6-membered ring in which -R₉-R₁₀- represents -CH₂-CH₂-CHR-; wherein R is chosen from among H, (C₅-C₆) aryl and (C₅-C₆) heteroaryl group, wherein said aryl or heteroaryl groups are optionally substituted by a halogen, trifluoromethyl, (C₁-C₆) alkyl, (C₁-C₆) alkoxy, and cyano; or
R₉ and R₁₀, together with the phosphorus atoms to which they are attached, form a 6-membered ring in which -R₉-R₁₀- represents -O-CH₂-CH₂-CHR-O-; wherein R is chosen from among H, (C₅-C₆) aryl and (C₅-C₆) heteroaryl group, wherein said aryl or heteroaryl groups are optionally substituted by a halogen, trifluoromethyl, (C₁-C₆) alkyl, (C₁-C₆) alkoxy, and cyano;
- R₈ is chosen from among H, OR, NHR₁₃, NR₁₃R₁₄, NH-NHR₁₃, SH, CN, N₃ and halogen;wherein
- R₁₃ and R₁₄ are each independently chosen from among H, (C₁-C8) alkyl and (C₁-C8) alkyl aryl, and -CR_{B}R_{C}-C(O)-OR_{D} wherein R_{B} and Rc are independently a hydrogen atom, (C₁-C₆) alkyl, (C₁-C₆) alkoxy, benzyl, indolyl or imidazolyl wherein the (C₁-C₆) alkyl and (C₁-C₆) alkoxy can optionally and each independently be substituted by one or more of the groups halogen, amino, amido, guanidyl, hydroxyl, thiol or carboxyl, and the benzyl group is optionally substituted by one or more halogen or hydroxyl groups, or R_{B} and Rc together with the carbon atom to which they are attached form a C₃-C₆ cycloalkyl group optionally substituted by one or more from among halogen, amino, amido, guanidyl, hydroxyl, thiol and carboxyl, and R_{D} is a hydrogen, (C₁-C₆) alkyl, (C₂-C₆) alkenyl, (C₂-C₆) alkynyl or (C₃-C₆) cycloalkyl;
- Y is chosen from among CH, CH₂, (C(CH₃)₂ and CCH₃;
- -̅ -̅ -̅ is a single or double bond depending on Y; and
- 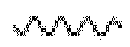 is the alpha or beta anomer depending on the position of R₁ or
a compound of formula (Ia): 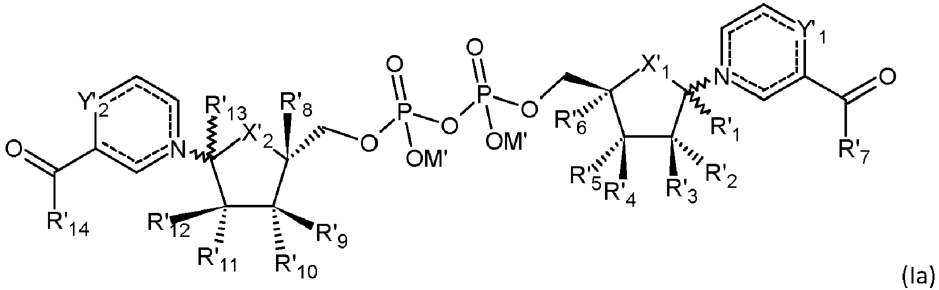 or a stereoisomer, salt, hydrate, solvate or crystal thereof, wherein:
- X'₁ and X_{'2} are independently chosen from among O, CH₂, S, Se, CHF, CF₂ and C=CH₂;
- R'₁ and R'₁₃ are independently chosen from among H, azido, cyano, C₁-C₈ alkyl, C₁-C₈ thioalkyl, C₁-C₈ heteroalkyl and OR, wherein R is chosen from among H and C₁-C₈ alkyl;
- R'₂, R'₃, R'₄, R'₅ R'₉, R'₁₀, R'₁₁, R'₁₂ are independently chosen from among H, a halogen, azido, cyano, hydroxyl, C₁-C₁₂ alkyl, C₁-C₁₂ thioalkyl, C₁-C₁₂ hetero-alkyl, C₁-C₁₂ haloalkyl and OR, wherein R can be chosen from among H, C₁-C₁₂ alkyl, C(O)(C₁-C₁₂) alkyl, C(O)NH(C₁-C₁₂) alkyl, C(O)O(C₁-C₁₂) alkyl, C(O) aryl, C(O)(C₁-C₁₂) aryl, C(O)NH(C₁-C₁₂) alkyl aryl, C(O)O(C₁-C₁₂) alkyl aryl or C(O)CHR_{AA}NH2 group wherein R_{AA} is a side chain chosen from among a proteogenic amino acid;
- R'₆ and R'₈ are independently chosen from among H, an azido, cyano, C₁-C₈ alkyl and OR, wherein R is chosen from among H and C₁-C₈ alkyl;
- R'₇ and R'₁₄ are independently chosen from among H, OR, NHR, NRR', NH-NHR, SH, CN, N₃ and a halogen, wherein R and R' are independently chosen from among H and (C₁-C8) alkyl aryl;
- Y'₁ and Y'₂ are independently chosen from among CH, CH₂, C(CH₃)₂ or CCH₃;
- M' is chosen from among H or a suitable counter-ion;
- - - - is a single or double bond as a function of Y'₁ and Y'₂; and
- 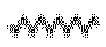 is an alpha or beta anomer as a function of the position of R'₁ and R'₁₃; and combinations thereof.

2. Nicotinamide mononucleotide (NMN), a pharmaceutically acceptable derivative thereof or a pharmaceutically acceptable salt thereof, for use according to claim 1, wherein the pharmaceutically acceptable derivative of NMN is chosen from among compound I-B, compound I-C, compound I-D, compound I-E, compound I-F, compound I-G, compound I-H, compound I-I, compound I-J, preferably compound I-B, compound I-C, compound I-D, compound I-F and combinations thereof.

3. Nicotinamide mononucleotide (NMN), a pharmaceutically acceptable derivative thereof or a pharmaceutically acceptable salt thereof, for use according to claim 1or 2, is chosen from among compounds Ia-A to Ia-I, preferably from among the compound of formula la-B, the compound of formula la-C, the compound of formula la-E, the compound of formula la-F, the compound of formula la-H, the compound of formula Ia-I and the compound of formula la-G.

4. Nicotinamide mononucleotide (NMN), a pharmaceutically acceptable derivative thereof or a pharmaceutically acceptable salt thereof, for use according to one of the preceding claims, wherein the joint pain concerns the nape of the neck, shoulder, scapula, elbow, wrist, hand joints, hip, sacroiliac joint, knee, ankle, foot joints, or combinations thereof, preferably the knee.

5. Nicotinamide mononucleotide (NMN), a pharmaceutically acceptable derivative thereof or a pharmaceutically acceptable salt thereof, for use according to one of the preceding claims, wherein the physical activity is the practising of a sport.

6. Nicotinamide mononucleotide (NMN), a pharmaceutically acceptable derivative thereof or a pharmaceutically acceptable salt thereof, for use according to one of the preceding claims, wherein it can be used in combination with at least one other therapeutic agent.

7. Nicotinamide mononucleotide (NMN), a pharmaceutically acceptable derivative thereof or a pharmaceutically acceptable salt thereof, for use according to any of the preceding claims, wherein the joint pain is not due to one of the pathologies chosen from among a tumour, arthritis, gout, arthrosis, joint deformation, disease of conjunctive issue, dorsopathy, neurodegenerative disease, neuropathy, genetic disease, autoimmune disease, myopathy, osteopathy, osteoporosis, chondropathy, vasculopathy, viral infection, fungal infection, bacterial infection, a parasite, side effect of a medication, surgical procedure, medical examination, calcification, trauma unless induced by physical activity, a malformation, and combinations thereof.

8. A composition comprising nicotinamide mononucleotide (NMN), a pharmaceutically acceptable derivative thereof or a pharmaceutically acceptable salt thereof and at least one pharmaceutically acceptable excipient, for use thereof via topical route in the prevention and/or treatment of joint pain induced by physical activity, wherein the pharmaceutically acceptable derivative is chosen from among dihydronicotinamide mononucleotide (NMN-H), alpha-NMN, a compound of formula (I): 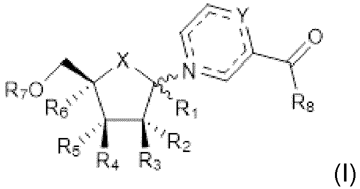 or a pharmaceutically acceptable stereoisomer, salt, hydrate, solvate, or crystal thereof, wherein:
- X is chosen from among O, CH₂, S, Se, CHF, CF₂ and C=CH₂;
- R₁ is chosen from among H, azido, cyano, C₁-C₈ alkyl, C₁-C₈ thioalkyl, C₁-C₈ heteroalkyl and OR; wherein R is chosen from among H and C₁-C₈ alkyl;
- R₂, R₃, R₄, and R₅ are each independently chosen from among H, halogen, azido, cyano, hydroxyl, C₁-C₁₂ alkyl, C₁-C₁₂ thioalkyl, C₁-C₁₂ heteroalkyl, C₁-C₁₂ haloakyl and OR; wherein R is chosen from among H, C₁-C₁₂ alkyl, C(O)(C₁-C₁₂)alkyl, C(O)NH(C1-C₁₂)alkyl, C(O)O(C₁-C₁₂)alkyl, C(O)aryl, C(O)(C₁-C₁₂)alkyl aryl, C(O)NH(C₁-C₁₂)alkyl aryl, C(O)O(C₁-C₁₂)alkyl aryl, and C(O)CHR_{AA}NH₂; wherein R_{AA} is a side chain chosen from among a proteinogenic amino acid;
- R₆ is chosen from among H, azido, cyano, C₁-C₈ alkyl, C₁-C₈ thioalkyl, C₁-C₈ heteroalkyl and OR; where R is chosen from among H and C1-C8 alkyl;
- R₇ is chosen from among H, P(O)R₉R₁₀ and P(S)R₉R₁₀ and 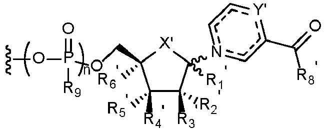 wherein n is an integer chosen to be 1, 2, or 3; wherein:
- R₉ and R₁₀ are each independently chosen from among OH, OR₁₁, NHR₁₃, NR₁₃R₁₄, C₁-C₈ alkyl, C₂-C₈ alkenyl, C₂-C₈ alkynyl, C₃-C₁₀ cycloalkyl, C₅-C₁₂ aryl, (C₁-C₈)alkyl aryl, (C₁-C₈)aryl alkyl, (C₁-C8) heteroalkyl, (C₁-C8) heterocycloalkyl, a heteroaryl and NHCHR_{A}R_{A'}C(O)R₁₂; wherein:
- R₁₁ is chosen from among the groups C₁-C₁₀ alkyl, C₃-C₁₀ cycloalkyl, C₅-C₁₈ aryl, C₁-C₁₀ alkylaryl, substituted C₅-C₁₂ aryl, C₁-C₁₀ heteroalkyl, C₃-C₁₀ heterocycloalkyl, C₁-C₁₀ haloalkyl, a heteroaryl,-(CH₂)ₙC(O)(C₁-C₁₅)alkyl, -(CH₂)ₙOC(O)C₁-C₁₅)alkyl,-(CH₂)ₙOC(O)O(C₁-C₁₅)alkyl, -(CH₂)ₙSC(O)(C₁-C₁₅) alkyl,-(CH₂)ₙC(O)O(C₁-C₁₅)alkyl aryl; wherein n is an integer chosen from 1 to 8; P(O)(OH)OP(O)(OH)₂, halogen, nitro, cyano, C₁-C₆ alkoxy, C₁-C₆ haloalkoxy, -N(R₁₁ₐ)₂, C₁-C₆ acylamino, -COR_{11b}, -O COR_{11b}; NHSO₂(C₁-C₆ alkyl), -SO₂N(R₁₁ₐ)₂SO₂ wherein each R₁₁ₐ is independently chosen from among H and a C₁-C₆ alkyl, and R_{11b} is independently chosen from among OH, C₁-C₆ alkoxy, NH₂, NH(C₁-C₆ alkyl) or N(C₁-C₆ alkyl)₂;
- R₁₂ is chosen from among H, C₁-C₁₀ alkyl, C₂-C₈ alkenyl, C₂-C₈ alkynyl, C₁-C₁₀ haloalkyl, C₃-C₁₀ cycloalkyl, C₃-C₁₀ heterocycloalkyl, C₅-C₁₈ aryl, C₁₋C₄ alkylaryl and C₅-C₁₂ heteroaryl; wherein said aryl or heteroaryl groups are optionally substituted by one or two groups chosen from among halogen, trifluoromethyl, C₁-C₆ alkyl, C₁-C₆ alkoxy and cyano; and
- R_{A} and R_{A'} are each independently chosen from among H, C₁-C₁₀ alkyl, C₂-C₁₀ alkenyl, C₂-C₁₀ alkynyl, C₃-C₁₀ cycloalkyl, C₁-C₁₀ thioalkyl, C₁-C₁₀ hydroxylalkyl, C₁-C₁₀ alkylaryl and C₅-C₁₂ aryl, C₃-C₁₀ heterocycloalkyl, a heteroaryl, -(CH₂)₃NHC(=NH)NH₂, (1H-indol-3-yl)methyl, (1H-imidazol-4-yl)methyl and a side chain chosen from among a proteinogenic amino acid or non-proteinogenic amino acid; wherein said aryl groups are optionally substituted by a group chosen from among a hydroxyl, C₁-C₁₀ alkyl, C₁-C₆ alkoxy, a halogen, nitro and cyano; or
- R₉ and R₁₀, together with the phosphorus atoms to which they are attached, form a 6-membered ring in which -R₉-R₁₀- represents -CH₂-CH₂-CHR; where R is chosen from among H, a (C₅-C₆) aryl and (C₅-C₆) heteroaryl group, wherein said aryl or heteroaryl groups are optionally substituted by a halogen, trifluoromethyl, (C₁-C₆) alkyl, (C₁-C₆) alkoxy, and cyano; or
R₉ and R₁₀, together with the phosphorus atoms to which they are attached, form a 6-membered ring in which -R₉-R₁₀- represents -O-CH₂-CH₂-CHR-O-; wherein R is chosen from among H, a (C₅-C₆) aryl and (C₅-C₆) heteroaryl group, wherein said aryl or heteroaryl groups are optionally substituted by a halogen, trifluoromethyl, (C₁-C₆) alkyl, (C₁-C₆) alkoxy, and cyano;
- R₈ is chosen from among H, OR, NHR₁₃, NR₁₃R₁₄, NH-NHR₁₃, SH, CN, N₃ and halogen, wherein:
- R₁₃ and R₁₄ are each independently chosen from among H, (C₁-C₈) alkyl and (C₁-C₈) alkyl aryl, and -CR_{B}R_{C}-C(O)-OR_{D} where R_{B} and Rc are independently a hydrogen atom,(C₁-C₆) alkyl, (C₁-C₆) alkoxy, benzyl, indolyl or imidazolyl, where the (C₁-C₆) alkyl and (C₁-C₆) alkoxy can optionally and each independently be substituted by one or more of the groups halogen, amino, amido, guanidyl, hydroxyl, thiol or carboxyl, and the benzyl group is optionally substituted by one or more halogen or hydroxyl groups, or R_{B} and Rc together with the carbon atom to which they are attached form a C₃-C₆ cycloalkyl group optionally substituted by one or more from among halogen, amino, amido, guanidyl, hydroxyl, thiol and carboxyl, and R_{D} is a hydrogen, (C₁-C₆) alkyl, (C₂-C₆) alkenyl, (C₂-C₆) alkynyl or (C₃-C₆) cycloalkyl;
- Y is chosen from among CH, CH₂, (C(CH₃)₂ and CCH₃;
- -̅ -̅ -̅ is a single or double bond depending on Y; and
- 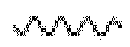 is the alpha or beta anomer depending on the position of R₁ or
a compound of formula (Ia): 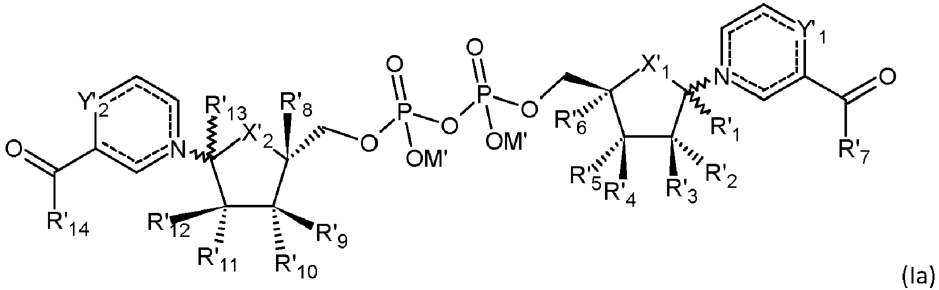 or a stereoisomer, salt, hydrate, solvate or crystal thereof, wherein:
- X'₁ and X'₂ are independently chosen from among O, CH₂, S, Se, CHF, CF₂ and C=CH₂;
- R'₁ and R'₁₃ are independently chosen from among H, azido, cyano, C₁-C₈ alkyl, C₁-C₈ thioalkyl, C₁-C₈ heteroalkyl and OR, wherein R is chosen from among H and C₁-C₈ alkyl,
- R'₂, R'₃, R'₄, R'₅, R'₉, R'₁₀, R'₁₁, R'₁₂ are independently chosen from among H, a halogen, azido, cyano, hydroxyl, C₁-C₁₂ alkyl, C₁-C₁₂ thioalkyl, C₁-C₁₂ hetero-alkyl, C₁-C₁₂ haloalkyl and OR, wherein R can be chosen from among H, C₁-C₁₂ alkyl, C(O)(C₁-C₁₂) alkyl, C(O)NH(C₁-C₁₂) alkyl, C(O)O(C₁-C₁₂) alkyl, C(O) aryl, C(O)(C₁-C₁₂) aryl, C(O)NH(C₁-C₁₂) alkyl aryl, C(O)O(C₁-C₁₂) alkyl aryl or C(O)CHR_{AA}NH₂ group wherein R_{AA} is a side chain chosen from among a proteogenic amino acid;
- R'₆ and R'₈ are independently chosen from among H, an azido, cyano, C₁-C₈ alkyl and OR, wherein R is chosen from among H and C₁-C₈ alkyl;
- R'₇ and R'₁₄ are independently chosen from among H, OR, NHR, NRR', NH-NHR, SH, CN, N₃ and a halogen, wherein R and R' are independently chosen from among H and (C₁-C₈) alkyl aryl;
- Y'₁ and Y'₂ are independently chosen from among CH, CH₂, C(CH₃)₂ or CCH₃;
- M' is chosen from among H or suitable counter-ion;
- - - - is a single or double bond as a function of Y'1 and Y'2; and
- 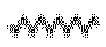 is an alpha or beta anomer as a function of the position of R'₁ and R'₁₃; and combinations thereof.

9. A composition according to claim 8 comprising nicotinamide mononucleotide (NMN), a pharmaceutically acceptable derivative thereof or a pharmaceutically acceptable salt thereof, in an amount of between 0.05% and 15% by weight, preferably between 1 and 10% by weight, more preferably between 3 and 5% by weight relative to the total weight of the composition.

10. The composition according to claim 8 or 9, in the form of a water-in-oil emulsion or oil-in-water emulsion, preferably in the form of an oil-in-water emulsion.

11. The composition according to one of claims 8 to 10, further comprising at least one additional therapeutic agent.

12. The composition according to claim 11, wherein the at least one additional therapeutic agent is chosen from among an analgesic, non-steroidal antiinflammatory, cortisone, derivative of cortisone, or combinations thereof.

13. The composition according to claim 12, wherein the pharmaceutically acceptable derivative is chosen from among compound I-B, compound I-C, compound I-D, compound I-E, compound I-F, compound I-G, compound I-H, compound I-I, compound I-J, preferably compound IB, compound IC, compound ID, compound IF, compound la-A, the compound of formula la-B, the compound of formula la-C, the compound of formula la-E, the compound of formula la-F, the compound of formula la-H, the compound of formula Ia-I, the compound of formula la-G, and the combinations thereof.
